(19) Europäisches Patentamt
European Patent Office
Office européen des brevets

(11) **EP 4 139 655 B1**

(12) **EUROPEAN PATENT SPECIFICATION**

(45) Date of publication and mention of the grant of the patent:
**03.12.2025 Bulletin 2025/49**

(21) Application number: **21728339.9**

(22) Date of filing: **21.04.2021**

(51) International Patent Classification (IPC):
*G01N 15/14* (2024.01)    *G01N 33/50* (2006.01)
*G01N 33/569* (2006.01)    *G01N 33/80* (2006.01)

(52) Cooperative Patent Classification (CPC):
**G01N 33/5094; G01N 15/1459; G01N 33/56972;**
**G01N 33/56983; G01N 33/80;** G01N 15/01;
G01N 2015/1486; G01N 2333/165

(86) International application number:
**PCT/US2021/028454**

(87) International publication number:
**WO 2021/216752 (28.10.2021 Gazette 2021/43)**

(54) **METHOD FOR ASSESSING VIRAL INFECTION**

METHODE ZUR BEURTEILUNG EINER VIRUSINFEKTION

MÉTHODE D'ÉVALUATION D'UNE INFECTION VIRALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.04.2020 US 202063013411 P**

(43) Date of publication of application:
**01.03.2023 Bulletin 2023/09**

(73) Proprietor: **Beckman Coulter, Inc.**
**Brea, CA 92821 (US)**

(72) Inventors:
• **LU, Jiuliu**
**Brea, CA 92821 (US)**
• **RAMIREZ, Carlos**
**Brea, CA 92821 (US)**
• **RILEY, John**
**Brea, CA 92821 (US)**

(74) Representative: **Maiwald GmbH**
**Elisenhof**
**Elisenstraße 3**
**80335 München (DE)**

(56) References cited:
• ELLIOTT D. CROUSER ET AL: "Improved Early Detection of Sepsis in the ED With a Novel Monocyte Distribution Width Biomarker", CHEST, vol. 152, no. 3, 15 June 2017 (2017-06-15), US, pages 518 - 526, XP055565295, ISSN: 0012-3692, DOI: 10.1016/j.chest.2017.05.039
• CROUSER ELLIOTT D. ET AL: "Monocyte Distribution Width : A Novel Indicator of Sepsis-2 and Sepsis-3 in High-Risk Emergency Department Patients*", CRITICAL CARE MEDICINE., vol. 47, no. 8, 1 August 2019 (2019-08-01), US, pages 1018 - 1025, XP055816565, ISSN: 0090-3493, Retrieved from the Internet <URL:https://mitalab.vn/wp-content/uploads/2020/05/Monocyte-Distribution-Width-A-Novel-Indicator-of-sepsis-2-and-sepsis-3.pdf> DOI: 10.1097/CCM.0000000000003799
• FRATER JOHN L. ET AL: "COVID-19 and the clinical hematology laboratory", INTERNATIONAL JOURNAL OF LABORATORY HEMATOLOGY, vol. 42, no. S1, 20 April 2020 (2020-04-20), GB; US, pages 11 - 18, XP055816538, ISSN: 1751-5521, Retrieved from the Internet <URL:https://onlinelibrary.wiley.com/doi/full-xml/10.1111/ijlh.13229> DOI: 10.1111/ijlh.13229

EP 4 139 655 B1

- **TAN LI ET AL: "Lymphopenia predicts disease severity of COVID-19: a descriptive and predictive study", SIGNAL TRANSDUCTION AND TARGETED THERAPY, vol. 5, no. 1, 27 March 2020 (2020-03-27), pages 33, XP055816125, Retrieved from the Internet <URL:https://www.nature.com/articles/s41392-020-0148-4.pdf> DOI: 10.1038/s41392-020-0148-4**
- **MOHAN SOWJANYA S ET AL: "The diagnostic and prognostic significance of relative lymphopenia in adult patients with influenza A", AMERICAN JOURNAL OF MEDICINE, EXCERPTA MEDICA, INC, UNITED STATES, vol. 118, no. 11, 1 November 2005 (2005-11-01), pages 1307 - 1309, XP086229545, ISSN: 0002-9343, [retrieved on 20051104], DOI: 10.1016/J.AMJMED.2005.06.018**
- **ZHU YUXIN ET AL: "Optimal Decision Rule for Combining Multiple Biomarkers into Tree-based Classifier and its Evaluation A dissertation submitted to The Johns Hopkins University in conformity with the requirements for the degree of Doctor of Philosophy", 1 January 2018 (2018-01-01), XP055816519, Retrieved from the Internet <URL:https://jscholarship.library.jhu.edu/bitstream/handle/1774.2/59997/ZHU-DISSERTATION-2018.pdf?sequence=1> [retrieved on 20210622]**
- **OGNIBENE AGOSTINO ET AL: "Elevated monocyte distribution width in COVID-19 patients: The contribution of the novel sepsis indicator", CLINICA CHIMICA ACTA, ELSEVIER BV, AMSTERDAM, NL, vol. 509, 3 June 2020 (2020-06-03), pages 22 - 24, XP086250684, ISSN: 0009-8981, [retrieved on 20200603], DOI: 10.1016/J.CCA.2020.06.002**

Remarks:

The file contains technical information submitted after the application was filed and not included in this specification

**Description**

FIELD OF THE INVENTION

The present disclosure relates to methods for assessing viral infection, wherein the viral infection is SARS-CoV-2 or influenza, using various hematology parameters.

BACKGROUND OF THE INVENTION

In certain systems, a significant number of individuals seeking care have an uncertain projected clinical course. The time required to investigate individuals with an uncertain projected clinical course can lead to prolonged wait times and dwell times, and can be a source of care facility inefficiencies. Furthermore, prolonged wait times and prolonged times to diagnosis may result in adverse outcomes, especially since certain severe conditions may deteriorate rapidly. Crouser et al., 2017 relates to the early detection of sepsis with a monocyte distribution width biomarker (CHEST, vol. 152, no. 3, 2017, 518-526). Crouser et al., 2019 relates to monocyte distribution width as an indicator of sepsis-2 and sepsis-3 in high-risk emergency department patients (Critical Care Medicine, vol. 47, no. 8, 2019, 1018-1025). Frater et al., 2020 discusses the clinical hematology laboratory's role in the diagnosis of COVID-19 infection, as well as biomarker analysis in COVID-19 infection (International Journal of Laboratory Hematology, vol. 42, no. S1, 2020, 11-18).

SUMMARY OF THE INVENTION

The present invention provides a method for assessing viral infection, wherein the viral infection is SARS-CoV-2 or influenza, the method comprising: characterizing a white blood cell count (WBC) in a blood sample; calculating a monocyte distribution width (MDW) value for the blood sample; if the MDW value for the blood sample is less than or equal to 20, indicating that viral infection is unlikely; and if the MDW value for the blood sample is greater than or equal to 20, evaluating percent lymphocytes and providing an indication of suspected viral infection if the lymphocyte percent is less than 15%.

In some embodiments, the method is automatically performed on any blood sample tested for CBC with differential (CBC-DIFF).

In some embodiments, the method further comprises assessing one or more of axial light loss (ALL) mean for monocytes; direct current (DC) mean for monocytes; standard deviation for ALL for monocytes; percent basophils; mean corpuscular hemoglobin concentration (MCHC); Low Hemoglobin Density (LHD) value; the standard deviation, in volume, of WBC; the standard deviation, in volume, of Monocytes and Neutrophils; or percent neutrophils.

In some embodiments, characterizing the WBC in a blood sample comprises impedance and light scatter measurements.

In some embodiments, characterizing the WBC in a blood sample does not rely on cytochemical stains or affinity-based markers.

In some embodiments, characterizing the WBC comprises less than a full CBC-DIFF.

In some embodiments, the method is automated.

BRIEF DESCRIPTION OF THE DRAWING

Some aspects are illustrated by way of example and not limitation in the figures of the accompanying drawings.

FIG. 1 is a schematic depiction of an example operating environment, in accordance with aspects of the present disclosure.

FIG. 2 is schematic depiction of an example analyzer, in accordance with aspects of the present disclosure.

FIG. 3 is a schematic depiction of an example analyzer process, in accordance with aspects of the present disclosure.

FIG. 4 is a schematic depiction of an example analysis engine, in accordance with aspects of the present disclosure.

FIG. 5 depicts a flow chart of an example method for evaluating a viral infection status from a blood sample, in accordance with aspects of the present disclosure.

FIG. 6 depicts a flow chart of an example method for evaluating a viral infection status from a blood sample, in accordance with aspects of the present disclosure.

FIG. 7 depicts a flow chart of an example method for evaluating a viral infection status from a blood sample, in accordance with aspects of the present disclosure.

FIG. 8 depicts a flow chart of an example method for detecting an infection, in accordance with aspects of the present disclosure.

FIG. 9 shows sequential assessment of sepsis probabilities according to WBC and MDW followed by CRP per sepsis-2 criteria, in accordance with aspects of the present disclosure.

FIG. 10 shows sequential assessment of sepsis probabilities according to WBC and MDW followed by PCT per sepsis-2 criteria, in accordance with aspects of the present disclosure.

FIG. 11 shows sequential assessment of sepsis probabilities according to WBC and MDW followed by PCT per sepsis-3 criteria, in accordance with aspects of the present disclosure.

FIG. 12 depicts a flow chart of an example method for evaluating an acuity of an individual, in accordance with aspects of the present disclosure.

FIG. 13 depicts a flow chart of an example method for assessing a severity of an infection, in accordance with aspects of the present disclosure.

FIG. 14 depicts a flow chart of an example method for evaluating an acuity of an individual, in accordance with aspects of the present disclosure.

FIG. 15 depicts a flow chart of an example method for assessing a severity of an infection, in accordance with aspects of the present disclosure

FIGS. 16A-26D depict violin plots of measurements of various blood parameters for certain populations of individuals and associated outcomes, in accordance with the example study described herein and in accordance with aspects of the present disclosure.

## DETAILED DESCRIPTION OF THE INVENTION

### GENERAL DESCRIPTION

The claimed invention provides a method for assessing viral infection, wherein the viral infection is SARS-CoV-2 or influenza in accordance with independent claim 1. Other conditions such as bacterial or fungal infections, or sepsis, are disclosed for context. The present disclosure relates to methods for assessing one or more parameters associated with an individual to make a medical determination regarding that individual. For example, these parameters, such as those measured by a hematology analyzer or calculated based on those measured by a hematology analyzer, may be used to determine whether the individual has a particular medical condition, whether the individual has an elevated risk of a particular medical condition, a severity of an individual's particular medical condition, an acuity associated with the individual, and the like. As used herein, parameters may refer to hematology, clinical chemistry, immunoassay, or a combination thereof. The medical condition, for exemplary purposes, may be inflammation, an infection (e.g., viral (influenza, Covid-19, etc.), bacterial, fungal) or a condition that is the result of an infection (e.g., sepsis). Certain parameters have been found to more closely correlate to the existence of a medical condition, the severity of a medical condition, or an individual's outcome.

In aspects provided herein, the severity of an individual's medical condition could be characterized by an individual's outcome, such as, for example, organ failure, organ dysfunction, or mortality or could be characterized by their need for services, such as emergency surgery, critical care, or hospitalization. As such, a correlation between one or more hematological parameters and an individual's outcome can be used by medical professionals to make a medical determination regarding the individual. In one instance, when the individual's medical condition is sepsis, for example, the one or more hematological parameters may be used to determine a severity of sepsis in the individual. The severity of sepsis could include just an infection without other symptoms, septic shock, etc. The correlation of one or more parameters to a medical condition or outcome also exists for individuals who are immunosuppressed.

Exemplary parameters used to make these determinations include characterization of blood cell populations (i.e. monocyctes, leukocytes, white blood cells, red blood cells, neutrophils, lymphocytes, immature granulocytes, etc.) and may include the population percentage, the average volume of the cell population, the average width of the cell population, the absolute count of the cell population, etc. More particularly, exemplary parameters include monocyte distribution width (MDW), white blood cell count (WBC), monocyte %, absolute lymphocyte count (ALC), lymphocyte %, absolute neutrophil (ANC), neutrophil %, procalcitonin (PCT), Lactic Acid, blood urea nitrogen (BUN), sodium (Na), potassium (K) or C-reactive protein (CRP). MDW, in particular, has been found to correlate to a severity and a risk of various medical conditions, in addition to an individual's acuity. For example, a value of MDW has been found to correlate to clinical acuity whether the individual's condition is bacterial, viral, inflammatory, etc. in individuals, including immunocompromised individuals. MDW values have been found to be particularly useful in this regard when it is assessed in combination with one or more other hematological parameters, such that those listed above.

As used herein, acuity generally refers to the level of care an individual needs and can also correlate with a severity of an illness or condition, or a risk of developing or having an illness or condition, even if undiagnosed or no likely suspected illness is known. Specific examples of acuity are described herein, including but not limited to, whether or not an individual needs critical care, the individual is at risk of in-hospital mortality (e.g., within 48 hours of admission), the individual is at risk of sepsis or is at risk of severe infection or other condition. As used herein, the term risk refers to likelihood, where for example, in the context of an individual at risk of particular illness or condition, means it is likely (or more likely than not) that that individual may have the illness or condition, or may develop such illness or condition. As used herein, an elevated risk, in the context of developing or having an illness or condition, refers to a risk of developing or having an illness or condition that is high enough to warrant treatment (e.g., preventative or other treatment) immediately or within 24-48 hours.

For exemplary purposes, MDW values, when compared to a predetermined criteria or threshold, have been found to correlate to a severity of a viral infection or a medical condition resulting from a viral infection, but even more so correlates to the severity when one or more other hematological parameters are assessed and compared to a predetermined criteria or threshold. For instance, lymphocyte % and/or neutrophil % strongly correlate to a severe instance of sepsis when used in conjunction with MDW. For a slightly less severe instance of sepsis, lymphocyte % and neutrophil % also strongly correlate in conjunction with MDW, but so do WBC, ANC, CRP, and BUN.

In some aspects provided herein, sequential applications of parameters may provide improved assessments, and, surprisingly, that this may be the case even when analysis shows that using the same parameters in combination did not show added value over the performance of a first parameter in sequence (e.g., a sequence in which CRP or PCT are evaluated after MDW may provide improved predictive power, even though CRP + MDW or PCT + MDW does not appear to provide added value over MDW alone). Additionally, individual measurements may be combined with particular cut offs. As described herein, this may improve earlier infection detection and potentially could reduce time to antibiotics administration. In some instances, particular combinations of individual measurements at coordinated cut off values may improve infection detection where use of the same measurements at different cut off values may not improve infection detection.

Additionally, the methods of the present disclosure relate to assessing viral infection by characterizing the WBC in a blood sample. Claim 1 is directed to assessing viral infection, wherein the viral infection is SARS-CoV-2 or influenza in accordance. The method comprises calculating the MDW value for the blood sample. If the MDW for the blood sample is less than or equal to a particular MDW value, 1.e. 20, the method comprises indicating that viral infection is unlikely. If the MDW value for the blood sample is greater than or equal to the particular MDW value (i.e., 20), the method comprises evaluating percent lymphocytes. One or more of a standard deviation for neutrophil LALS, a WBC percent eosinophils, a monocyte index, a mean ALL for monocytes, a standard deviation for monocyte MALS, a monocyte opacity mean, a standard deviation for ALL for monocytes, a WBC percent basophils, LHD, a standard deviation for volume for WBC, a standard deviation for volume for monocytes and neutrophils, and a WBC percent neutrophils. may also be evaluated. The disclosure relates to a method for assessing viral infection which, in some aspects, is severe acute respiratory syndrome coronavirus 2 (the virus causing the disease known as COVID-19. The method comprises characterizing the WBC in a blood sample. The method comprises evaluating the likelihood that the blood sample is from an individual with an active viral infection based on MDW and a WBC percent lymphocytes. A standard deviation for neutrophil LALS and a WBC percent eosinophils may also be evaluated. The evaluation may comprise a decision rule, a linear combination of two or more parameters, and calculating a probability that the blood sample is from an individual with a viral infection based on two or more parameters.

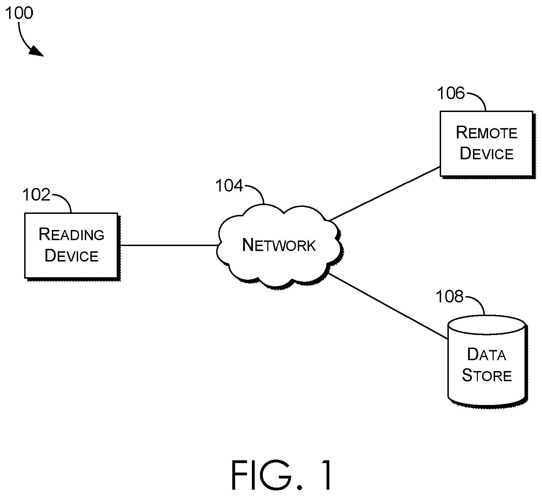

Turning to FIG. 1, an example operating environment 100 is depicted in accordance with some aspects described herein. Generally, example operating environment 100 includes systems that can facilitate diagnostic, predictive, and medical intervention action described herein. Operating environment 100 is one example of a suitable environment and system architecture for implementing an embodiment of the disclosure. As described above, some methods may be implemented using a system, comprising one or more computers and associated network and equipment, upon which a method or computer software application is executed. Accordingly, the present disclosure may combine software and hardware aspects that may all generally be referred to herein as a "module" or "system." Further, the methods of the present disclosure may take the form of a computer application embodied in computer readable media having machine-readable application software embodied thereon. In this regard, a machine-readable storage media may be any tangible medium that can contain, or store a software application for use by the computing apparatus.

Operating environment 100 may include at least one analyzer 102. An analyzer is a clinical diagnostic machine capable of measuring one or more anatomical or physiological properties of a sample, including: vitals, metabolic measurements (also referred to as blood chemistry); cell counts; viral protein, viral gene or microbial cell measurements; urine measurements; genomic characterizations; or mass spectrometry and/or immunological measurements. Analyzers as used herein include workcells or modular systems where two or more types of measurements are taken; for example, a workcell comprising blood chemistry and immunoassay.

Example operating environment 100 may include network 104. Network 104 generally facilitates communication between analyzer 102 and any other device communicatively coupled to network 104. As such, network 104 can include access points, routers, switches, or any other network component commonly understood to facilitate communication among devices. By way of example network 104 can include one or more wide area networks, one or more local area networks, one or more public networks, one or more private networks, one or more telecommunications networks, or any combination thereof. In other words, network 104 may include multiple networks, or a network of networks, but is depicted in a simple form so as not to obscure aspects described herein.

Example operating environment 100 may include remote device 106. Remote device 106 can take on a variety of forms, such as a personal computer (PC), a smart phone, a smart watch, a laptop computer, a mobile phone, a mobile device, a tablet computer, a wearable computer, a personal digital assistant (PDA), any combination of these delineated devices, or any other device that can communicate directly or indirectly with an analyzer (e.g., analyzer 102) and/or a data store (e.g., data store 108). For example, remote device 106 may comprise a work station PC that can execute a local client application. The local client application can communicatively connect with analyzer 102, data store 108, or both. For example, the local client application can be an application that facilitates user interaction with the analyzer 102. The local client application For another example, the local client application can be a electronic medical record system application that facilitates user interaction with an electronic medical record system maintained by a data store.

Example operating environment 100 may include one or more data stores 108. Data store 108 generally stores, maintains, and communicates data through network 104. Data store 108 may comprise hardware, software, firmware in any combination. For example, data store 108 can include an electronic medical record (EMR) system. The EMR system can store medical information (e.g., demographic, physical, biological, and so forth) about a plurality of individuals. In other words, an EMR is a real-time, comprehensive collection of patent data including medical history, physician notes, diagnoses, medication, allergies, immunizations, laboratory test results and vital signs. An EMR system stores and maintains a plurality of EMRs.

For another example, data store 108 may comprise a laboratory information system (LIS). A LIS is a software system that stores, processes, and manages laboratory analyzer data, and information about an individual, including sample measurements. Laboratory test results derived from an individual's biological sample, such as WBC and MDW, may also be input to the LIS manually, by a laboratory professional, indirectly through laboratory middleware connected to one or more analyzers, or directly from an analyzer. An LIS system may add or modify patent data stored in an EMR system.

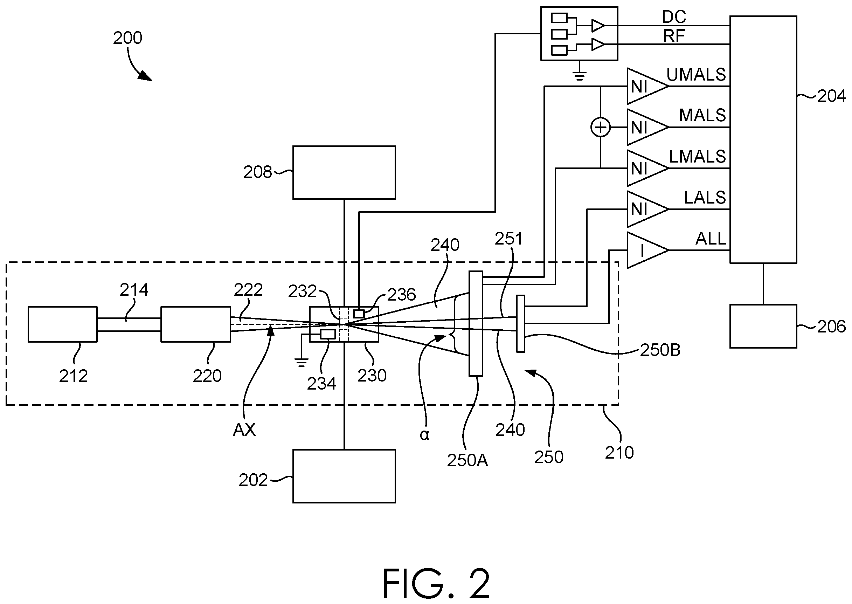

Turning to FIG. 2, a depiction of an example analyzer 200 is provided consistent with aspects described herein. Analyzer 200 depicts components in a system which may be used to take measurements of a sample, e.g., a blood sample. As will be understood by those skilled in the art, analyzers are available that work on many principles, including electrical impedance, stained fluorescence analysis, cell image analysis, and light scatter analysis. In particular, many commercially available hematology analyzers use a combination of these methodologies. For example, a Beckman Coulter D×H™ 900 Hematology Analyzer uses electrical impedance (also called DC current) to size and count cells and uses Radio Frequency (RF), light loss, and light scatter to evaluate cell morphology and further distinguish sub-populations of cells. Exemplary systems and methods are described, for example, in US 5,125,737. As will be appreciated from the disclosure of US 5,125,737, there is often more than one way of distinguishing cells in a blood sample. For example, cells may be distinguished based on volume (often measured by impedance), or by light scatter, or by combinations of parameters. If distinguished by light scatter, different angles of light scatter may be used, such as low angle light scatter (LALS), axial light loss (ALL), upper median angle light scatter (UMALS), and the like. In some cases, cells that are similar in size and morphology may best be distinguished using combinations of different measures, which may use plots (e.g., with one measure on the x-axis and another measure on the y-axis) or formulas, such as ratios or sums. As one example, eosinophils have several light scatter measures similar to neutrophils, and can be difficult to distinguish based on any single measurement. However, by looking at medium angle light scatter (MALS), a combination of UMALS and lower median angle light scatter (LMALS), eosinophils can be clearly distinguished from neutrophils as well as monocytes, lymphocytes, and basophils.

There are many thousands of possible combinations of sensor readings and calculated relationships that might correlate to a particular characteristic of a blood sample, and, once subpopulations of cells have been identified, a particular subpopulation of cells may be further characterized by one or more sensor readings (such as LALS, ALL, UMALS, LMALS, MALS, impedance, etc.), in addition to or in lieu of cytochemical staining, marker affinity, or other cell identification techniques. That is, hematology analyzers can often provide data about a subpopulation of cells that is much richer than simply a count or proportion of those cells compared to other subpopulations of cells within a sample. One example is Monocyte Distribution Width (MDW), a calculation of the standard deviation of cell volumes within the subpopulation of monocytes within a blood sample. This characterization of the monocyte population is associated with sepsis, as described, for example, in US patent applications 62/288,091; 62/927,835; 62/660,795; 62/873,806; 62/873,575; and 62/685,753. In some cases, more than one characterization of a subpopulation of cells or relationship between subpopulation of cells may be indicative of the same or related conditions, such as viral infection, sepsis, anemia, leukemia, etc.

As shown here, analyzer 200 includes a transducer module 210 having a light or irradiation source such as a laser 212 emitting a beam 214. The laser 212 can be, for example, a 635 nm, 5 mW, solid-state laser. In some instances, analyzer 200 may include a focus-alignment system 220 that adjusts beam 214 such that a resulting beam 222 is focused and positioned at a cell interrogation zone 232 of a flow cell 230. In some instances, the flow cell 230 receives a sample aliquot from a preparation system 202. Various fluidic mechanisms and techniques can be employed for hydrodynamic focusing of the sample aliquot within flow cell 230.

In some instances, the aliquot generally flows through the cell interrogation zone 232 such that its constituents pass through the cell interrogation zone 232 one at a time. In some cases, an analyzer 200 may include a cell interrogation zone or other feature of a transducer module or blood analysis instrument such as those described in U.S. Pat. Nos. 5,125,737; 6,228,652; 7,390,662; 8,094,299; 8,189,187; and 9,939,453. For example, a cell interrogation zone 232 may be defined by a square transverse cross-section measuring approximately 50×50 microns, and having a length (measured in the direction of flow) of approximately 65 microns. Flow cell 230 may include an electrode assembly having first and second electrodes 234, 236 for performing DC impedance and/or RF conductivity measurements of the cells passing through cell interrogation zone 232. Signals from electrodes 234, 236 can be transmitted to the analysis system 204. The electrode assembly can analyze volume and conductivity characteristics of the cells using low-frequency current and high-frequency current, respectively. For example, low-frequency DC impedance measurements can be used to analyze the volume of each individual cell passing through the cell interrogation zone. High-frequency RF current measurements can be used to determine the conductivity of cells passing through the cell interrogation zone. Because cell walls act as conductors to high frequency current, the high frequency current can be used to detect differences in the insulating properties of the cell components, as the current passes through the cell walls and through each cell interior. High frequency current can be used to characterize nuclear and granular constituents and the chemical composition of the cell interior.

The light source in FIG. 2 has been described as a laser, however, the light source may alternatively or additionally include a xenon lamp, an LED lamp, an incandescent lamp, or any other suitable source of light, including combinations of the same or different kinds of lamps (e.g., multiple LED lamps or at least one LED lamp and at least one xenon lamp). As shown in Fig. 2, for example, incoming beam 222 irradiates the cells passing through cell interrogation zone 232, resulting in light propagation within an angular range α (e.g. scatter, transmission) emanating from the zone 232. Exemplary systems are equipped with sensor assemblies that can detect light within one, two, three, four, five, or more angular ranges within the angular range α, including light associated with an extinction or axial light loss measure. As shown, light propagation 240 can be detected by a light detection assembly 250, optionally having a light scatter detector unit 250A and a light scatter and/or transmission detector unit 250B. In some instances, light scatter detector unit 250A includes a photoactive region or sensor zone for detecting and measuring upper median angle light scatter (UMALS), for example, light that is scattered or otherwise propagated at angles relative to a light beam axis within a range from about 20 to about 42 degrees. In some instances, UMALS corresponds to light propagated within an angular range from between about 20 to about 43 degrees, relative to the incoming beam axis, which irradiates cells flowing through the interrogation zone. Light scatter detector unit 250A may also include a photoactive region or sensor zone for detecting and measuring lower median angle light scatter (LMALS), for example, light that is scattered or otherwise propagated at angles relative to a light beam axis within a range from about 10 to about 20 degrees. In some instances, LMALS corresponds to light propagated within an angular range from between about 9 to about 19 degrees, relative to the incoming beam axis which irradiates cells flowing through the interrogation zone.

A combination of UMALS and LMALS is defined as median angle light scatter (MALS), which may be light scatter or propagation at angles between about 9 degrees and about 43 degrees relative to the incoming beam axis which irradiates cells flowing through the interrogation zone. One of skill in the art will understand that these angles (and the other angles described herein) may vary somewhat based on the configuration of the interrogation, sensing and analysis systems.

As shown in FIG. 2, the light scatter detector unit 250A may include an opening 251 that allows low angle light scatter or propagation 240 to pass beyond light scatter detector unit 250A and thereby reach and be detected by light scatter and transmission detector unit 250B. According to some embodiments, light scatter and transmission detector unit 250B may include a photoactive region or sensor zone for detecting and measuring lower angle light scatter (LALS), for example, light that is scattered or propagated at angles relative to an irradiating light beam axis of less than about 5.1 degrees. In some instances, LALS corresponds to light propagated at an angle of less than about 9 degrees, relative to the incoming beam axis, which irradiates cells flowing through the interrogation zone. In some instances, LALS corresponds to light propagated at an angle of less than about 10 degrees, relative to the incoming beam axis, which irradiates cells flowing through the interrogation zone. In some instances, LALS corresponds to light propagated at an angle of about 1.9 degrees ±0.5 degrees, relative to the incoming beam axis, which irradiates cells flowing through the interrogation zone. In some instances, LALS corresponds to light propagated at an angle of about 3.0 degrees ±0.5 degrees, relative to the incoming beam axis, which irradiates cells flowing through the interrogation zone. In some instances, LALS corresponds to light propagated at an angle of about 3.7 degrees ±0.5 degrees, relative to the incoming beam axis, which irradiates cells flowing through the interrogation zone. In some instances, LALS corresponds to light propagated at an angle of about 5.1 degrees ±0.5 degrees, relative to the incoming beam axis, which irradiates cells flowing through the interrogation zone. In some instances, LALS corresponds to light propagated at an angle of about 7.0 degrees ±0.5 degrees, relative to the incoming beam axis, which irradiates cells flowing through the interrogation zone. In each instance, LALS may correspond to light propagated an angle of about 1.0 degrees or more. That is, LALs may correspond to light propagated at angles between about 1.0 degrees and about 1.9 degrees; between about 1.0 degrees and about 3.0 degrees; between about 1.0 degrees and about 3.7 degrees; between about 1.0 degrees and about 5.1 degrees, between about 1.0 degrees and about 7.0 degrees, between about 1.0 degrees and about 9.0 degrees; or between about 1.0 degrees and about 10.0 degrees.

According to some embodiments, light scatter and transmission detector unit 250B may include a photoactive region or sensor zone for detecting and measuring light transmitted axially through the cells, or propagated from the irradiated cells, at an angle of about 0 degrees relative to the incoming light beam axis. In some cases, the photoactive region or sensor zone may detect and measure light propagated axially from cells at angles of less than about 1 degree relative to the incoming light beam axis. In some cases, the photoactive region or sensor zone may detect and measure light propagated axially from cells at angles of less than about 0.5 degrees relative to the incoming light beam axis less. Such axially transmitted or propagated light measurements correspond to axial light loss (ALL or AL2). As noted in U.S. Pat. No. 7,390,662, when light interacts with a particle, some of the incident light changes direction through the scattering process (i.e., light scatter) and part of the light is absorbed by the particles. Both of these processes remove energy from the incident beam. When viewed along the incident axis of the beam, the light loss can be referred to as forward extinction or axial light loss. Additional aspects of axial light loss measurement techniques are described in U.S. Pat. No. 7,390,662 at column 5, line 58 to column 6, line 4.

As such, the analyzer 200 provides means for obtaining light propagation measurements, including light scatter and/or light transmission, for light emanating from the irradiated cells of the biological sample at any of a variety of angles or within any of a variety of angular ranges, including ALL and multiple distinct light scatter or propagation angles. For example, light detection assembly 250, including appropriate circuitry and/or processing units, provides a means for detecting and measuring UMALS, LMALS, LALS, MALS, and ALL.

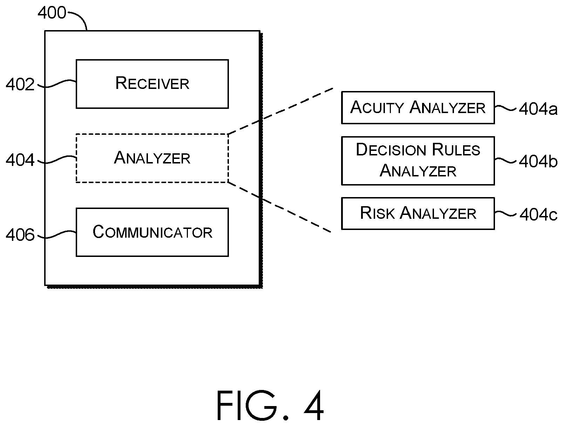

Wires or other transmission or connectivity mechanisms can transmit signals from the electrode assembly (e.g. electrodes 234, 236), light scatter detector unit 250A, and/or light scatter and transmission detector unit 250B to the analysis system 204 for processing. For example, measured DC impedance, RF conductivity, light transmission, and/or light scatter parameters can be provided or transmitted to the analysis system 204 for data processing. In some instances, analysis system 204 may include computer processing features and/or one or more modules or components, which can evaluate the measured parameters, identify and enumerate biological sample constituents, and correlate a subset of data characterizing elements of the biological sample with one or more features or parameters of interest. Some aspects of analysis system 204 include an analysis engine such as described in relation to FIG. 4.

Additionally, or alternatively, as depicted in FIG. 2, analyzer 200 may generate or output a report 206 presenting measurements made or parameters calculated for the sample. The measurements made or parameters calculated for a sample can include UMALS, LMALS, LALS, MALS, ALL, WBC, MDW, monocyte %, absolute lymphocyte count (ALC), lymphocyte %, eosinophil %, absolute neutrophil count (ANC), neutrophil %, or any combination thereof.

In some instances, excess biological sample from transducer module 210 can be directed to an external (or alternatively internal) waste system 208. In some instances, the analyzer 200 may include one or more features of a transducer module or blood analysis instrument such as those described in U.S. Pat. Nos. 5,125,737; 6,228,652; 8,094,299; 8,189,187 and 9,939,453.

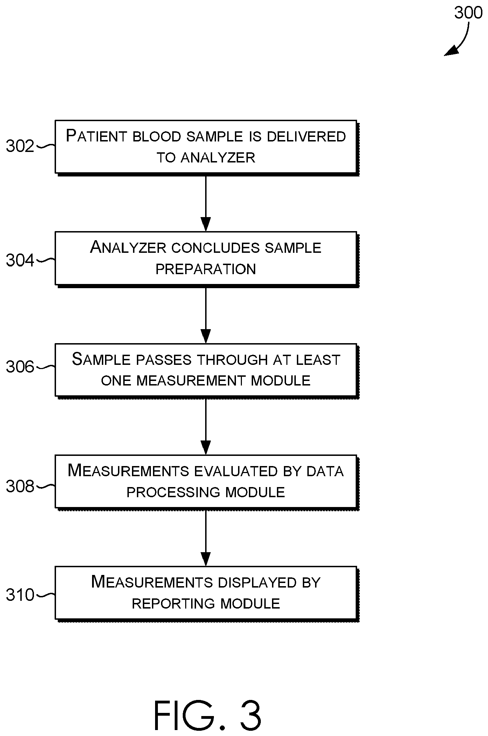

FIG. 3 schematically depicts an exemplary analyzer process 300, e.g., which can optionally utilize the analyzer 200 of FIG. 2. In this embodiment, at the step 302, an individual's blood sample may be delivered to the analyzer, at which point the analyzer may prepare the sample for analysis. Once the sample preparation is concluded at step 304, the sample may pass through one or more measurement modules at the step 306. The measurement modules of the step 306 can include a conductivity module, a light scatter module, an RF module, or any combination thereof. Other modules may be used instead of or in addition to a conductivity module or a light scatter module. For example, a hematology analyzer may use sensors to detect dyes or fluorescent markers, imaging, immunoassay markers, size sorting, or other approaches to identify cells or other sample components. Sample measurements may then be evaluated by a data processing module in the step 308. In some aspects, once the sample measurements are complete, the measurements may be displayed by a reporting module in the step 310. Additionally, or alternatively, once the sample measurements are complete the measurements may be communicated to an analysis engine for further processing, such as the example analysis engine 400 of FIG. 4.

FIG. 4 depicts an example analysis engine 400, in accordance with aspects described herein. Aspects of analysis engine 400 can be incorporated into a processing feature and/or module or component of an analyzer (such as analysis system 204 depicted in FIG. 2), an application executed by a remote device (e.g., remote device 106 depicted in FIG. 1), or can operate as an independent component of an operating environment (e.g., operating environment 100 depicted in FIG. 1).

Generally, the analysis engine 400 evaluates a set of measurements or parameters, identifies and enumerates biological sample constituents, and correlates a subset of data characterizing elements of the biological sample with one or more features or parameters of interest. As such, analysis engine 400 includes a receiver module, an analyzer module, and a communicator module.

A receiver, such as receiver 402, generally collects measurements made or parameters calculated based on analysis of an individual's sample. The data (e.g., measurements made or parameters calculated) can be received directly from a subsystem of an analyzer or from a data store in some aspects. Receiver 402 can use any data collection technique known in the art.

Data analyzer 404 includes modules that include logical expressions for the evaluation of measurements and parameters received by the analysis engine 400. The logical expressions can include linear or parallel processes that evaluate measurements made by or parameters calculated by a hematology analyzer, such as analyzer 102 described in relation to FIG 1 or analyzer 200 described in relation to FIG. 2. The data analyzer 404 includes at least one of an acuity analyzer 404a, a decision rules analyzer 404b, a risk analyzer 404c.

Acuity analyzer 404a comprises a library of rules, models, and logic expressions, in any combination that facilitate the determination of a probability and/or risk of one or more outcomes based on one or more parameters or characteristics of a blood sample. A potential outcome can be associated with a recommendation, treatment, or intervention in some aspects. For example, where an individual's outcome corresponds with a risk of shock, a recommendation to transfer the individual to an intensive care/critical care unit can be associated therewith.

Decision rules analyzer 404b comprises a library of decision rules. A decision rule is a logic expression that compares an individual parameter or characteristic of a blood sample with a threshold value. The decision rules analyzer 404b assembles one or more decision rules from the library to build a logical expression that the analysis engine can evaluate. The analyzer 404b may utilize a linear combination or two or more parameters. In combination, the decision rules can be used to determine a probability that an individual associated with a blood sample currently has a condition, such as, for example, an infection, including viral infection.

Risk analyzer 404c can include rules, models, logic expressions, in any combination that are configured to forecast medical conditions. For example, the risk analyzer module can characterize the information received from an analyzer to determine an individual's risk of developing sepsis. Additionally, some aspects of the risk analyzer module can character the information received from the analyzer to determine a probability of sepsis severity.

The data analysis engine 400 may incorporate the operations of one or more analyzer modules to generate an output. For example, the decision rules maintained by a decision rules analyzer 404b can be used to determine if an individual currently has a condition, such as an infection. In response to determination that the individual has a probability of infection above a particular threshold, some aspects of the data analysis engine 400 can activate the risk analyzer 404c to facilitate the determining if the individual is at an elevated risk of developing sepsis or septic shock. In response to the determination that the individual has an elevated risk of developing sepsis or septic shock, some aspects of the data analysis engine 400 can activate the acuity analyzer 404a to facilitate determining a recommended level of care or disposition. The acuity analyzer 404a may first identify an individual is at risk of needing critical care and/or at risk of in-hospital mortality, e.g., within 48 hours of obtaining the blood sample, then one or more of the decision rules analyzer 404b and/or the risk analyzer 404c can be utilized for further determinations.

Communicator 406 generally communicates the results of the analysis engine 400 to at least one predetermined target. The predetermined target may include a remote device that is executing a local client of a laboratory information system or a local client of an electronic medical record system (e.g., remote device 106 described in relation to FIG. 1). The results may include presentation of a visual display or audio signal that provides a recommendation of care, recommendation to authorize discharge, a recommendation of diagnosis, or an alert that the individual corresponding to the analyzed sample may develop sepsis or other severe condition.

The predetermined target may include a data store maintaining a laboratory information system or an electronic medical record system (e.g., data store 108 described in relation to FIG. 1). The communicated results may include entering orders for the individual associated with the analyzed sample's medical records. For example, the orders can include transferring the individual to a critical care unit, increasing monitoring of the individual by medical personal or devices, or specific testing or standard of care protocols.

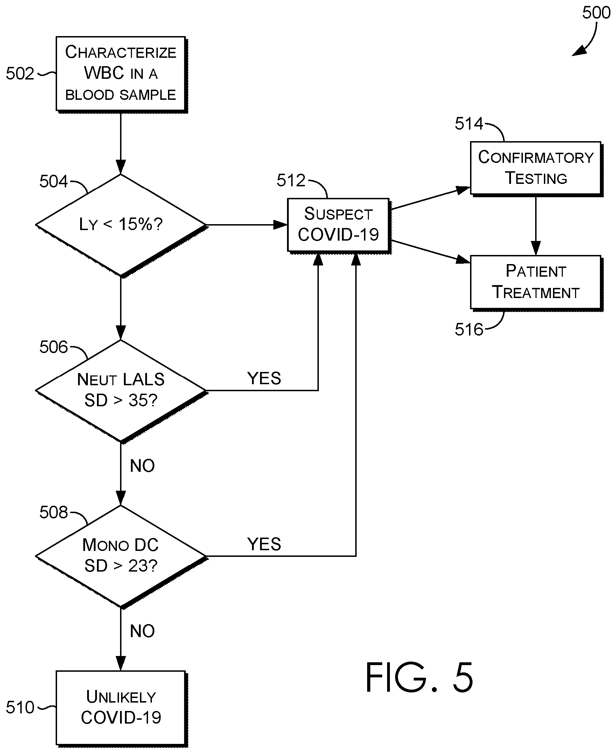

As described in more detail above, data analysis engine 400 includes at least one analyzer that processes the measurements or parameters provided to the analysis engine. The processing can include rules, models. logic expressions, in any combination that are configured to detect and/or forecast medical conditions. For example, some aspects of a decision rules analyzer (e.g., decision rules analyzer 404b described in relation to FIG. 4) can include programs that characterize the information received from an analyzer to determine a probability that an individual has developed a viral infection. In particular, a method 500 for assessing a viral infection status, such as a COVID-19 infection status, is depicted in FIG. 5 in accordance with aspects described herein. Method 500 may generally be described as a "decision rules" approach, where individual parameters or characteristics of a blood sample are considered against threshold values for each parameter or characteristic.

## HEMATOLOGICAL ASSESSMENT OF COVID INFECTION

Clinical hematology testing may include cell counting and cell population analysis. For example, a complete blood count (CBC) may report red and white blood cells in a blood sample, hemoglobin levels, hematocrit levels, and the like. A CBC-DIFF or CBC with differential may further report sub-populations of white blood cells, such as monocytes, lymphocytes, eosinophils, basophils, and neutrophils. The reported Cell Population Data (CPD) may include numbers or percentages of cells; average cell characteristics, such as volume; or cell population characteristics, such as ranges or standard deviations of measurements. Reported hematology ranges are typically correlated to information of known clinical significance, such as cell counts or an estimate of hemoglobin content. Hematology analyzers may collect additional sensor data which is not correlated to known cell characteristics or is not known to have specific clinical significance, and, therefore, is not typically reported. Further, different combinations of hematology parameters may have different clinical significance.

It has been found that certain combinations of hematology parameters correlate to a separate diagnosis of COVID-19, such as a positive RT-PCR test. Because automated or semi-automated hematology testing is readily available, relatively inexpensive, and relatively fast, using hematology parameters to identify COVID-19 patients would be advantageous over many available tests. For hematology parameters with sufficient specificity (low false positives) and sensitivity (low false negatives), clinicians might rely on the hematology testing to assess whether a patient has a viral infection, or, more particularly, whether a patient has COVID-19. In addition to hematology testing, clinicians may use condition-specific testing, such as RT-PCR or antibody affinity testing, to confirm suspicion of viral infection, such as COVID-19 infection. One of skill in the art will appreciate that a patient can have more than one kind of infection or condition active at the same time. For example, a patient can have an active viral infection and an active fungal infection at the same time. As such, an indicator of a viral infection does not necessarily rule out other infections or conditions, and vice versa.

Hematology analyzers are available that work on many principles, including electrical impedance, stained fluorescence analysis, cell image analysis, and light scatter analysis. Many commercially available hematology analyzers use a combination of these methodologies. For example, a Beckman Coulter DxH 900 Hematology Analyzer uses electrical impedance (also called DC current) to size and count cells and uses Radio Frequency (RF), light loss, and light scatter to evaluate cell morphology and further distinguish sub-populations of cells. Exemplary systems and methods are described, for example, in US5125737. As will be appreciated from the disclosure of US5125737, there is often more than one way of distinguishing cells in a blood sample. For example, cells may be distinguished based on volume (often measured by impedance), or by light scatter, or by combinations of parameters. If distinguished by light scatter, different angles of light scatter may be used, such as low angle light scatter (LALS), axial light loss (ALL), upper median angle light scatter (UMALS), and the like. In some cases, cells that are similar in size and morphology may best be distinguished using combinations of different measures, which may use plots (e.g., with one measure on the x-axis and another measure on the y-axis) or formulas, such as ratios or sums. As one example, eosinophils have several light scatter measures similar to neutrophils, and can be difficult to distinguish based on any single measurement. However, by looking at medium angle light scatter (MALS), a combination of UMALS and lower median angle light scatter (LMALS), eosinophils can be clearly distinguished from neutrophils as well as monocytes, lymphocytes, and basophils.

There are many thousands of possible combinations of sensor readings and calculated relationships that might correlate to a particular characteristic of a blood sample, and, once subpopulations of cells have been identified, a particular subpopulation of cells may be further characterized by one or more sensor readings (such as LALS, ALL, UMALS, LMALS, MALS, impedance, etc.), in addition to or in lieu of cytochemical staining, marker affinity, or other cell identification techniques. That is, hematology analyzers can often provide data about a subpopulation of cells that is much richer than simply a count or proportion of those cells compared to other subpopulations of cells within a sample. One example is Monocyte Distribution Width (MDW), a calculation of the standard deviation of cell volumes within the subpopulation of monocytes within a blood sample. This characterization of the monocyte population is associated with sepsis, as described, for example, in US patent applications US 16/073,757; US 16/925,933; PCT/US19/28486; US 16/925,943; US 16/925,937; US 16/390,597. In some cases, more than one characterization of a subpopulation of cells or relationship between subpopulation of cells may be indicative of the same or related conditions, such as viral infection, sepsis, anemia, leukemia, etc.

Method 500 comprises characterizing white blood cells (WBC) in a blood sample at block 502. The WBC characterization may comprise a CBC-DIFF. The WBC characterization may not require a complete CBC-DIFF, and may include characterizations that are not typically reported as part of a clinical CBC-DIFF. For example, in a particular aspect of block 502, method 500 includes determining a percentage of WBCs that are lymphocytes. As described above, an analyzer may count and differentiate the various WBCs included in a blood sample. Based on that information, the analyzer can determine the lymphocyte %. In an alternative aspect of block 502, an analyzer engine may query an individual's medical record for a data value that corresponds to the most recent lymphocyte %.

At decision block 504, the method 500 comprises determining whether lymphocytes make up less than 15% of the WBC in the blood sample. In other words, the analyzer determines whether the lymphocyte % determined in block 502 is less than 15% of the total WBCs counted by the analyzer. In some aspects, if lymphocytes make up less than 15% of the WBC, the sample test report may indicate suspicion of a viral infection (such as COVID-19), shown as step 512.

Alternatively, if at decision block 504 it is determined that lymphocytes make up 15% or more of the WBC, the method may proceed to decision block 506. Decision block 506 comprises identifying a subpopulation of neutrophils based on LALS measurements captured by an analyzer. For example, the method may comprise collecting LALS measurements for the subpopulation of neutrophils. The method may comprise calculating a standard deviation for the LALS measurements for the subpopulation of neutrophils. The method may comprise determining whether the standard deviation of the LALS measurements for neutrophils is greater than or equal to 35. If the analyzer determines that the standard deviation of the LALS measurements for neutrophils is greater than or equal to 35, the sample test report may indicate suspicion of a viral infection (such as COVID-19). In other words, if the analyzer determines that the standard deviation of the LALS measurements exceeds a predetermined threshold some aspects of method 500 proceed to block 512.

Alternatively, if at decision block 506 it is determined that the standard deviation of the LALS measurements for neutrophils is less than 35, the method 500 may proceed to block 508. At block 508, the analyzer determines if the standard deviation of DC measurements for monocytes in the sample exceed a predetermined threshold, such as 23. As such, method 500 may comprise identifying a subpopulation of monocytes and collecting DC measurements for the subpopulation of monocytes. The method 500 may further comprise calculating a standard deviation for the monocyte DC measurements as reported by the MDW parameter. The method 500 may further comprise determining whether the standard deviation of the DC measurements for the monocytes is greater than 23. If the analyzer determines that the standard deviation of the DC measurements for the monocytes is greater than 23, the sample test report may indicate suspicion of COVID-19, shown as step 22. In other words, if the analyzer determines that the standard deviation of the DC measurements for the monocytes exceeds a predetermined threshold some aspects of method 500 proceed to block 512.

Alternatively, if at decision block 508 it is determined that the standard deviation of the DC measurements for the monocytes is less than or equal to 23, method 500 may proceed to block 510. At block 510 the analyzer may determine that the viral infection (such as by COVID-19) is unlikely. As such, an analyzer may generate a sample test report and trigger a communicator to send the report to a predetermined target. The sample test report may indicate that viral infection (such as by COVID-19) is unlikely.

At block 512, an analyzer may generate a suspect message. As depicted, in some instances a suspect message may include a flag, message, or other signal on a test report to indicate possible viral infection (such as by COVID-19) to a clinician or researcher. In some aspects, the suspect message may include an audio or visual message communicated to a remote device that indicates that the individual associated with the sample has a possible viral infection. The indication may be provided on a screen, such as a display for a hematology analyzer, Laboratory Information System (LIS) or Electronic Medical Record (EMR), or may be provided in a print-out, fax, e-mail or other digital or hard copy report of the hematology test results.

Additionally, or alternatively, the suspect message may include modifying the individual's EMR with an indication of suspected viral infection. Further, the modification of the individual's EMR may include adding a conformation test as depicted in block 514. If COVID-19, or other viral infection, is suspected, a researcher or clinician may pursue confirmatory testing, shown as block 514. Confirmatory testing may be delayed pending hematology test results because of the cost, time, or other resources necessary to do more specific testing, such as RT-PCR or antibody affinity testing. With or without confirmatory testing 514, a suspect COVID-19 message may prompt treatment of the individual 516 adapted to possible viral and/or COVID-19 infection. As an example, a suspected viral infection may lead to different pharmaceutical treatments than a suspected bacterial infection. Similarly, a suspected viral infection may lead to different treatments than suspected trauma or cancer.

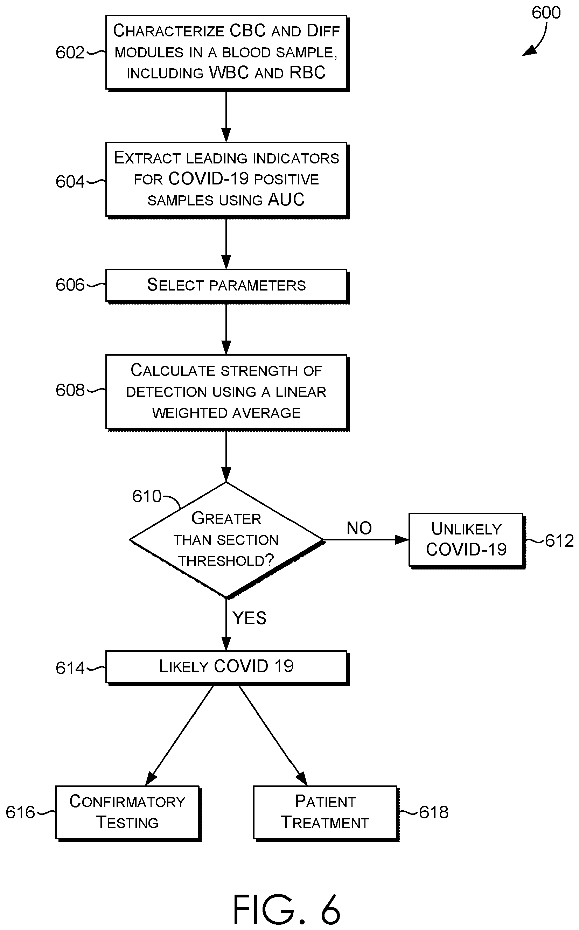

Turning to FIG. 6, a method 600 for detecting viral infections is provided in accordance with methods described herein. Similar to method 500, method 600 may generally be described as a "decision rules" approach, where individual parameters or characteristics of a blood sample are considered against threshold values for each parameter or characteristic. Method 600 may be facilitated in part by an analyzer device (e.g., analyzer 102 from FIG. 1). Additionally, or alternatively, method 600 can be facilitated by a remote device (e.g., remote device 106 from FIG. 1). The analyzer device and/or the remote device may include an analyzer engine (e.g., analyzer engine 300 from FIG. 3) that includes at least a decision rules analyzer module (e.g., decision rules analyzer 304b).

As shown, method 600 generally includes characterizing the constituent parts of a blood sample. The constituent parts are measured and parameters are determined using a hematology analyzer device. The hematology analyzer device may use optical elements, electrode elements, RF elements, any other detection elements, or any combination thereof. The constituent part measurements and parameters can be extracted from the report generated by the analyzer. Based on the measurements and parameters, an analyzer engine can calculate a predictive strength of detection based on a linear weighted average. The value calculated can be compared to a predetermined threshold and based on the comparison the analyzer engine determines if the individual associated with the analyzed sample likely has a viral infection, such as a COVID-19 infection. The analyzer engine may generate instructions to modify an individual's EMR (e.g., add or modify orders in the individual's record, add or modify notes in an individual's chart) in response to the comparison. The analyzer engine may generate an audio or visual alert that is communicated to one or more responsible care providers (e.g., a physician or nurse). In other words, aspects of method 600 facilitate detecting viral infection, such as COVID-19 infection and providing recommendations for care and treatment.

As such, method 600 may comprise characterizing CBC and Diff modules in a blood sample, including WBC and, optionally, RBC, as shown in step 602. The WBC and RBC characterization of step 502 in FIG. 5 may be the same or different from the WBC and RBC characterization in step 602 in FIG. 6. Stated differently, method 500 and method 600 may begin with the same base analysis, which then proceeds differently. Alternately, if it is known that method 500 or method 600 is desirable at the outset of testing, only certain subsets of measurements or characteristics may be obtained in the characterization of the WBC and/or RBC in the blood sample. For example, it may be unnecessary to fully quantify and characterize eosinophils to perform method 500, and it may be unnecessary to fully quantify and characterize lymphocytes to perform method 600. In a system based on DC and light scatter measures, it may be trivial in terms of sample processing times to collect a full WBC characterization, however, in systems using cytochemical staining or marker affinity testing, for example, running subsets of analyses may save money on reagents or time for running assays that are not critical to a particular methodology.

Method 600 may include characterizing WBC in a blood sample as shown in step 602. For example, block 602 may comprise identifying a subpopulation of monocytes in the blood sample. Additionally, block 602 may comprise evaluating the volume of individual cells in the subpopulation of monocytes. The estimated volumes of the individual monocytes may be used to calculate a standard deviation for the volume measurements for the monocyte population (MDW).

Method 600 may comprise extracting leading indicators for viral infection, shown at block 604. The leading indicators of viral infection (such as by COVID-19) are identified using area under the curve (AUC) values, as shown in block 604. For example, this may include determining whether an MDW value for the blood sample is greater than 20.9.

Method 600 may comprise a method for selecting such parameters, as shown at block 606, and calculating the strength of detection using a linear weighted average, shown at block 608. Method 600 may also include determining whether the weighted combination of these parameters is greater than a selected threshold, as shown at block 610. For exemplary purposes only and not limitation, if the MDW value is less than or equal to 20.9, a sample test report may indicate that viral infection (e.g., COVID-19) is unlikely, shown as block 612, or that viral infection (e.g., COVID-19) is likely, as shown at block 614. Based on this determination, method 600 may involve confirmatory testing, as shown at block 616 or treatment as shown at block 618. For example, an analyzer engine may generate instructions to modify an individual's EMR (e.g., add or modify orders in the individual's record, add or modify notes in an individual's chart, or so on) in response to the comparison. The analyzer engine may generate an audio or visual alert that is communicated to one or more responsible care providers (e.g., a physician or nurse).

Method 600 may also comprise identifying multiple characteristics of the WBC blood sample in combination. For exemplary purposes only, the eosinophils population and the mean cellular hemoglobin concentration (MCHC) in the blood sample could be used together. Method 600 may comprise a weighted average of these parameters, as shown as block 608. If the linear combination of characteristics of the WBC blood sample is less than a predetermined threshold, the sample test report may indicate that viral infection (e.g, COVID-19 infection) is unlikely, shown as step 612. If the linear combination of characteristics of the WBC blood sample is greater than a predetermined threshold, the sample test report may indicate that (e.g, COVID-19 infection) is likely, shown as step 614. In this non-limiting example, if the linear combination of the following parameters exceeds a threshold of -35.1, the report may indicate a suspicion of COVID-19:

$$x \text{ MDW } -2.695 \text{ x Eo\% } -1.661 \text{ x MCHC} > -35.1$$

The threshold used in the non-limiting example above, -35.1, may be adjusted to achieve the desired clinical sensitivity and specificity.

Alternately, or in addition to, a decision rule or linear combination of parameters, in some cases a probability that a blood sample is from an individual or research subject with a viral infection, such as COVID-19, can be calculated. For example, percent lymphocytes (as a percent of WBC), the standard deviation for neutrophil LALS, and MDW can be linearly combined to produce a probability of the disease:

$$\text{ProbCovid} = 1 / (1 + \exp(\text{-index}))$$

Where index may be:

$$-0.26 * \text{LY\%} + 0.28 \text{ neutrophil LALS SD} + 0.8 \text{ MDW} - 21.5.$$

The weight assigned to each element of the index can be modified to reflect sub-populations (e.g., geographic, demographic, or clinical sub-populations). The threshold probability necessary to signal (or to not signal) a likely infection on a test results report can be adjusted to reflect the desired sensitivity and specificity.

As mentioned above, the sample test report indications (e.g., the output of an analyzer engine) of method 600 may be the same as or different than the sample test report indications of method 500. For example, a different message or a different indicator may be used for the different methods, particularly if both methods are available on the same analyzer system, to help a clinician or researcher understand why a sample was flagged as likely or unlikely associated with viral infection, such as COVID-19 infection. Alternately, the same message or indicator may be used for either method.

Similarly, the confirmatory testing 616 and testing of the individual 618 in method 600 may be the same or different from the confirmatory testing and treatment in method 500. For example, different hematological indications of infection may be associated with different presentations, which may lead a clinician or researcher to request different confirmatory tests or a clinician to direct different treatments. In other words, and for example, because method 500 and method 600 evaluate different hematological signs of possible viral infections, blood samples that trigger a suspicion of viral infection using one method but not the other may be associated with different symptoms than blood samples that trigger a suspicion of viral infection using the other method, or using both methods. Varied signs (such as varied hematological observations) and symptoms may favor different confirmatory tests, possibly including additional tests to rule out unrelated infections or conditions, such as underlying bacterial or fungal infections or multiple, concurrent viral infections. Varied signs and symptoms may also favor different treatments, particularly, but not exclusively, if treatment is primarily supportive rather than curative.

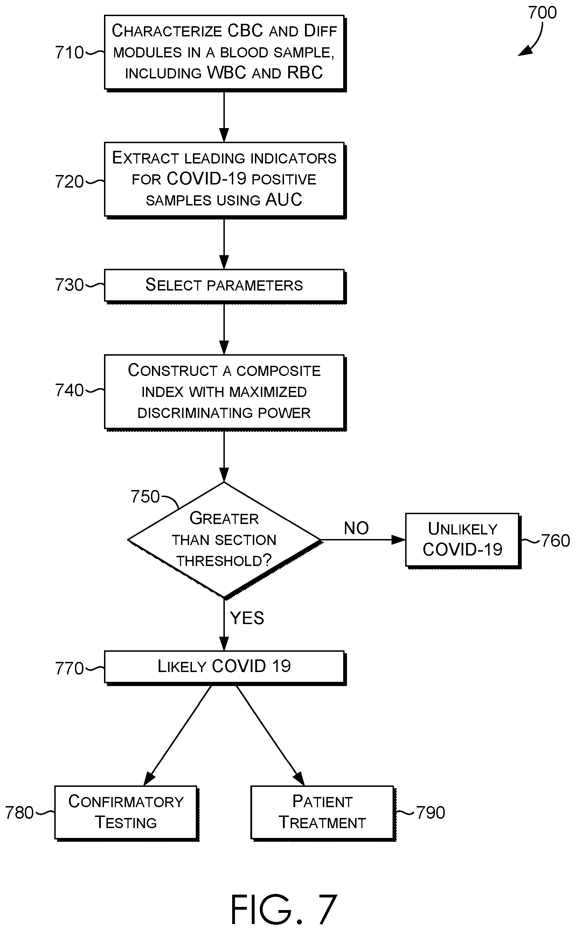

As shown in Fig. 7, a method 700 for detecting viral infection, such as COVID-19 infection, may comprise characterizing WBC and/or RBC in a blood sample 710. The WBC and/or RBC characterization of step 12 in Fig. 1 may be the same or different from the WBC and/or RBC characterization in step 210 in Fig. 2 as well as in step 710 in Figure 7.

Similar to the methods described above (methods 500 and method 600), method 700 may generally be described as a "decision rules" approach, where individual parameters or characteristics of a blood sample are considered against threshold values for each parameter or characteristic. Method 700 may be facilitated in part by an analyzer device (e.g., analyzer 102 from FIG. 1). Additionally, or alternatively, method 700 may be facilitated by a remote device (e.g., remote device 106 from FIG. 1). The analyzer device and/or the remote device may include an analyzer engine (e.g., analyzer engine 300 from FIG. 3) that includes at least a decision rules analyzer module (e.g., decision rules analyzer 304b).

As shown, a method 700 generally includes characterizing the constituent parts of a blood sample. The constituent parts are measured and parameters are determined using a hematology analyzer device. The hematology analyzer device may use optical elements, electrode elements, RF elements, any other detection elements, or any combination thereof. The constituent part measurements and parameters can be extracted from the report generated by the analyzer. Based on the measurements and parameters, an analyzer engine can calculate a composite index that is configured to maximize the discriminating power of the selected parameters. The value calculated can be compared to a predetermined threshold and based on the comparison the analyzer engine determines if the individual associated with the analyzed sample likely has a viral infection, such as a COVID-19 infection. The analyzer engine may generate instructions to modify an individual's EMR (e.g., add or modify orders in the individual's record, add or modify notes in an individual's chart, or so on) in response to the comparison. The analyzer engine may generate an audio or visual alert that is communicated to one or more responsible care providers (e.g., a physician or nurse). In other words, method 700 may facilitate detecting viral infection, such as COVID-19 infection and providing recommendations for care and treatment of the individual.

Method 600 may being at block 710 with one or more analyzers characterizing CBC and Diff modules in a blood sample, including WBC and, optionally, RBC. The WBC and RBC characterization of step 502 in FIG. 5 may be the same or different from the WBC and RBC characterization in step 710 in FIG. 7. Stated differently, method 500 and method 700 may begin with the same base analysis, which then proceeds differently.

Method 700 may include extracting leading indicators for viral infections (e.g., COVID-19). The leading indicators may be determined based on analysis of known viral infection positive samples based on AUC calculations as shown in block 720. In other words, samples of known status can be supplied to an analyzer. The results of the analysis can be used to identify indicators associated with the known viral agent. Those identified indicators can be stored by an analyzer engine associated with the analyzer as a selection filter. The selection filter can be associated with viral infections generally, or a particular viral infection (such as COVID-19).

Method 700 may include a selection of parameters that may be based on whether the parameters indicate viral infection (e.g., COVID-19) as shown at block 730. The selection of parameters may be based on the leading indicators identified at block 720. For example, method 700 may comprise evaluating the volume of individual cells in the subpopulation of monocytes. A standard deviation for the volume measurements for the monocytes (e.g., MDW) can be calculated based on the evaluated monocytes.

At block 740, method 700 comprises calculating the mean of the monocyte population on the axial light loss parameter and the monocyte median angle light scatter standard deviation combining these to construct a composite index as at block 740. An example of this would be:

Monocyte Index = MDW.Value * (monocyte axial light loss mean) ^2 * (monocyte median angle light scatter standard deviation)

At block 750, the composite index value is compared to a predetermined threshold. The threshold may be adjusted to achieve the desired clinical sensitivity and specificity. For example, the monocyte index parameter produced an area under the curve of 0.929. However, as will be understood by those skilled in the art alternative composite indexes, including a monocyte opacity mean index, can be determined. If the composite index generated for a sample is greater than a threshold, block 750 proceeds to block 770. Alternatively, if the composite index generated for a sample is less than or equal to the threshold, block 750 may proceed to block 760. In other words, method 700 may include a determination of whether viral infection (e.g., COVID-19) is likely and proceed to block 670, or unlikely and proceed to block 760. Method 700 may include confirmatory testing as shown in step 780 or may inform treatment decisions as shown in step 790.

It should be understood that the methods as presented do not necessarily have to be conducted in the order presented, and that different sub-combinations of the steps in the methods may be useful independently. For example, although MDW is shown as the third decision block of method 500 (e.g., block 508 in method 500 depicted in FIG. 5), a normal

MDW value alone makes it somewhat less likely that an individual has a viral infection, such as a COVID-19 infection, than if the MDW value is elevated. In addition, other factors may be considered, including, without limitation, the ALL mean for monocytes; the DC mean for monocytes; the standard deviation for ALL for monocytes; the percent basophils (relative to WBC); mean corpuscular hemoglobin concentration (MCHC) or related measures of hemoglobin; Low Hemoglobin Density (LHD value), derived from the mean cell hemoglobin concentration; the standard deviation, in volume, of WBC which may be measured by a nucleated red blood cell (NRBC) module (Whiteinnrbc DC standard deviation); the standard deviation, in volume, of Monocytes and Neutrophils which may be measured by a NRBC module (monograninnrbc DC standard deviation); and percent neutrophils (relative to WBC). In addition to or in lieu of these individual features, certain combinations of features may be of interest, including, without limitation: the ratio between the Lymphocyte Volume Standard Deviation (LY DC SD) and Lymphocyte Percent (LY%) may be higher for COVID-19 positive cases; the ratio between Monocyte Volume Standard Deviation, as reported by the MDW parameter, multiplied by the Monocyte Volume Mean (MO DC MEAN), divided by the Monocyte Opacity Mean (MO OP MEAN) (altogether, MDW * MO DC MEAN/MO OP MEAN) may also or separately be elevated for COVID-19 positive cases.

## METHODS OF DETECTING SEPSIS USING PRIMARY AND SECONDARY PARAMETERS

The present disclosure relates to methods for detecting an infection, including infections resulting in sepsis, by using parameters measured by a hematology analyzer. These methods are useful for understanding the invention. A variety of parameters and vital signs have been used in the detection of sepsis, and it has been observed that sequential applications of parameters may provide improved assessments, and, surprisingly, that this may be the case even when analysis shows that using the same parameters in combination did not show added value over the performance of a first parameter in sequence (e.g., a sequence in which CRP or PCT are evaluated after MDW may provide improved predictive power, even though CRP + MDW or PCT + MDW does not appear to provide added value over MDW alone). Additionally, individual measurements may be combined with particular cut offs. As described herein, this may improve earlier sepsis detection and potentially could reduce time to antibiotics administration. In some instances, particular combinations of individual measurements at coordinated cut off values may improve sepsis detection where use of the same measurements at different cut off values do not improve sepsis detection.

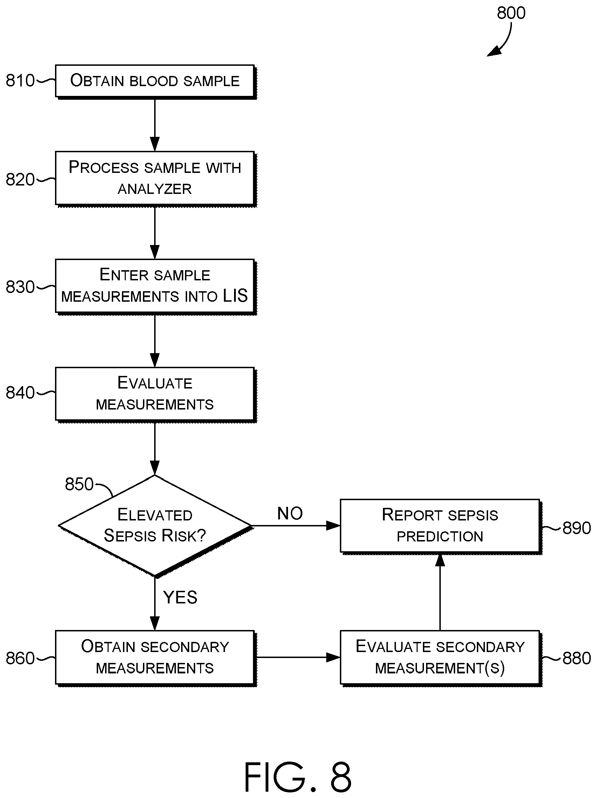

Turning to FIG. 8, and with brief reference to FIGS. 2 and 3, as mentioned above, an analyzer may comprise an analyzer engine (e.g., analyzer engine 400 described in relation to FIG. 4) that includes at least a risk analyzer module (e.g., risk analyzer module 404c described in relation to FIG. 4). As described in more detail below, the risk analyzer module can include rules, models, logic expressions, in any combination that are configured to detect and/or forecast medical conditions. For example, the risk analyzer module can characterize the information received from an analyzer to determine an individual's risk of developing sepsis. Additionally, the risk analyzer module may character the information received from the analyzer to determine a probability of sepsis severity.

In other words, the present disclosure may improve the early detection of sepsis through combinations of measurements. The measurements may be determined by a hematology analyzer. For example, in some implementations, evaluation of a combination of MDW and WBC may be used as a systematic screening test, followed by PCT or CRP measurement in cases of test results which indicate an elevated risk of sepsis. This may be accomplished by an analyzer engine by evaluating these measurements against predetermined criteria, which may be a range of values considered to be abnormal for a healthy adult. For example, an abnormal WBC count may be determined to be equal to the SIRS criteria of a value less than 4,000/mm3 (4.0x103/$\mu$L) or greater than 12,000/mm3 (12.0x103/$\mu$L). Similarly, an abnormal WBC count may be equal to the medical definition of a value less than about 5,000/mm3 and greater than about 10,000/mm3. An abnormal MDW value may be a value greater than 20.0 channels. An abnormal MDW value may be a value based on a type of container used for a sample, with MDW values above 21.5 channels being treated as abnormal for samples collected in K3EDTA anticoagulant (e.g., drawn into K3EDTA tubes), and MDW values above 20.0 channels being treated as abnormal for samples collected in K2EDTA anticoagulant (e.g., drawn into K2EDTA tubes). An abnormal PCT value may be values greater than 0.25 µg/L. An abnormal CRP cut-off may be 22 mg/L. One of skill in the art understands that these cut-offs can be modified to address, for example, specific sub-populations (such as individuals with cancer, pediatric individuals seeking care, etc.) or to modify the sensitivity and/or specificity of the test (e.g., by opening a range to make it more inclusive, or further limiting a range to make it more exclusive).

WBC is a test that measures the number of white blood cells, also called leukocytes, in an individual's body. These cells are important for fighting infections in a body, and an increased WBC number can indicate infection or other underlying conditions in the body, in some instances before an individual presents clinical symptoms or when an individual presents ambiguous clinical symptoms. A normal (non-SIRS) WBC count for a healthy adult can vary between about 5,000 to 10,000 white blood cells per microliter (µl or mcL) or cubic millimeter (mm3) of blood. This is different from the normal count defined by SIRS criteria (4,000 to 12,000 WBC/mcL).

Sub-types of white blood cells may be measured as a differential (CBC-diff), with each sub-type being within a typical percentage of the total WBC: neutrophil (55 to 73 percent), lymphocyte (20 to 40 percent), eosinophil (1 to 4 percent), monocyte (2 to 8 percent), and basophil (0.5 to 1 percent).

Measuring an individual's WBC can require a blood draw, otherwise known as a venipuncture. This procedure, often performed by a phlebotomist, involves the insertion of a small needle into an individual's vein and collecting blood into a 3ml to 10 mL tube. This blood tube may then be transferred to an automated machine that will analyze the sample to determine the number of white blood cells, an embodiment of which is depicted in FIG. 2. In an automated embodiment, in addition to the percentage of each white blood cell type, it is possible to obtain detailed morphological information about the blood cells, such as volume and size. This automated measurement may be based on the direct current (DC) impedance measured from cells in a blood sample. This traditional method, also known as the Coulter Principle, is accomplished by an analyzer through passing an electric current through a blood sample and measuring the number of individual cells based on a change in impedance resulting from the cells passing through a measurement module. Another automated method is a laser flow cytometry system which transmits light through a blood sample. One or more absorption signals are measured, and the resulting light scatter is measured at different angles to determine cell morphology. Another method is fluorescent flow cytometry, which works like flow cytometry but, with the addition of fluorescent reagents, has an extended capability of measuring more specific cell populations and more specific morphological information, such the nucleus-to-plasma ratio of certain cells. Imaging is another method and involves a camera which automatically collects images of stained cells and can use image processing and pattern-recognition techniques to classify the cells automatically or present detailed cell images to a professional for review.

MDW is the standard deviation of monocyte volumes. A monocyte is a type of white blood cell. The monocyte volume parameter may be determined by passing an electric current through a blood sample and measuring the volume of individual cells passing through a measurement module based on measuring the amplitude of the resulting impedance measurement (e.g., in a flow cell 230 of a system such as shown in FIG. 2). This volume may also be measured by a system which transmits light through a blood sample and measures the resulting light scatter to determine cell volume. Methods to detect the presence of sepsis and/or SIRS using WBC population data, including MDW, have been described, for example, in U.S. Provisional Application No. 62/288,091, filed January 28, 2016; PCT Application No. PCT/US2017/014708, filed January 24, 2017; and Park, D.-H., "Screening of sepsis using leukocyte cell population data from the Coulter automatic blood cell analyzer DxH800," Int. Jnl. 15 Lab. Hem., 2011, 33, 391-399.

Analyzer information can be stored directly on an analyzer's software system or collected by a LIS. A LIS is a software-based laboratory information management system and is involved with inputting, processing, and storing a variety of information from analyzers throughout the lab as well as information associated with the individual. This includes the processing and storage of sample measurements associated with an individual, such as MDW or WBC. A LIS may also gather information associated with the individual from an EMR, such as vital sign measurements as well as completed sample measurements from an analyzer. This information may be combined with the information in the LIS and analyzed by the software to make disease state predictions. This analysis may be executed by evaluating whether selected measurements meet a predetermined criterion. Based on which predetermined criteria is met for the inputted measurements, the probability that an individual will develop sepsis may be determined. A prediction report or alert may be sent to a medical professional so that they may decide how to best treat an individual.

In a particular example, performance of various biomarkers was evaluated in a study of 1517 individuals aged 18-89 years who had a CBC with differential test ordered upon presentation to the emergency department (ED) who remained at least four hours. In this study, an additional K3EDTA tube was drawn from participating individuals, together with a sample for PCT and CRP measurement, as well as routine blood tests at the discretion of the treating physician. All blood samples were analyzed on a UniCel® DxH™ 900 analyzer (Beckman Coulter, Inc., Brea, CA) with version 1.0.0.329 software within 2 hours of collection. This instrument measures specific cell volume variables and the distribution of cell volumes within a group of white blood cells (WBC). Quality control was performed daily with COULTER 6C Plus Cell Control to monitor the DxH™ 900 system performance. COULTER LATRON CP-X Control was used as part of the daily quality control procedure, to monitor volume, conductivity, and light scatter measurements. PCT and CRP concentrations were measured on a Cobas analyzer (Roche Diagnostics, Meylan, France) a Liaison XL (Diasorin, Saluggia, Italy) or a AU5800 (Beckman Coulter, Inc, Brea, CA, USA) analyzers, depending on the site. The MDW, PCT and CRP test results that were performed by protocol (not ordered by physicians) were not reported to the treating physicians.

Clinical data at presentation, including past medical history, assessment of vital signs, symptoms, SIRS criteria, qSOFA and SOFA scores microbiological testing and treatments were recorded on an electronic case report form and individuals followed up for at least 12 hours. The clinical research team was blinded to MDW results at the time of clinical data entry and during assignment of the individuals to a clinical category.

Study subjects were categorized by qualified physician or expert based on the "Sepsis-2" consensus criteria, such as non-SIRS (i.e., zero or one SIRS criterion and no infection), SIRS ($\geq$ 2 SIRS criteria and no infection), Infection (suspected or confirmed infection with 0 - 1 SIRS criteria), sepsis (infection plus $\geq$ 2 SIRS) (including sepsis [no organ failures], severe sepsis [sepsis with one or more organ failures], and septic shock [sepsis with refractory hypotension]. Adjudicated categories per Sepsis-3 criteria included controls, infection, and sepsis (based on SOFA score criteria). The presence of infection was determined based on the retrospective chart review of tests performed and clinical data available within the first 12 hours of ED presentation. If no infection work-up was performed within 12 hours, or if the adjudicator believed that the infection work-up showed no evidence of infection, the individual was categorized as "not infected" or SIRS by the adjudicator. Test results were extracted from the records 7-10 days later, including cultures, molecular tests (e.g., polymerase chain reaction and antigens), relevant imaging, and tissue pathology. Subjects who were discharged from the ED within 4 hours had a follow-up at day 30 by a phone call to confirm the ED final diagnosis and exclude the development of sepsis.

In this study, in order to characterize sepsis as being present upon ED admission, sepsis criteria had to be fulfilled within 12 hours of the initial CBC in individuals with suspected infection (as reflected by initiation of diagnostic infection workup) and appropriate clinical categorization was verified by expert review of the extracted electronic medical record data by at least two independent adjudicators at each site. Discordances were arbitrated by a third independent physician reviewer.

Tables 1 and 2, below, provides performances of the biomarkers MDW, WBC, PCT and CRP, both alone and in combination, for diagnosing sepsis in the above described study, using area under ROC curve (AUC) as a measure of how well each parameter or combination of parameters could distinguish between sepsis and non-sepsis individuals.

**Table 1: Performances of MDW, WBC, PCT, CRP alone or in combination for baseline measurement of Sepsis-2**

| Parameter | AUC | SE | Lower (95% Confidence) | Upper (95% Confidence) |
|---|---|---|---|---|
| MDW | 0.81 | 0.01 | 0.78 | 0.84 |
| WBC | 0.76 | 0.02 | 0.72 | 0.79 |
| PCT | 0.78 | 0.02 | 0.75 | 0.81 |
| CRP | 0.85 | 0.01 | 0.83 | 0.87 |
| MDW + WBC | 0.86 | 0.01 | 0.84 | 0.88 |
| MDW + PCT | 0.81 | 0.01 | 0.78 | 0.84 |
| MDW + CRP | 0.85 | 0.01 | 0.82 | 0.87 |
| MDW + WBC + PCT | 0.86 | 0.01 | 0.84 | 0.89 |
| MDW + WBC + CRP | 0.87 | 0.01 | 0.85 | 0.89 |

**Table 2: Performances of MDW, -WBC, PCT, CRP alone or in combination for baseline measurement of Sepsis-3**

| Parameter | AUC | SE | Lower (95% Confidence) | Upper (95% Confidence) |
|---|---|---|---|---|
| MDW | 0.82 | 0.02 | 0.79 | 0.85 |
| WBC | 0.65 | 0.03 | 0.60 | 0.70 |
| PCT | 0.84 | 0.02 | 0.81 | 0.87 |
| CRP | 0.85 | 0.01 | 0.82 | 0.87 |
| MDW + WBC | 0.83 | 0.02 | 0.79 | 0.86 |
| MDW + PCT | 0.82 | 0.02 | 0.79 | 0.86 |

(continued)

| Parameter | AUC | SE | Lower (95% Confidence) | Upper (95% Confidence) |
|---|---|---|---|---|
| MDW + CRP | 0.85 | 0.01 | 0.82 | 0.88 |
| MDW + WBC + PCT | 0.83 | 0.02 | 0.80 | 0.86 |
| MDW + WBC + CRP | 0.85 | 0.01 | 0.82 | 0.87 |

This analysis used a cut off value for CRP of greater than 22 mg/L based on the Youden index, a single statistic that captures the maximum effectiveness of a biomarker. This same analysis also identified a cut off of greater than 21.5 channels for MDW samples, which were drawn into K3EDTA tubes. Cut off values of less than 4,000/mm3 or greater than 12,000/mm3 were used for WBC, and a cut off value of greater than 0.25 $\mu$g/L was used for PCT.

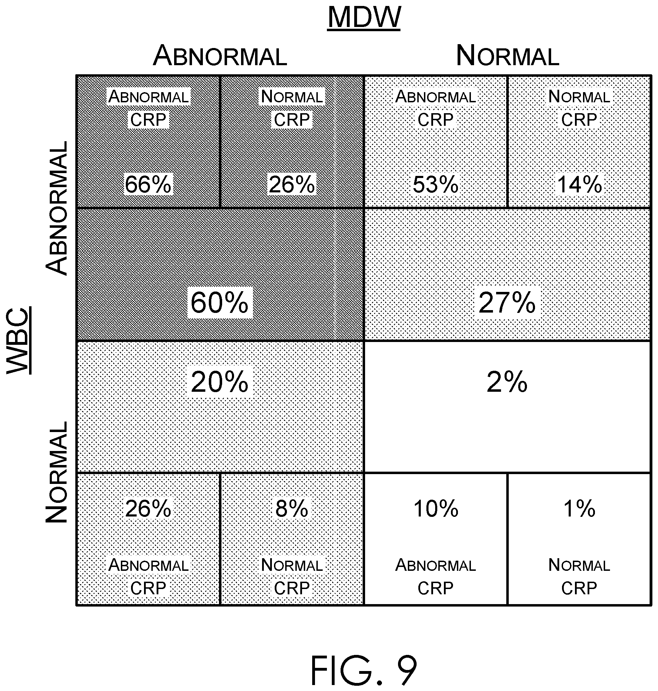

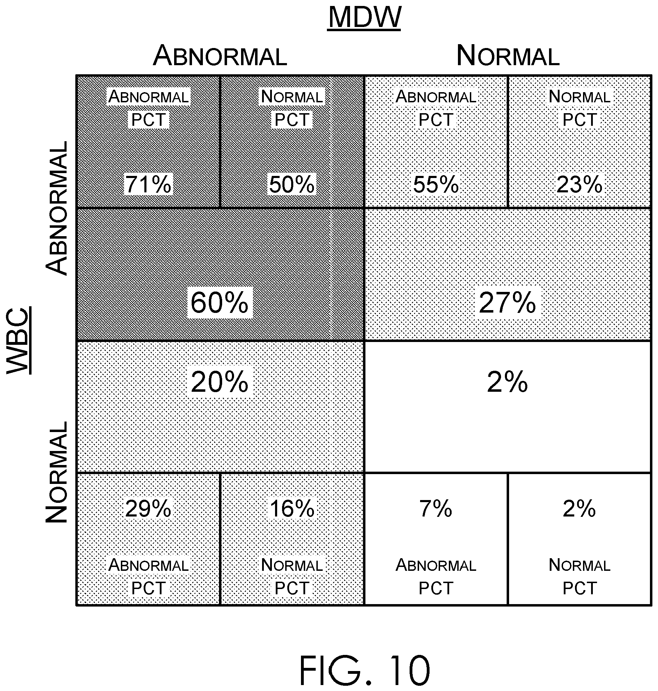

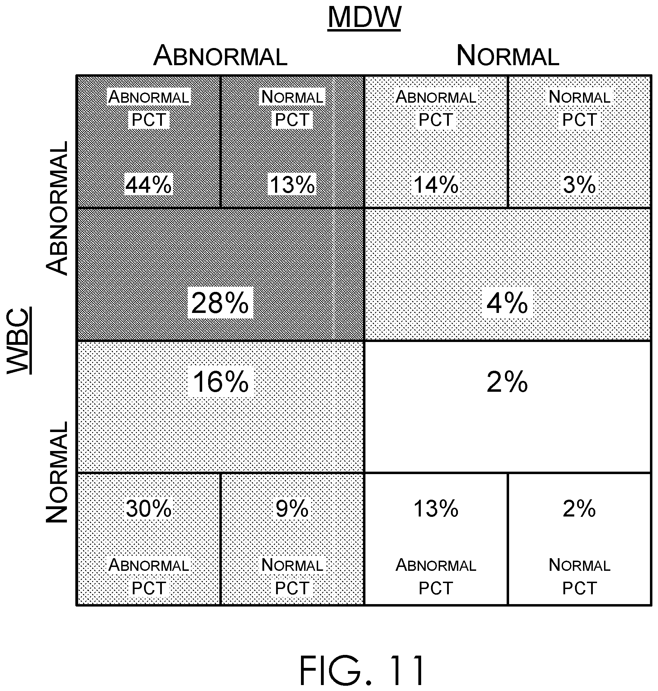

As can be seen in tables 1 and 2, combinations of PCT or CRP with MDW + WBC did not appear to improve diagnostic accuracy for sepsis. Surprisingly though, a sequential approach using MDW and WBC as a systematic screening test, followed by PCT or CRP as a measurement in the case of discordant test results provided improved results for sepsis-2, potentially leading to earlier detection and reduction of time to antibiotic administration. This is illustrated graphically in FIG. 9 and FIG. 10, which show sequential assessment of sepsis probabilities according to WBC and MDW followed by CRP (in FIG. 9) or PCT (in FIG. 10) per sepsis-2 criteria. A second surprising result was that a sequential approach using MDW and WBC as a systematic screening test, followed by PCT or CRP as a measurement in the case both MDW and WBC values were abnormal was also found to increase diagnostic accuracy. This is illustrated graphically in FIG. 10 and FIG. 11, in which FIG. 10 illustrates probability for sepsis-2 increases from 60% to 71% when an abnormal PCT measurement is added to initial abnormal MDW and WBC values, and in which FIG. 11 shows that the probability for sepsis-3 increases from 28% to 44% when an abnormal PCT measurement is added to initial abnormal MDW and WBC measurements. Finally, in this case, an optimal cut off value for MDW drawn into K3EDTA tubes was found to be 21.5 channels, though a cut off value between 20-22.5 channels may be used. Similarly, when measuring MDW drawn into K2EDTA tubes, a cut off within the range of 18.5-21 channels is used in some aspects. In a particular aspect, an optimal cut off value has been found to be 20 channels.

When evaluating CRP, a cut off value between 14-40 mg/L may be used. In this case, an optimal cut off value of 22 mg/L was found based on Youden index analysis. Finally, when evaluating PCT, a range of 0.05-0.25 $\mu$g/L may be used. In this case, though the optimal PCT cut off based on Youden index analysis was found to be 0.12 $\mu$g/L, a cut off value of 0.25 $\mu$g/L was used as it was found to maximize specificity. When combining these parameters, the cutoff values may be adjusted within these ranges. For example, when using MDW as a screening tool, the cutoff value may be lowered to enhance sensitivity. This may then be followed by selecting cut off values for PCT or CRP which enhance specificity.

Returning to FIG. 8, a method 800 for identifying a sample with an elevated risk of developing sepsis based on hematological analysis of the sample. Method 800 may be facilitated, at least in part, by an analyzer (e.g., analyzer 200 of FIG. 2) and an analyzer engine (e.g., analyzer engine 300 of FIG. 3). The analyzer engine may be a module incorporated into the analyzer. Additionally, and or alternatively, method 800 may be facilitated in part by remote device (e.g., remote device 106 of FIG. 1) and a data store (e.g., data store 108 of FIG. 1) that is maintaining the data of an LIS or EMR system. An analyzer engine may be incorporated into a module of the remote device or an application executed by the remote device.

As depicted, method 800 may begin at block 810. At block 810 a blood sample may be obtained from an individual. The sample may be obtained in any way. For example, the blood sample may be collected in a K2EDTA tube or a K3 EDTA tube. This blood sample may then be processed by a hematology analyzer at block 820. In an aspect, the blood sample measurements may include WBC and MDW.

The blood sample parameters may be entered into a laboratory information system (LIS) for processing either manually or automatically at block 830. The LIS may include a processor and a non-transitory computer readable storage medium. The computer readable medium may be programmed with an application to cause the processor to evaluate the sample parameters to determine whether the parameters indicate an elevated sepsis risk at block 840. If no elevated risk is found based on the comparison to the predetermined criteria, aspects of method 800 proceed to block 890.

Alternatively, if an elevated risk is found based on the comparison of the sample parameters to the predetermined criteria, method 800 may proceed to block 860. For example, where the evaluated parameters are MDW and WBC, if at least one of those parameters is abnormal when compared against predetermined criteria (e.g., WBC abnormal + MDW normal, WBC normal + MDW abnormal, or WBC and MDW both abnormal), the parameters may be treated as indicating an elevated sepsis risk. In such an instance, method 800 may proceed to block 860.

Block 860 comprises obtaining secondary parameters for the blood sample. This could be done, for example, by the analyzer performing tests for CRP and/or PCT or by retrieving results of those tests from a data store or local memory, in the case where they had already been performed. Additionally, block 860 may comprise automatically generating an order for a PCT or a CRP test for the individual, or recommending to an attending physician that such a test be performed.

The results of the secondary parameter(s) are then evaluated at block 880. The secondary parameters may be evaluated by comparison with corresponding predetermined criteria, and the results of this evaluation can be used to report 105 a sepsis prediction for the individual.

In an implementation following a process such as described above which uses predefined criteria for evaluating primary and/or secondary parameters, the predetermined criteria may be a range of values considered to be abnormal for a healthy adult, and predictive of developing sepsis alone or in combination with other parameters. In some implementations, these parameters may be stored in an electronic medical record (EMR) and extracted from the EMR by a LIS. Laboratory test results derived from an individual biological sample, such as WBC and MDW, may also be input to the LIS manually, by a laboratory professional, or directly from an analyzer. An analyzer is a clinical diagnostic machine capable of measuring one or more anatomical or physiological properties of a sample, including: metabolic measurements (also referred to as blood chemistry); cell counts; viral protein, viral gene or microbial cell measurements; urine measurements; genomic characterizations; or immunological measurements.

The description of FIG. 8 provided above describes an instance of method 800 in which detection of an elevated risk of sepsis triggers obtaining secondary parameters at block 860 rather than immediately reporting a sepsis prediction at block 890. However, a sufficiently high or otherwise conclusive risk of sepsis may trigger reporting a Sepsis prediction at block 890 rather than obtaining secondary parameters at block 860. For instance, in a case where MDW and WBC are evaluated to determine an elevated risk of sepsis, some instances may simply report at block 890 a sepsis prediction if both MDW and WBC are abnormal relative to predetermined criteria, and may only obtain secondary parameters in the event that the MDW and WBC measurements are discordant (normal MDW + abnormal WBC, or abnormal MDW + normal WBC).

## DETECTION OF MEDICAL CONDITION, SEVERITY, RISK, AND ACUITY USING PARAMETERS

As described in more detail above, an analysis engine includes at least one analyzer that processes the measurements or parameters provided to the analysis engine. This description is useful for understanding the claimed invention. The processing can include rules, models, logic expressions, in any combination that are configured to detect and/or forecast medical conditions. For example, an acuity analyzer (e.g., acuity analyzer 404a described in relation to FIG. 4) may include programs that characterize the information received from an analyzer to determine an acuity for individual. An acuity analyzer (e.g., acuity analyzer 404a described in relation to FIG. 4) may include programs that characterize the information received from an analyzer to: identify individuals for discharge and/or evaluate whether or not an individual is responding to care. An acuity analyzer e.g., acuity analyzer 404a described in relation to FIG. 4) may include programs that characterize the information received from an analyzer to: assess a severity of infection, and/or determine if an individual is at risk of sepsis or shock. An acuity analyzer e.g., acuity analyzer 404a described in relation to FIG. 4) may include programs that characterize the information received from an analyzer to: stratify the risk associated with febrile neonates, assess or predict a risk of systemic infection, assess or predict if the individual is at risk of exhibiting a hyper-inflammatory state, discern if an individual suspected of an infection has a viral infection, discern if an individual suspected of an infection has a bacterial infection, discern whether antibiotic treatment should be provided or not, discern if an individual is experiencing respiratory exacerbations, e.g., cystic fibrosis, related to inflammation and/or infection, assessing a severity of infection of an immune-compromised individual, or a combination thereof.

In certain aspects disclosed herein, methods are provided that are directed to evaluating acuity of an individual. In certain instances, a majority of individuals that arrive at an emergency department (ED) have an uncertain projected clinical course. For instance, in certain scenarios, the majority of individuals entering an ED are likely stable, and the ED will need multiple types of resources, e.g., lab tests and/or imaging, to investigate or treat the individual. In certain instances, such individuals with an uncertain projected clinical course can be classified initially as an emergency severity index level 3. The time required to investigate individuals with an uncertain projected clinical course leads to prolonged wait times and dwell times, and can be a source of ED or care inefficiencies. Furthermore, diagnosing individuals seeking care in an ED can be challenging due to overlapping symptoms for various ailments, including conditions associated with an infection or not. Prolonged wait times and prolonged times to diagnosis may result in adverse outcomes.

The methods disclosed herein can alleviate one or more of the above problems. For instance, in certain aspects, the methods disclosed herein can assess acuity of an individual. In such aspects, evaluating or assessing acuity of an individual can identify individuals early on that may need increased care, e.g., individuals having a risk or elevated risk of sepsis, severe sepsis, or other severe condition, and can end up being diverted to the appropriate care units, such as an critical care facility (e.g., an Intensive Care Unit (ICU)), more efficiently. In the same or alternative aspects, by identifying individuals having a risk of sepsis or severe sepsis early on, via the methods disclosed herein, additional standard of care sepsis tests can then be ordered.

As used herein, acuity generally refers to the level of care an individual needs and can also correlate with a severity of an illness or condition, or a risk of developing or having an illness or condition, even if undiagnosed or no likely suspected illness is known. Specific examples of acuity are described herein, including but not limited to, whether or not an individual needs critical care, the individual is at risk of in-hospital mortality (e.g., within 48 hours of admission), the individual is at risk of sepsis or is at risk of severe infection or other condition. As used herein, the term risk refers to likelihood, where for example, in the context of an individual at risk of particular illness or condition, means it is likely (or more likely than not) that that individual may have the illness or condition, or may develop such illness or condition. As used herein, an elevated risk, in the context of developing or having an illness or condition, refers to a risk of developing or having an illness or condition that is high enough to warrant treatment (e.g., preventative or other treatment) immediately or within 24-48 hours.

In various aspects, the methods disclosed herein can assess acuity of an individual based at least partly on an MDW value from a blood sample associated with an individual. In such aspects, the methods disclosed herein can compare an individual's MDW value with one or more predetermined criteria to evaluate acuity. For example, in certain aspects, as discussed herein, an MDW value may be associated with an elevated risk of needing critical care and/or an elevated risk of in-hospital mortality. In such aspects, independent from a disease, and/or independent from (or prior to) an ultimate diagnosis, the MDW value of the individual alone, or in combination with other markers as discussed herein, may indicate the individual is at risk of needing critical care and/or at risk of in-hospital mortality. In the same or alternative aspects, independent from a disease exhibited by the individual, the methods disclosed herein can determine an individual's disposition, e.g., needing to go to ICU, ready for discharge, etc.

In aspects, by quickly identifying and/or assessing an individual's risk of needing critical care and/or risk of in-hospital mortality, the appropriate resources can be diverted to such individuals in a more efficient and timely manner, which can lead to reduced adverse outcomes. In various aspects, the individual's MDW value is taken or computed within about 6 hours of arrival at an ED, within about 4 hours of arrival at an ED, or within about 2 hours of arrival at an ED.

In various aspects, the methods disclosed herein can identify individuals for discharge. For instance, in certain aspects, one or more MDW values can be compared to one or more predetermined criteria in order to identify an individual as a candidate for discharge. In various aspects, multiple MDW values can be obtained on multiple blood samples over the course of care, or observation. In such aspects, identifying an individual for discharge can aid in freeing up hospital resources, and/or allocate hospital resources more efficiently.

In certain aspects, the methods disclosed herein can include evaluating whether or not an individual is responding to care. For instance, in various aspects, multiple MDW levels can be obtained over the course of care and such MDW levels can be compared to one or more predetermined criteria. When the MDW level returns to a normal or reference level, or is trending in that direction over the course of multiple parameters, it can be determined that the individual is responding positively to care. The converse outcome can also apply, i.e., where the MDW levels stay at the elevated level, or are increasing over the course of care, it can be determined that the individual is not responding positively to the care regimen.

In certain aspects, the methods disclosed herein can aid in assessing a severity of infection. In one example aspect, as discussed herein, one or more MDW values can be compared to one or more predetermined criteria to determine if an individual is at risk of sepsis or shock. In certain aspects, the predetermined criteria can include one or more threshold ranges which can distinguish between a risk to sepsis and a risk to shock.

In various aspects, one or more additional parameters, e.g., additional parameter values from a blood sample, can aid in assessing a severity of infection. In various aspects, the methods disclosed herein for assessing severity of infection can be utilized with febrile neonates, e.g., to stratify the risk associated with such a population. For instance, if a febrile neonate exhibits an elevated MDW level (e.g. compared to one or more predetermined criteria) than it may be determined that their fever symptoms are due to an infection. Further, in such aspects, a severity of infection, if applicable, may be determined for a febrile neonate determined to have a risk of infection, which can lead to stratification of the risk associated with such an individual. In certain aspects, the methods disclosed herein for assessing severity of infection can also be utilized to assess or predict a risk of systemic infection. In various aspects, the methods disclosed herein for assessing severity of infection can also be utilized to assess or predict if the individual is at risk of exhibiting a hyper-inflammatory state.

In aspects, being able to discern a severity of infection or inflammatory state, or a risk thereof, in some cases prior to confirming the presence of infection and/or infection type, can aid in connecting an individual with appropriate type and level of care in a more efficient manner, while diverting scarce hospital resources to individuals at greatest risk of severe infections. In certain aspects, while lactic acid and/or PCT measurements may be ordered to assess an infection, such parameters typically do not come until later in care and/or observation of an individual. According to the aspects disclosed herein, comparing an MDW value to one or more predetermined criteria to assess a severity of infection or suspected infection can be performed early on, leading to an earlier assessment of severity of infection and better outcomes for the individual.

In aspects of the invention, as discussed herein, an individual's MDW value in combination with one or more other parameters, e.g., one or more additional measurement values from a blood sample, can be utilized to discern if an individual suspected of an infection has a viral infection. In such aspects, being able to identify an individual as suspected of having a viral infection may aid in reducing prescription of antibiotics to such individuals which may reduce adverse consequences. An individual's MDW value alone or in combination with one or more other parameters may be utilized to discern if an individual suspected of an infection has a bacterial infection, including but not limited to tuberculosis, a fungal infection, or infection due to a parasite, e.g., malaria. An MDW value alone or in combination with one or more other parameters may be utilized to discern whether antibiotic treatment should be provided or not. In such instances, since such a method relies on an individual's MDW level, which can be obtained early in assessment of the individual, antibiotics could be administered to individuals suspected of a bacterial infection earlier than under normal standard of care, e.g., earlier than waiting for cultures of other detection test results. In the same or alternative aspects, multiple MDW levels of an individual may be obtained and used to detect and/or monitor an infection, e.g., a systemic infection, in an individual. For instance, in such aspects, by determining if the MDW levels are decreasing or increasing it can be determined if the systemic infection is decreasing or increasing, respectively, in severity.

An individual's MDW value alone or in combination with one or more other parameters, e.g., one or more additional measurement values from a blood sample, may be utilized to discern if an individual is experiencing respiratory exacerbations, e.g., cystic fibrosis, related to inflammation and/or infection. An individual's MDW value alone or in combination with one or more other parameters, e.g., one or more additional measurement values from a blood sample, may be utilized to discern if an individual is suffering from pulmonary damage.

In certain aspects, the method disclosed herein can be utilized with individuals who are immune-compromised. An immune-compromised individual is one who has a reduced immune function compared to a healthy individual of similar age. Individuals may be considered immune-compromised for several reasons. For instance, in aspects, an immune-compromised individual have had an organ transplant, prior or current cancer treatment, prior or current HIV infection, or for suffering one or more burns. In such aspects, the medical condition itself may be at least partly the cause of the compromised immune system or immune function. In the same or alternative aspects, one or more treatments for a medical condition may reduce immune activity, such as immune suppressing treatments due to organ transplant. In aspects, an immune-compromised individual may not exhibit parameters from a complete Blood Count (CBC) and/or metabolic parameters within general reference ranges or normal ranges due to their conditions related to immunosuppression. For instance, a white blood cell count (WBC) parameter may be outside a normal range for an immune-compromised individual at least partly due to being immune-compromised (or the associated treatments). In such aspects, the CBC parameters that may generally be utilized in certain prior systems to ascertain the status of a current infection or other conditions may not be useful. It has been unexpectedly determined that, while such conventional parameters, e.g., CBC parameters, may not be useful for ascertaining the status of an infection or other condition (and/or assessing acuity) in an immune-compromised individual in isolation, the MDW parameter can. For example, in certain aspects, an MDW of an immune-compromised individual can be utilized to assess or evaluate acuity of the individual, as discussed generally above. For instance, in certain aspects, methods disclosed herein can assess acuity of an immune-compromised individual. In such aspects, evaluating or assessing acuity of an immune-compromised individual can be based at least partly on an MDW parameter from a blood sample associated with an immune-compromised individual. In such aspects, the methods disclosed herein can compare an immune-compromised individual's MDW parameter with one or more predetermined criteria to evaluate acuity. For example, in certain aspects, as discussed herein, a MDW parameter value may be associated with risk or elevated risk of needing critical care and/or a risk or an elevated risk of in-hospital mortality. In such aspects, independent from (and/or prior to) an ultimate diagnosis, the MDW parameter value of the immune-compromised individual alone, or in combination with other markers as discussed herein, may indicate the immune-compromised individual is at risk of needing critical care and/or at risk of in-hospital mortality. In various aspects, the immune-compromised individual's MDW parameter occurs within about 6 hours of arrival at an ED, within about 4 hours of arrival at an ED, or within about 2 hours of arrival at an ED.

In another example aspect, the methods disclosed herein can aid in assessing a severity of infection of an immune-compromised individual. In one aspect, as discussed herein, one or more MDW value can be compared to one or more predetermined criteria to determine if an immune-compromised individual is at risk of sepsis or shock. In certain aspects, the predetermined criteria can include one or more threshold ranges to distinguish between a risk to sepsis and a risk to shock. In the same or alternative aspects, one or more additional parameters, e.g., additional parameter values from a blood sample, can aid in assessing a severity of infection. In certain aspects, as discussed herein, an MDW value alone or in combination with one or more other parameters, e.g., one or more additional parameter values from a blood sample, can be utilized to discern if an immune-compromised individual suspected of an infection has a viral infection.

In the following, and elsewhere in the specification, various parameter ranges, thresholds, or cutoff values are described. It should be understood that such ranges, thresholds, or cutoff values can be modified to address, for example, specific sub-populations (such as individuals having cancer, pediatric individuals, etc.) or to modify the sensitivity and/or specificity of the test (e.g., by opening a range to make it more inclusive, or further limiting a range to make it more exclusive). In various aspects, as disclosed herein, one or more parameters are described as being compared to one or more predetermined criteria. In such aspects, the predetermined criteria can include the specific parameter ranges, thresholds, and cutoff values described herein, and/or the one or more predetermined criteria may include a range of values considered to be abnormal for a healthy adult or pediatric individual.

As discussed above, in certain aspects, methods disclosed herein can include evaluating an acuity of individual, e.g., based on comparing an individual's MDW value to one or more predetermined criteria. In various aspects, the individual's MDW value can be determined from an individual's blood sample that was obtained within 2 hours, 4 hours, or 6 hours of arrival at a care center, such as an emergency department. In the same or alternative aspects, the MDW value can be determined within 15 minutes, 30 minutes, 1 hour, or two hours of obtaining the blood sample from the individual. In aspects, the one or more predetermined criteria can include one more threshold MDW values. In one aspect, an MDW value above of about 19.0 or above, 20.0 or above, 21.0 or above, or 23.0 or above can be utilized to determined an acuity of the individual. For example, an individual having an MDW value above one or more of the aforementioned thresholds can indicate that the individual is at risk of in-hospital mortality, needing critical care, at risk of sepsis, at risk of severe infection, or the like. In the same or alternative aspects, one or more secondary or parameters, e.g., from a blood sample of an individual may be utilized in determining an acuity of an individual, in addition to an MDW value. In such aspects, any of the below secondary parameters and thresholds may be utilized for a specific condition, outcome, or disposition.

In various aspects, the methods disclosed herein can include assessing whether or not an individual is at risk of needing critical care (or needs critical care). In such aspects, an MDW value of the individual can be compared to one or more predetermined criteria to discern the risk of needing critical care. In various aspects, the individual's MDW value can be determined from an individual's blood sample that was obtained within 2 hours, 4 hours, or 6 hours of arrival at a care center, such as an emergency department. In the same or alternative aspects, the MDW value can be determined within 15 minutes, 30 minutes, 1 hour, or two hours of obtaining the blood sample from the individual. In aspects, the one or more predetermined criteria can include an MDW threshold value. In one aspect, an MDW value above an MDW threshold value of about 19.0 channels or above, or about 20.0 channels or above, can be utilized to determined whether or not the individual needs or is in need of critical care. For example, an individual having an MDW value above one or more of the aforementioned thresholds can indicate that the individual is at risk of needing critical care, or needs critical care.

In aspects, one or more secondary or parameters, e.g., from a blood sample of an individual, may be utilized in assessing whether or not an individual is at risk of needing critical care (or needs critical care), in addition to an MDW value. In aspects, the one or more secondary parameters can include one or more of white blood cell count (WBC), monocyte %, absolute lymphocyte count (ALC), lymphocyte %, absolute neutrophil (ANC), neutrophil %, eosinophil %, procalcitonin (PCT), Lactic Acid, blood urea nitrogen (BUN), sodium (Na), potassium (K), C-reactive protein (CRP), estimated plasma volume status (ePVS). In certain aspects, one or more of the secondary parameters may be determined as part of a CBC, metabolic panel, or other tests known to one of skill in the art. In various aspects, one or more of these secondary parameters may be compared to respective one or more predetermined criteria, e.g., a respective threshold value.

For example, in aspects, measurement values of an individual's WBC, ANC, neutrophil %, lactic acid, CRP, or BUN that are at or above a respective threshold value may be utilized, in combination with an MDW value (and optionally with other secondary parameters), to discern whether or not the individual is at risk of needing, or needs, critical care. In such aspects, the threshold values for WBC can be about $9 \times 10^9$ white blood cells/L or more, or about $10 \times 10^9$ white blood cells/L or more. In aspects, the threshold values for ANC can be about $5.5 \times 10^9$ neutrophils/L or more, or about $6 \times 10^9$ neutrophils/L or more. In aspects, the threshold values for neutrophil % can be about 70 % or more, or about 75 % or more. In various aspects, the threshold values for lactic acid can be about 2 millimoles (mmol)/L or more, or about 2.25 mmol/L or more. In various aspects, the threshold values for CRP can be about 5 milligrams (mg)/L or more, or about 8 mg/L or more. In various aspects, the threshold values for BUN can be about 15 mmol/L or more, about 20 mmol/L or more, or about 21 mmol/L or more.

In various aspects, measurement values of an individual's ALC and/or lymphocyte % that are at or below a respective threshold value may be utilized, in combination with an MDW value (and optionally with other secondary parameters), to discern whether or not the individual is at risk of needing, or needs, critical care. In various aspects, the threshold values for ALC can be about $1.5 \times 10^9$ lymphocytes/L or less, or about $1.2 \times 10^9$ lymphocytes/L or less. In aspects, the threshold values for lymphocyte % can be about 18% or less, or about 16% or less.

In various aspects, the methods disclosed herein can include assessing whether or not an individual is at risk of in-hospital mortality. In one aspect, the methods disclosed herein can include assessing whether or not an individual is at risk of in-hospital mortality within 96 hours, within 72 hours, or within 48 hours of arrival or admission at a hospital, e.g., at an emergency department. In aspects, an MDW value of the individual can be compared to one or more predetermined criteria to discern the risk of in-hospital mortality. In various aspects, the individual's MDW value can be determined from an individual's blood sample that was obtained within 2 hours, 4 hours, or 6 hours of arrival at a care center, such as an emergency department. In the same or alternative aspects, the MDW value can be determined within 15 minutes, 30 minutes, 1 hour, or two hours of obtaining the blood sample from the individual. In aspects, the one or more predetermined criteria can include an MDW threshold value. In one aspect, an MDW value above an MDW threshold value of about 20.0 channels or above, or about 23.0 channels or above, can be utilized to determined whether or not the individual is at risk of in-hospital mortality. For example, an individual having an MDW value above one or more of the aforementioned thresholds can indicate that the individual is at risk of in-hospital mortality. As discussed above, an evaluation of risk of in-hospital mortality can be independent from an ultimate diagnosis of the individual and can be evaluated prior to diagnosing the individual.

In aspects, one or more secondary parameters, e.g., from a blood sample of an individual, may be utilized in assessing whether or not an individual is at risk of in-hospital mortality, in addition to an MDW value. In aspects, the one or more secondary parameters can include one or more of white blood cell count (WBC), monocyte %, absolute lymphocyte count (ALC), lymphocyte %, absolute neutrophil (ANC), neutrophil %, eosinophil %, procalcitonin (PCT), Lactic Acid, blood urea nitrogen (BUN), sodium (Na), potassium (K), C-reactive protein (CRP), estimated plasma volume status (ePVS). In certain aspects, one or more of the secondary parameters may be determined as part of a CBC, metabolic panel, or other tests known to one of skill in the art. In various aspects, one or more of these secondary parameters may be compared to respective one or more predetermined criteria, e.g., a respective threshold value.

For example, in aspects, parameter values of an individual's WBC, ANC, neutrophil %, lactic acid, CRP, or BUN that are at or above a respective threshold value may be utilized, in combination with an MDW value (and optionally with other secondary parameters), to discern whether or not the individual is at risk of in-hospital mortality. In such aspects, the threshold values for WBC can be about 9 x 109 white blood cells/L or more, or about 10 x 109 white blood cells/L or more. In aspects, the threshold values for ANC can be about 5 x 109 neutrophils/L or more, or about 6 x 109 neutrophils/L or more. In aspects, the threshold values for neutrophil % can be about 70 % or more, or about 75 % or more. In various aspects, the threshold values for lactic acid can be about 2 millimoles (mmol)/L or more, or about 2.25 mmol/L or more. In various aspects, the threshold values for CRP can be about 5.3 milligrams (mg)/L or more, or about 8 mg/L or more. In various aspects, the threshold values for BUN can be about 15 mmol/L or more, about 20 mmol/L or more, or about 21 mmol/L or more.

In various aspects, parameter values of an individual's ALC and/or lymphocyte % that are at or below a respective threshold value may be utilized, in combination with an MDW value (and optionally with other secondary parameters), to discern whether or not the individual is at risk of in-hospital mortality. In various aspects, the threshold values for ALC can be about 1.3 x 109 lymphocytes/L or less, or about 1.0 x 109 lymphocytes/L or less. In various aspects, the threshold values for lymphocyte % can be about 18% or less, or about 16 % or less.

In various aspects, the methods disclosed herein can include assessing a severity of infection. For instance, in aspects, as discussed above, the methods disclosed herein can include assessing an individual's risk or elevated risk of developing sepsis, shock, or both. In one example aspect, as discussed herein, one or more MDW values can be compared to one or more predetermined criteria to assess an individual's risk or elevated risk of developing sepsis, shock, or both. In aspects, assessing that a risk of sepsis or shock is associated with a blood sample of an individual can be at least partly based on, sepsis-2 criteria, sepsis-3 criteria, or a combination thereof.

In one aspect, the methods disclosed herein can include assessing an individual's risk or elevated risk of developing sepsis, shock, or both within 48 hours, within 24 hours, within 12 hours, or within 6 hours of arrival or admission at a care facility, e.g., at an emergency department. In aspects, an MDW value of the individual can be compared to one or more predetermined criteria to assess an individual's risk or elevated risk of developing sepsis and/or shock. In various aspects, the individual's MDW value can be determined from an individual's blood sample that was obtained within 2 hours, 4 hours, or 6 hours of arrival at a care center, such as an emergency department. In the same or alternative aspects, the MDW value can be determined within 15 minutes, 30 minutes, 1 hour, or 2 hours of obtaining the blood sample from the individual. In aspects, the one or more predetermined criteria can include an MDW threshold value.

In one aspect, an MDW value above an MDW threshold value in a range of about 18.0 channels to 25 channels, or about 22 channels to 26 channels, about 19.0 channels or above, about 20.0 channels or above, about 21.0 channels or above, or about 23.0 channels or above, can be utilized to assess an individual's risk or elevated risk of developing sepsis and/or shock. For example, an individual having an MDW value above one or more of the aforementioned thresholds can indicate that the individual is at risk of developing sepsis and/or shock. In one aspect, an individual having an MDW value in the range of 22 to 26 channels, or above 23 channels, can be considered to exhibit an elevated risk of developing (or having) sepsis and/or shock. In the same or alternative aspects, an individual having an MDW value of about 20 channels or more, may be considered to exhibit a risk of developing (or having) sepsis and/or shock. In various aspects, an individual exhibiting an MDW value in a range of 19 channels or more, or 20 channels or more may be considered at risk of developing organ failure. In aspects, an evaluation of the risk of developing (or having) sepsis and/or shock can be independent from an ultimate infection diagnosis of the individual and/or can be evaluated prior to such a diagnosis.

In aspects, one or more secondary parameters, e.g., from a blood sample of an individual, may be utilized in assessing an individual's risk or elevated risk of developing (or having) sepsis and/or shock, in addition to an MDW value. In aspects, the one or more secondary parameters can include one or more of white blood cell count (WBC), monocyte %, absolute lymphocyte count (ALC), lymphocyte %, absolute neutrophil (ANC), neutrophil %, eosinophil %, procalcitonin (PCT), Lactic Acid, blood urea nitrogen (BUN), sodium (Na), potassium (K), C-reactive protein (CRP), estimated plasma volume status (ePVS). In certain aspects, one or more of the secondary parameters may be determined as part of a CBC, metabolic panel, or other tests known to one of skill in the art. In various aspects, one or more of these secondary parameters may be compared to respective one or more predetermined criteria, e.g., a respective threshold value.

For example, in aspects, parameter values of an individual's WBC, ANC, neutrophil %, lactic acid, CRP, or a combination thereof that are at or above a respective threshold value may be utilized, in combination with an MDW value (and optionally with other secondary parameters), to assess an individual's risk or elevated risk of developing (or having) sepsis and/or shock. In such aspects, the threshold values for WBC can be about 7 x 109 white blood cells/L or more. In aspects, the threshold values for ANC can be about 5.5 x 109 neutrophils/L or more, or about 6 x 109 neutrophils/L or more. In aspects, the threshold values for neutrophil % can be about 70 % or more, or about 75 % or more. In various aspects, the threshold values for lactic acid can be about 2 millimoles (mmol)/L or more, or about 2.1 mmol/L or more. In various aspects, the threshold values for CRP can be about 5 milligrams (mg)/L or more, or about 8 mg/L or more.

In various aspects, parameter values of an individual's lymphocyte % that are at or below a respective threshold value may be utilized, in combination with an MDW value (and optionally with other secondary parameters), to assess an individual's risk or elevated risk of developing sepsis and/or shock. In aspects, the threshold values for lymphocyte % can be 19 % or less, 18 % or less, 15 % or less, or 13 % or less. In the same or alternative aspects, the threshold values for lymphocyte % can be 19 % or less, or 13 % or less in the case of sepsis-3 criteria, and/or 18 % or less, or 15 % or less in the case of sepsis-2 criteria.

In certain aspects, the WBC parameter and threshold values, the ANC parameter and threshold values, the neutrophil % parameter and threshold values, the lymphocyte % parameter and threshold values, the CRP parameter and threshold values, or combinations thereof may be utilized in assessing the risk of sepsis or shock, according to the sepsis-2 criteria. In the same or alternative aspects, the lymphocyte % parameter and threshold values, the neutrophil % parameter and threshold values, the lactic acid parameter and threshold values, or a combination thereof may be utilized in assessing the risk of sepsis or shock, according to sepsis-3 criteria.

In various aspects, one or more of the above threshold values can be modified for various subpopulation, e.g., pediatric individuals.

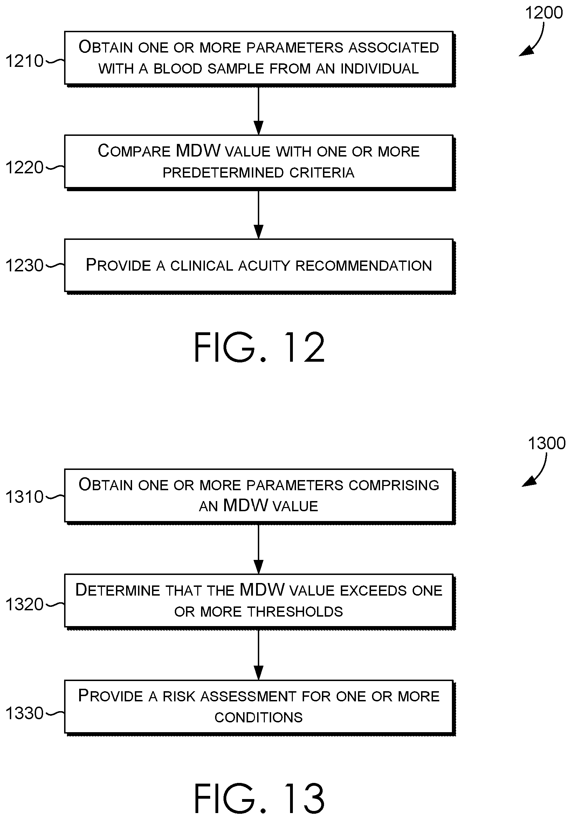

Turning to FIG. 12, a method 1200 for evaluating acuity of an individual is depicted. The individual may be immune-compromised. Method 1200 may be implemented or facilitated at least in part by one or more components of a hematology analyzer (e.g., analyzer 200 of FIG. 2). At block 1210, one or more parameters associated with a blood sample from an individual, including at least MDW, are obtained. Delivering at least a portion of the blood sample to an interrogation zone of a direct current element, an optical element, or a radio frequency element.

The one or more parameters associated with an individual may further comprise one or more secondary parameters comprising white blood cell count (WBC), monocyte %, absolute lymphocyte count (ALC), lymphocyte %, absolute neutrophil (ANC), neutrophil %, procalcitonin (PCT), Lactic Acid, blood urea nitrogen (BUN), sodium (Na), potassium (K), or C-reactive protein (CRP). The one or more secondary parameters may comprise lymphocyte %, and the corresponding one or more predetermined criteria for lymphocyte % is a threshold level of 18% or less. The one or more secondary parameters may comprise BUN, and the corresponding one or more predetermined criteria for BUN is a threshold level of 15 mmol/L or more. The one or more secondary parameters may comprise BUN, and wherein the corresponding one or more predetermined criteria for BUN is a threshold level of 15 mmol/L or more. The one or more secondary parameters may comprise PCT, and the corresponding one or more predetermined criteria for PCT is a threshold level of 0.25 $\mu$g/L or more. The one or more secondary parameters may comprise eosinophil %, and the corresponding one or more predetermined criteria for eosinophil % is a threshold level of 1.5% or more.

At block 1220, the MDW value is compared to one or more predetermined criteria. Comparing the MDW value with one or more predetermined criteria may comprise determining if the MDW value exceeds an MDW threshold value. The MDW threshold value, may be based at least in part on one or more additives associated with a container used for the blood sample. The container may be a K2EDTA container or a K3EDTA container. For exemplary purposes only, the MDW threshold may be 20.0 channels or 21.5 channels, or the MDW threshold may be in a range of 18.0 channels to 25 channels.

At block 1230, a clinical acuity recommendation is provided, at least partly in response to the comparing of the MDW with the one or more predetermined criteria. In method 1200, providing the clinical acuity recommendation may comprise identifying a level of clinical care for the individual. The level of clinical care may be associated with whether or not the individual is at risk of needing critical care, in-hospital mortality within 48 hours, sepsis that warrants immediate or near-immediate intervention, or a combination. A recommended disposition may be provided based on evaluating the acuity. The recommended disposition may comprise, for example, admission to a critical care facility, non-critical care hospitalization, or discharge. Further, an indication that the individual is at risk of infection, sepsis, or shock may be provided. Providing the clinical acuity recommendation may be additionally based on comparing the one or more secondary parameters to corresponding one or more predetermined criteria.

Method 1200 may be stored as executable instructions in a non-transitory storage medium. When executed by a processor, the instructions cause the processor to perform operations similar to those described above.

Method 1200 can be carried out by an analyzer system that comprises a processor and non-transitory storage medium that stores the executable instructions. The instructions can cause the processor to perform operations similar to those described above. The analyzer may further comprise an optical element comprising an interrogation zone adapted to receive a hydro dynamically focused stream of blood samples. The optical element can determine a monocyte volume measure based on measurements of cells as they individually pass through the interrogation zone. The analyzer may further comprise a DC element comprising an interrogation zone adapted to receive a hydro dynamically focused stream of blood samples. The DC element can determine a monocyte volume measurement based on impedance measurements of cells as they individually pass through the interrogation zone.

Turning to FIG. 13, a method 1300 for assessing a severity of infection associated with a blood sample obtained from an individual is depicted. The individual may be immune-compromised. At block 1310, one or more parameters associated with the blood sample are obtained. Obtaining one or more parameters comprises delivering at least a portion of the blood sample to an interrogation zone of: an optical element; a direct current element; a radio frequency element; or a combination thereof. The blood sample may be obtained from an individual that has an infection or suspected infection. The one or more parameters comprise monocyte distribution width (MDW). The one or more parameters may comprise one or more secondary parameters that include at least one parameter selected from a group that includes white blood cell count (WBC), monocyte %, absolute lymphocyte count (ALC), lymphocyte %, absolute neutrophil (ANC), neutrophil %, procalcitonin (PCT), Lactic Acid, blood urea nitrogen (BUN), sodium (Na), potassium (K), or C-reactive protein (CRP).

Comparing the one or more secondary parameters may comprise determining whether an eosinophil % exceeds a threshold value of 1.5%. The one or more secondary parameters may comprise lymphocyte %, and wherein the corresponding one or more predetermined criteria for lymphocyte % is a threshold level of 18% or less. The one or more secondary parameters may comprise ANC, and wherein the corresponding one or more predetermined criteria for ANC is a threshold level of 5.5 x 109 neutrophils/L or more. The one or more secondary parameters may comprise PCT, and wherein the corresponding one or more predetermined criteria for PCT is a threshold level of 0.25 $\mu$g/L or more. The one or more secondary parameters may comprise lactic acid, and wherein the corresponding one or more predetermined criteria for lactic acid is a threshold level of 2 mmol or more.

At block 1320, it is determined that the MDW value exceeds one or more thresholds. The one or more thresholds may comprise a threshold in a range of 18.0 channels to 25 channels, or 22 channels to 26 channels. Additionally, the threshold can vary based at least in part on one or more additives associated with a container used for the sample from which the MDW parameters were obtained. For example, a blood sample collected in a K2EDTA tube can have a first threshold and a blood sample collected in a K3EDTA tube can have a second threshold. The one or more secondary parameters may be compared with a corresponding one or more predetermined criteria. This may include comparing a WBC value to a corresponding one or more predetermined criteria, and comparing a PCT value, a CRP value, or both, to corresponding one or more predetermined criteria. Comparing the one or more secondary parameters with a corresponding one or more predetermined criteria is performed at least partly based on the determining that the MDW value exceeds the one or more thresholds.

At block 1330, at least partly based on determined that the MDW value exceeds one or more thresholds, a risk assessment is provided for one or more conditions associated with the infection or the suspected infection for the individual. Providing the risk assessment may comprise providing a risk assessment of sepsis, shock, organ failure, or a combination thereof. The risk may be an elevated risk indicating immediate or near immediate intervention is warranted. The providing the risk assessment is at least partly based on sepsis-2 criteria, sepsis-3 criteria, or a combination thereof. The one or more thresholds may comprise a threshold in a range of 18.0 channels to 25 channels, and the providing the risk assessment comprises assessing that a risk of sepsis is associated with the blood sample. The one or more thresholds may comprise a threshold in a range of 22 channels to 26 channels, and the assessing comprises assessing that a risk of shock is associated with the blood sample.

Responsive to providing a risk assessment for one or more conditions associated with the infection or the suspected infection for the individual (e.g., organ failure, sepsis shock) a recommendation is generated for disposition of the individual. The disposition may include, for example, transferring the individual to a critical care unit.

Method 1300 may be stored as executable instructions in a non-transitory storage medium. When executed by a processor, the instructions cause the processor to perform operations similar to those described above.

Method 1300 may be carried out by an analyzer system that comprises a processor and non-transitory storage medium that stores the executable instructions. The instructions can cause the processor to perform operations similar to those described above. The analyzer may further comprise an optical element comprising an interrogation zone adapted to receive a hydro dynamically focused stream of blood samples. The optical element can determine a monocyte volume measure based on measurements of cells as they individually pass through the interrogation zone. The analyzer may further comprise an DC element comprising an interrogation zone adapted to receive a hydro dynamically focused stream of blood samples. The DC element can determine a monocyte volume measure based on impedance measurements of cells as they individually pass through the interrogation zone. The individual having a blood sample obtained may be immune-compromised.

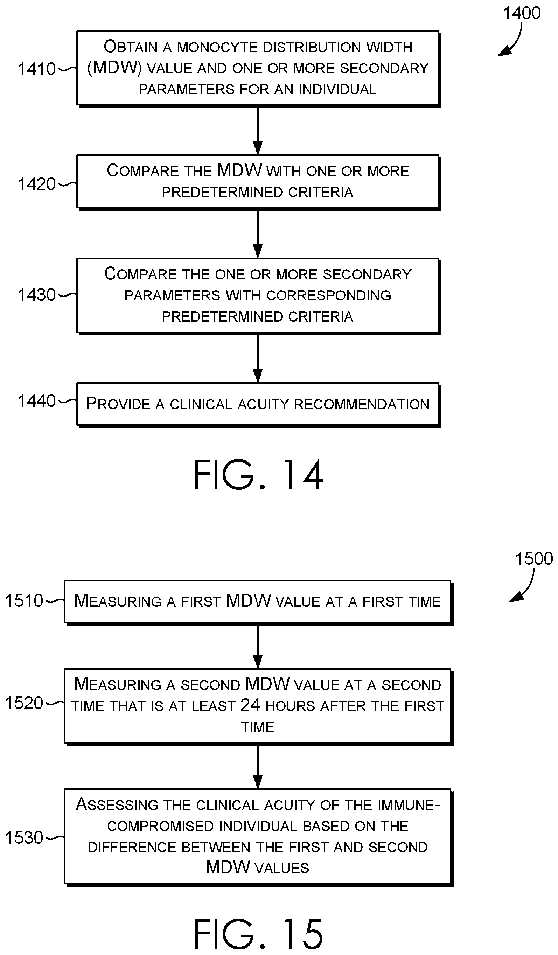

Turning to FIG. 14, a method 1400 for providing clinical decision support information comprising one or more clinical acuity recommendations to a clinician is depicted. At block 1410, an MDW value and one or more secondary parameters for an individual are obtained from one or more blood samples from the individual. The individual may be immune-compromised.

At block 1420, the MDW value is compared with one or more predetermined criteria. The one or more predetermined criteria may comprise a threshold. For example, the threshold may be in a range of 18.0 channels and 25 channels. Alternatively, the MDW range could be 20.0 channels or 21.5 channels. Additionally, the threshold can vary based at least in part on one or more additives associated with a container used for the sample from which the MDW values were obtained. For example, a blood sample collected in a K2EDTA tube can have a first threshold and a blood sample collected in a K3EDTA tube can have a second threshold.

At block 1430, the one or more secondary parameters are compared to a corresponding predetermined criteria. The one or more secondary parameters include at least one parameter selected from a group that includes white blood cell count (WBC), monocyte %, absolute lymphocyte count (ALC), lymphocyte %, absolute neutrophil (ANC), neutrophil %, procalcitonin (PCT), Lactic Acid, blood urea nitrogen (BUN), sodium (Na), potassium (K), or C-reactive protein (CRP). The corresponding predetermined criteria may be a threshold. For example, in an instance that includes CRP as a secondary parameter the predetermined threshold corresponding to CRP may be in a range of 14 and 40 mg/L. For another example, in an instance that includes WBC as a secondary parameter the predetermined threshold corresponding to WBC can be in less than or equal to 4,000/mm3 or greater than or equal to 12,000/mm3. For another example, in an instance that includes PCT as a secondary parameter the predetermined threshold corresponding to PCT can be 0.25 $\mu$g/L.

The one or more secondary parameters may comprise lymphocyte %, and the corresponding one or more predetermined criteria for lymphocyte % is a threshold level of 18% or less. The one or more secondary parameters may comprise BUN, and the corresponding one or more predetermined criteria for BUN is a threshold level of 15 mmol/L or more. The one or more secondary parameters may comprise BUN, and wherein the corresponding one or more predetermined criteria for BUN is a threshold level of 15 mmol/L or more. The one or more secondary parameters may comprise PCT, and the corresponding one or more predetermined criteria for PCT is a threshold level of 0.25 $\mu$g/L or more. The one or more secondary parameters may comprise eosinophil %, and the corresponding one or more predetermined criteria for eosinophil % is a threshold level of 1.5% or more.

At block 1440, a clinical acuity recommendation is provided, where the clinical acuity recommendation is at least partly based on a comparison of the MDW value with one or more predetermined criteria and the one or more secondary parameters with a corresponding predetermined criteria. In block 1440, providing the clinical acuity recommendation may include generating a recommendation for disposition of the individual, which may include identifying a level of clinical care for the individual. The level of clinical care is associated with whether or not the individual is at risk of: needing critical care, in-hospital mortality within 48 hours, sepsis warranting immediate or near-immediate medical intervention, or a combination thereof. For example, the recommendation can be to move the individual to a critical care unit. As another example, the recommendation can be to discharge the individual. As another example, the recommendation can be to increase observation of the individual for a predetermined period of time or order additional medical tests for the individual, or to move the individual to non-critical care hospitalization.

In instances where a sample exhibits an MDW value of 20 channels or more, the one or more secondary parameters may comprise a neutrophil % of 75% or more, a BUN of 21 mmol/L or more, a CRP of 8 mg/L or more, a lactic acid of 2.25 mmol/L or more, an ANC of 5.5 x 109 neutrohils/L or more, an ALC of 1.3 x 109 lymphocytes/L or less, a WBC of 9 x 109 white blood cells/L or more, or a combination thereof.

Method 1400 may be stored as executable instructions in a non-transitory storage medium. When executed by a processor, the instructions cause the processor to perform operations similar to those described above.

Method 1400 may be carried out by an analyzer system that comprises a processor and non-transitory storage medium that stores the executable instructions. The instructions can cause the processor to perform operations similar to those described above. The analyzer may further comprise an optical element comprising an interrogation zone adapted to receive a hydro dynamically focused stream of blood samples. The optical element can determine a monocyte volume measure based on measurements of cells as they individually pass through the interrogation zone. The analyzer may further comprise a DC element comprising an interrogation zone adapted to receive a hydro dynamically focused stream of blood samples. The DC element can determine a monocyte volume measure based on impedance measurements of cells as they individually pass through the interrogation zone.

Turning to FIG. 15, a method 1500 for assessing clinical acuity of an immune-compromised individual. At block 1510, a first MDW value is measured or obtained at a first time. At block 1512, a second MDW value is measured or obtained at a second time. The second time may be at least 24 hours after the first time, such that a difference between the first and second MDW values may be determined. At block 1530, clinical acuity is assessed of the immune-compromised individual based on the difference between the first and second MDW values.

Aspects of the systems and methods disclosed herein may be further understood by reference to the following non-limiting examples.

## EXAMPLES

A single site observational study was conducted on individuals admitted to an emergency department (ED) in the United States.

Over approximately one month, 8,875 blood samples were collected from individuals seeking care in an ED meeting the following inclusion criteria: all adult (> 18 years) individuals who received a complete blood count (CBC) in the ED as part of routine practice. Blood samples for the CBC were collected in EDTA collection vials and Monocyte Distribution Width (MDW) measurements were made using waste sample leftover from the CBC measurements. MDW was obtained using a Beckman Coulter UniCel DxH 900 analyzer and recorded within two hours of blood sample collection, e.g., within two hours of venipuncture.

After removal of samples due to errors, artifacts, and/or late measurements, (e.g., more than 2 hours after sample collection) 7,242 individual MDW parameters included in this study. 5,428 MDW parameters corresponded to samples for individuals seeking care in the ED collected less than or equal to six hours of ED arrival, 1,297 MDW parameters corresponded to collection of sample at six hours or more from ED arrival, and 1,465 serial samples were measured over the course or an individual's stay at the hospital.

MDW parameters were evaluated in relation to the ED disposition outcomes, which may be associated with severity of illness. The outcomes were discerned after 48 hours from obtaining the blood sample, and/or arrival in the ED. Table 3 below details the ED disposition outcomes evaluated.

**Table 3: Outcomes Evaluated**

| Outcomes | Definition |
|---|---|
| Illness Severity | |
| Mortality | In-hospital mortality |
| Emergency Surgery | Operating Room within 12 hours of disposition |
| Critical Care | Any direct admission to the Intensive Care Unit (ICU) or in-hospital mortality |
| Hospitalization | Admitted to the hospital |
| Organ Failure | Any in-hospital mortality, ICU admission, high-flow oxygen, Noninvasive positive-pressure ventilation (NIPPV), mechanical ventilation, vasopressor requirement |
| Organ Dysfunction | Any SpO2 < 88%, oxygen > 2L nasal cannula (NC), respiratory(resp.) rate > 24 for > 30 min., systolic blood pressure (sbp) < 80 or heart rate > 125 for > 30 min, elevated troponin, 72-hr return requiring hospitalization |
| Sepsis II (2) | |
| Non-SIRS and no infection | Non-SIRS is < 2 SIRS criteria, or with no microbial testing ordered |
| Non-SIRS with infection | Non-SIRS with final ED diagnostic codes that include a specific infection, or with any microbial testing ordered |
| SIRS and no infection | 2 or more SIRS criteria met without diagnostic codes corresponding to infection |
| Sepsis | 2 or more SIRS criteria met with final ED diagnostic codes that include sepsis or specific infection or with microbial testing ordered |
| Severe Sepsis | Sepsis with one or more organ failure: Final ED diagnostic codes include severe sepsis or meets criteria for sepsis (above) *plus* ED diagnostic codes for organ dysfunction *or* laboratory evidence of acute organ dysfunction (SBP <90 mmHg, MAP < 65 mmHg, lactate >2 mmol/L, SpO2 <90% on room air, creatinine >2 mg/dL without renal insufficiency or chronic dialysis, bilirubin >2 mg/dL without liver disease or cirrhosis, international normalized ratio >1.5 without anticoagulation) |
| Septic Shock | Final ED diagnostic codes include septic shock or meets criteria for sepsis above *plus* lactate >2 and vasopressors initiated |
| Sepsis III (3) | |
| Sepsis | Presumed serious infection (blood culture obtained, regardless of result, and antibiotics x 4 days-starting within $\pm 2$ days of blood culture day) plus acute organ disfunction (any one of the following criteria $\pm 2$ days of blood culture day: [1] vasopressor initiation, [2] initiation of mechanical ventilation, [3] doubling in serum creatinine level, [4] decrease by 50% of estimated glomerular filtration rate relative to baseline) |

(continued)

| Sepsis III (3) | |
|---|---|
| Septic Shock | Final ED diagnostic codes include septic shock or meets criteria for sepsis above plus lactate >2 and vasopressorst initiated |
| Sepsis-3 Exclusions<br>Individuals with end-stage kidney disease: this will be defined as individuals with bilirubin level >2.0mg/dL and doubling from baseline, platelet count <100 cells/$\mu$L and >50% decline from baseline (baseline must be >100 cells/$\mu$L), serum lactate> 2.0 mmol/Ld | |
| Viral Positivity | |
| Influenza | Positive/Negative laboratory test result |
| SARS-CoV-2 | Positive/Negative laboratory test result |

Univariate analysis was performed with respect to MDW, optionally select other CBC markers, and optionally select metabolic panel parameters in relation to the various outcomes described above. Cohort sub-populations that were used in various aspects of the analyses described below include the total cohort being the total number of MDW measured, sub-population cohorts being: any infection; viral infection, COVID-19; Influenza, no infection; immune-suppressed. As discussed above, immune-suppressed individuals may exhibit one or more conditions, e.g., cancer, HIV, individuals with burns, organ transplant individuals where conventional infection biomarkers may not be useful.

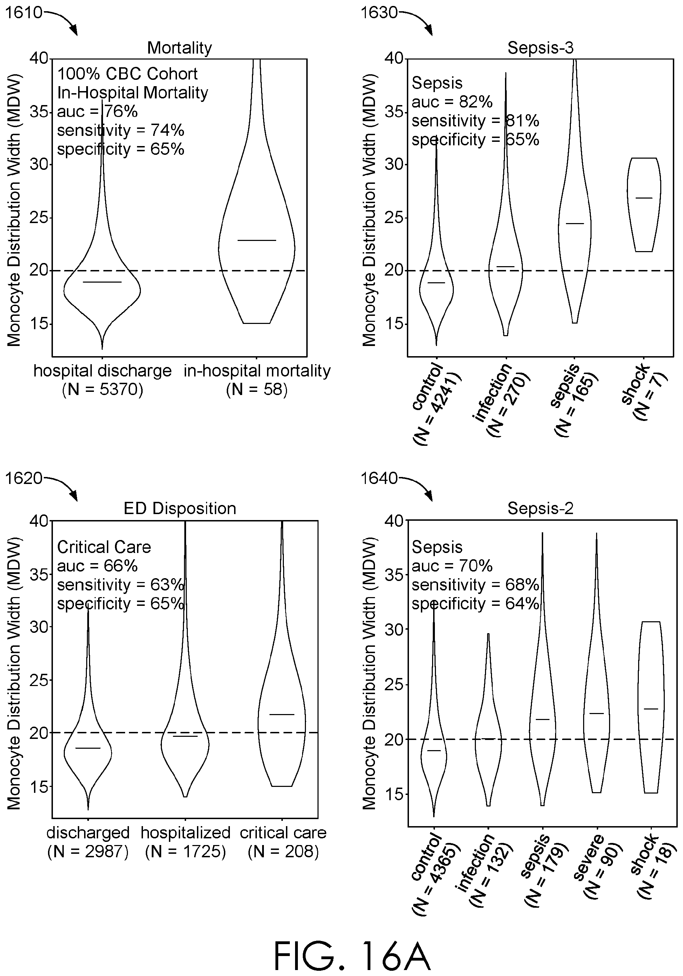

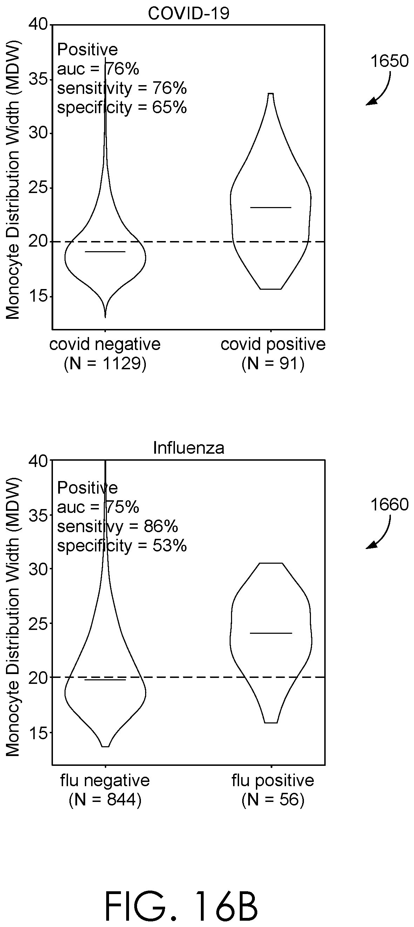

FIGS. 16A and 16B depict violin plots of MDW parameters for various individuals and outcomes. Violin plots visualize the distribution of the data and its probability density and are known to one of skill in the art. Plots 1610 and 1620 display general severity outcomes with the plot 1610 depicting mortality and discharge outcomes for 100% of the full CBC cohort in this study, and the plot 1620 depicting critical, hospitalized, and discharge outcomes. As can be seen, the plot 1610, the MDW parameters of individuals with in-hospital mortality were unexpectedly increased relative to the individuals that were ultimately discharged. The area under the curve (AUC), of a receiver operating characteristic (ROC) curve for the data in the plot 1610 is 76%, with a sensitivity of 74% and a specificity of 65%. Further, as can be seen in the plot 1620, the MDW parameters for individuals needing critical care exhibited an MDW parameter that was increased relative to the individuals that were hospitalized (not in the ICU) and relative to the individuals discharged. The AUC for the data in the plot 120 is 66% with a sensitivity of 63% and a specificity of 65%.

Plots 1630 and 1640 generally display infection and severity outcomes. For instance, the plot 1630 is a violin plot of the MDW parameters for a control group, an infection group, a sepsis group, and a shock group (with each group exclusive of the other). As can be seen in the plot 1630, the MDW parameters were unexpectedly increased for the sepsis and shock groups relative to the infection and control groups, with an AUC of 82%, a sensitivity of 81%, and a specificity of 65%. The plot 1640 is a violin plot of the MDW parameters for a control group, an infection group, a sepsis group, a sever sepsis group, and a shock group (with each group exclusive of the other). As can be seen in the plot 1640, the MDW parameters were unexpectedly increased for the sepsis, sever sepsis, and shock groups relative to the infection and control groups, with an AUC of 70%, a sensitivity of 68%, and a specificity of 64%.

Plots 1650 and 1660 generally display viral infection outcomes. For instance, the plot 1650 is a violin plot of the MDW parameters for Covid positive and Covid negative groups. As can be seen in the plot 1650, the MDW parameters were unexpectedly increased for the covid positive group relative to the covid negative group, with an AUC of 75%, a sensitivity of 76%, and a specificity of 65%. The plot 1660 is a violin plot of the MDW parameters for influenza positive and influenza negative groups. As can be seen in the plot 1660, the MDW parameters were unexpectedly increased for the sepsis, sever sepsis, and shock groups relative to the infection and control groups, with an AUC of 70%, a sensitivity of 68%, and a specificity of 64%.

The data in this Example and in particular as shown in FIGS. 16A and 16B, MDW parameters within hours, e.g., within 6 hours, of being admitted to an ED are a statistically significant marker for assessing acuity of an individual and/or assessing the risk an individual may develop one or more significant outcomes.

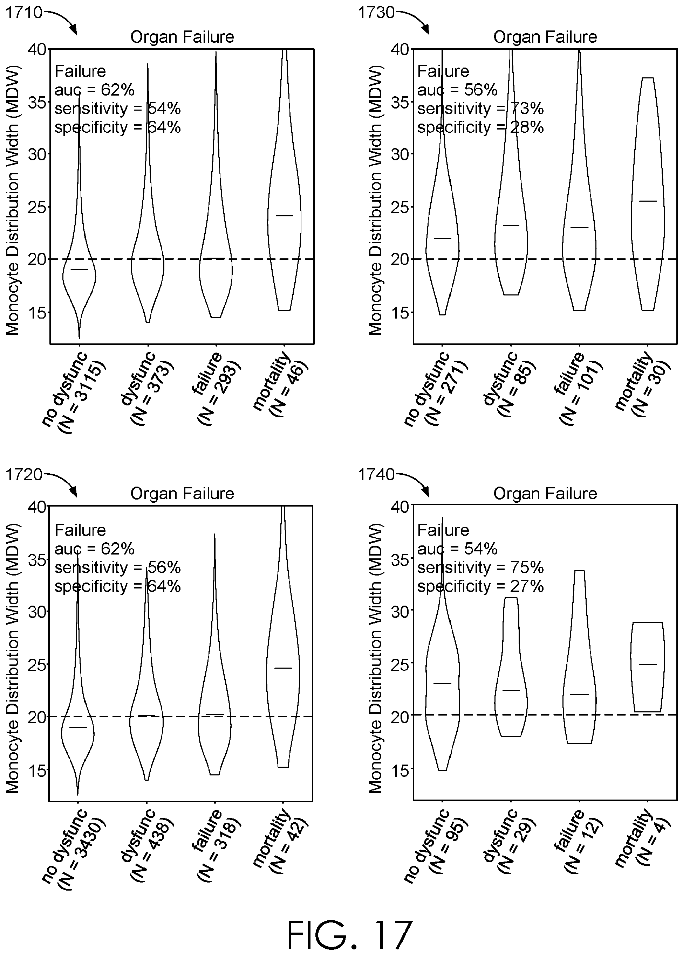

FIG. 17 depicts violin plots of MDW parameters for various individuals and outcomes associated with organ failure. Plot 1710 displays the total CBC cohort of the individuals in the study grouped by having no organ dysfunction, organ dysfunction, organ failure, or in-hospital mortality. As can be seen in the organ dysfunction group and organ failure group (and the in-hospital mortality group) exhibited an increased MDW parameter relative to the no dysfunction group. The AUC for the organ failure group was 62%, with a sensitivity of 54%, and a specificity of 64%.

Plot 1720 displays the no infection cohort of the individuals in the study grouped by having no organ dysfunction, organ dysfunction, organ failure, or in-hospital mortality. As can be seen in the organ dysfunction group and organ failure group (and the in-hospital mortality group) exhibited an increased MDW parameter relative to the no dysfunction group. In the data of the plot 1720, the AUC for the organ failure group was 62%, with a sensitivity of 56%, and a specificity of 64%.

Plot 1730 displays the any infection cohort of the individuals in the study grouped by having no organ dysfunction, organ dysfunction, organ failure, or in-hospital mortality. For the data of the plot 1730, the AUC for the organ failure group was 56%, with a sensitivity of 73%, and a specificity of 28%. Plot 1740 displays the viral infection cohort of the individuals in the study grouped by having no organ dysfunction, organ dysfunction, organ failure, or in-hospital mortality. For the data of the plot 1740, the AUC for the organ failure group was 54%, with a sensitivity of 75%, and a specificity of 27%.

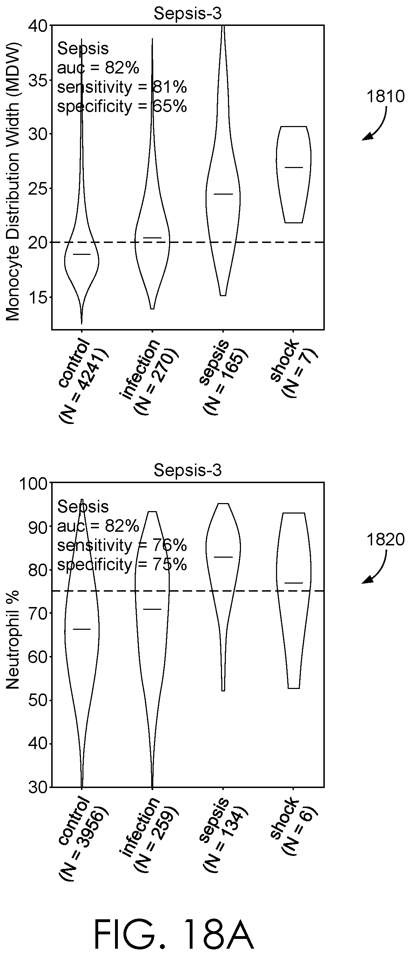

FIGS. 18A-18D depict a series of markers (CBC and/or metabolic) and their relationship with a sepsis-3 outcome for various individual groups (control, infection group, sepsis group, and shock group - all groups exclusive of one another). FIG. 18A includes the plots 1810 and 1820 depicting the data for the MDW and neutrophil % markers, respectively. The plot 1810 is identical to the plot 1630 of FIG. 16A discussed above. As can be seen in the plot 1820, the neutrophil % for the sepsis and shock groups exhibit an increased value compared to the infection and control groups. For the data of the plot 1820, the AUC for the sepsis group is 82%, with a sensitivity of 76% and a specificity of 75%.

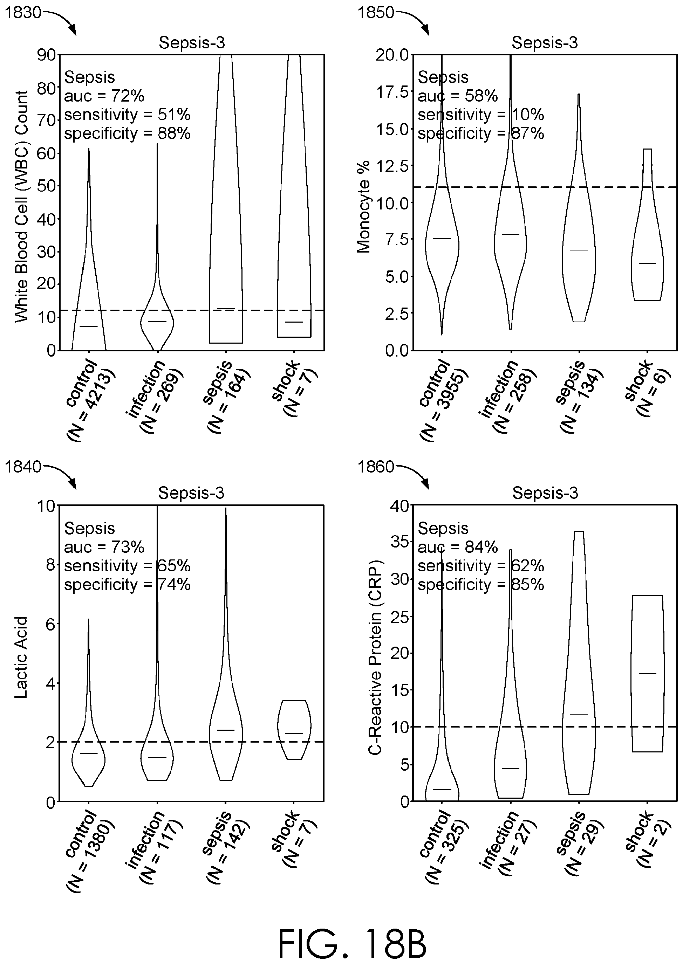

FIG. 18B depicts the plots 1830, 1840, 1850, and 1860 depicting the data for the white blood cell count (WBC), lactic acid, monocyte %, and C-Reactive Protein (CRP) markers, respectively. For the data of the plot 1830, the AUC for the sepsis group is 72%, with a sensitivity of 51% and a specificity of 88%. As can be seen in the lot 1840, the sepsis group and the shock group unexpectedly exhibited increased lactic acid levels compared to the infection and control groups. For the data of the plot 1840, the AUC for the sepsis group is 73%, with a sensitivity of 65% and a specificity of 74%. For the data of the plot 1850, the AUC for the sepsis group is 58%, with a sensitivity of 10% and a specificity of 87%. For the data of the plot 1860, the AUC for the sepsis group is 84%, with a sensitivity of 62% and a specificity of 85%.

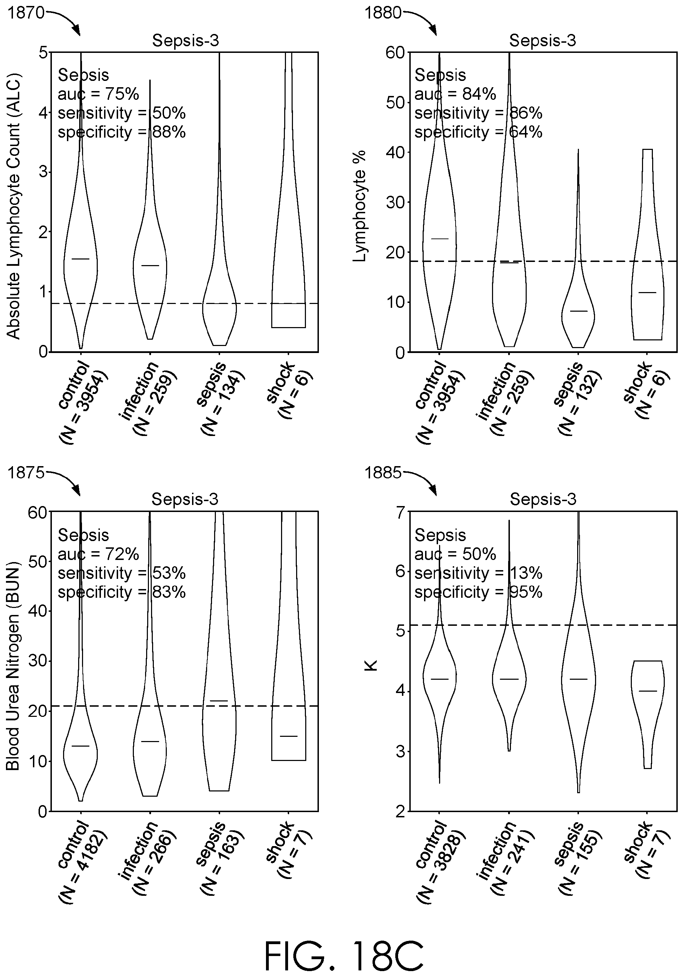

FIG. 18C depicts the plots 1870, 1875, 1880, and 1885 depicting the data for the absolute lymphocyte count (ALC), Blood Urea Nitrogen (BUN), lymphocyte % and potassium markers, respectively. For the data of the plot 1870, the AUC for the sepsis group is 75%, with a sensitivity of 50% and a specificity of 88%. For the data of the plot 1875, the AUC for the sepsis group is 72%, with a sensitivity of 53% and a specificity of 83%. As can be seen in the plot 1880, the lymphocyte % for the sepsis and shock groups was unexpectedly lower than that of the infection and control groups. For the data of the plot 1880, the AUC for the sepsis group is 84%, with a sensitivity of 86% and a specificity of 64%. For the data of the plot 1885, the AUC for the sepsis group is 50%, with a sensitivity of 13% and a specificity of 95%.

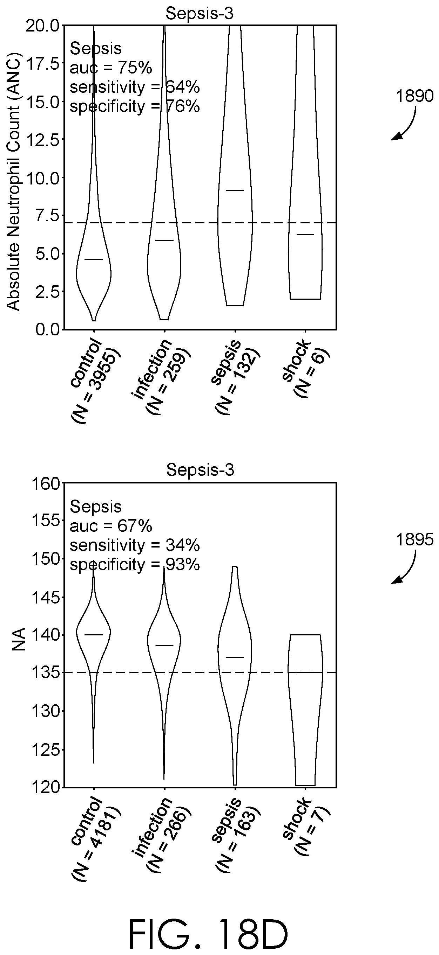

FIG. 18D includes the plots 1890 and 1895 depicting the data for the Absolute Neutrophil Count (ANC) and sodium (Na) markers, respectively. For the data of the plot 1890 the AUC for the sepsis group is 75%, with a sensitivity of 64% and a specificity of 76%. For the data of the plot 1895 the AUC for the sepsis group is 67%, with a sensitivity of 34% and a specificity of 93%.

The data in this Example and in particular as shown in FIGS. 18A-18D, MDW values, lymphocyte %, Neutrophil % and lactic acid measurements, each alone (and/or in various combinations), may aid in assessing a sepsis diagnosis and/or assessing sepsis severity.

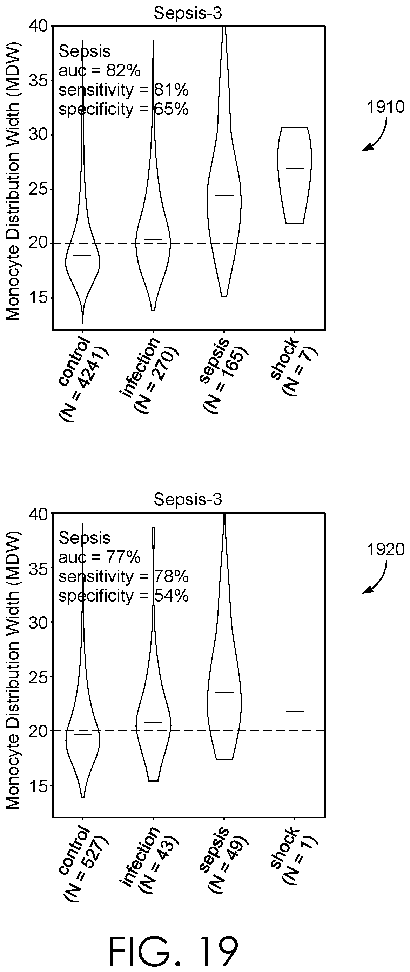

FIG. 19 depict violin plots of MDW values and their relationship with a sepsis-3 outcome for various individual groups (control, infection group, sepsis group, and shock group - all groups exclusive of one another). FIG. 19 includes the plots 1910 and 1920 depicting the data for the MDW for the total cohort and immunosuppressed cohort, respectively. The plot 1910 is identical to the plot 1630 of FIG. 16A discussed above. As can be seen in the plot 1920, the MDW values for the sepsis and shock groups exhibit an increased value compared to the infection and control groups. For the data of the plot 1920, the AUC for the sepsis group is 77%, with a sensitivity of 78% and a specificity of 54%.

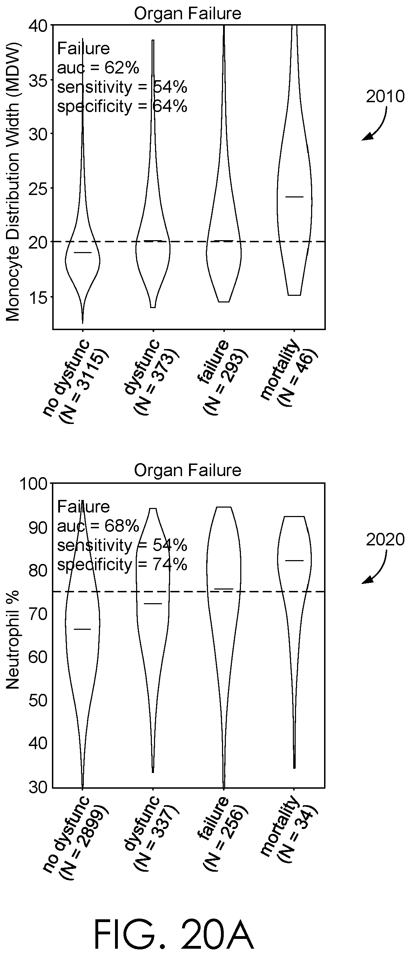

FIGS. 20A-20D depict a series of markers (CBC and/or metabolic) and their relationship with an organ failure outcome for various individual groups (no organ dysfunction, organ dysfunction, organ failure, and in-hospital mortality - all groups exclusive of one another). FIG. 20A includes the plots 2010 and 2020 depicting the data for the MDW and neutrophil % markers, respectively. For the data of the plot 2010, the AUC for the organ failure group is 62%, with a sensitivity of 54% and a specificity of 64%. For the data of the plot 2020, the AUC for the organ failure group is 68%, with a sensitivity of 54% and a specificity of 74%.

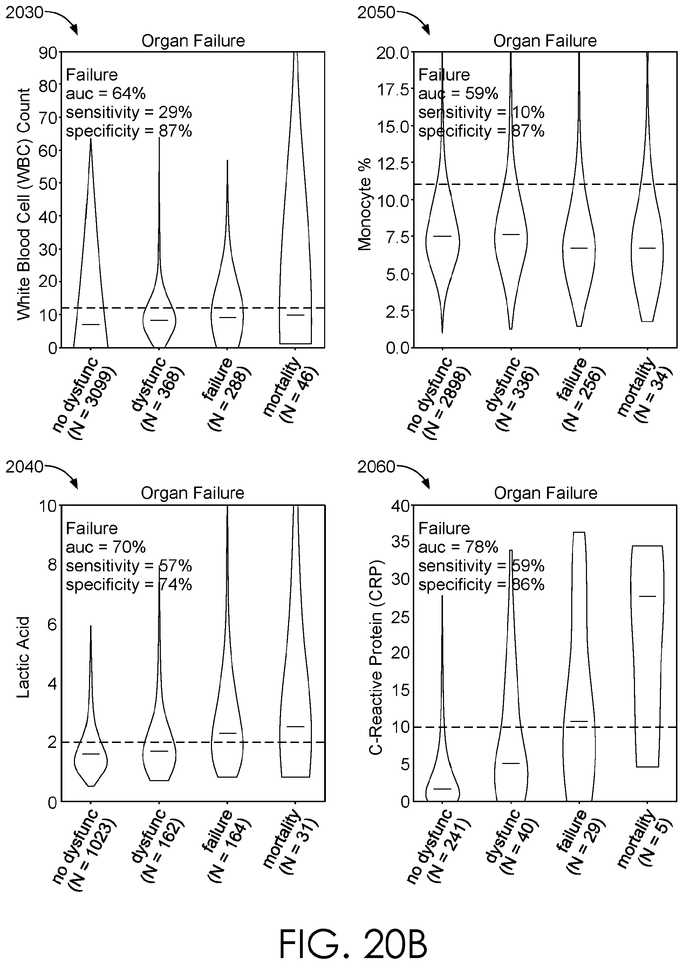

FIG. 20B depicts the plots 2030, 2040, 2050, and 2060 depicting the data for the white blood cell count (WBC), lactic acid, monocyte %, and C-Reactive Protein (CRP) markers, respectively. For the data of the plot 2030, the AUC for the organ failure group is 64%, with a sensitivity of 29% and a specificity of 87%. For the data of the plot 2040, the AUC for the organ failure group is 70%, with a sensitivity of 57% and a specificity of 74%. For the data of the plot 2050, the AUC for the organ failure group is 59%, with a sensitivity of 10% and a specificity of 87%. For the data of the plot 2060, the AUC for the organ failure group is 78%, with a sensitivity of 59% and a specificity of 86%.

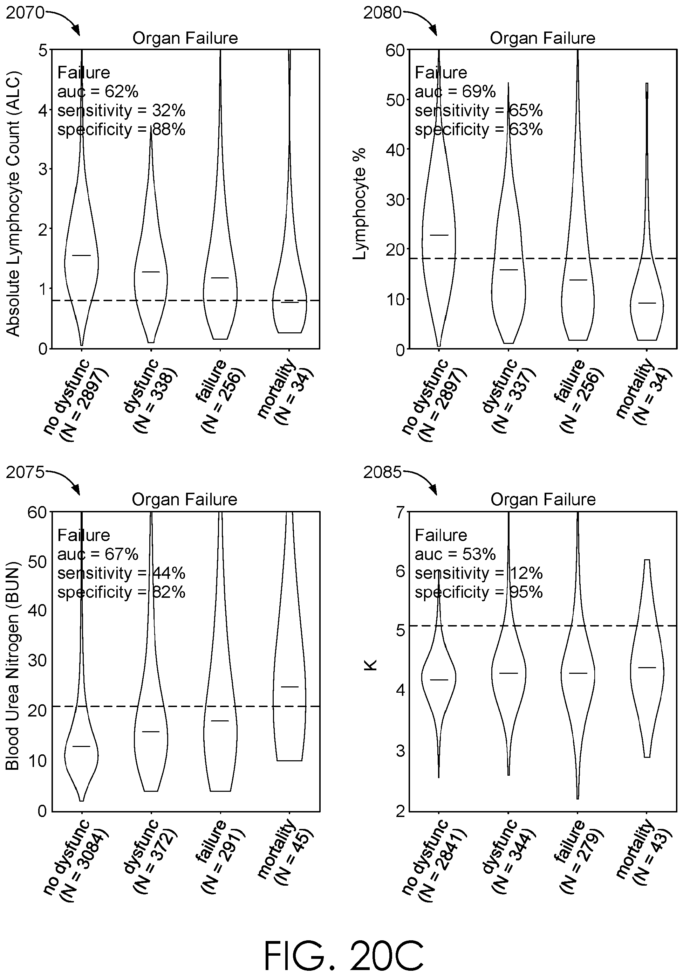

FIG. 20C depicts the plots 2070, 2075, 2080, and 2085 depicting the data for the absolute lymphocyte count (ALC), Blood Urea Nitrogen (BUN), lymphocyte % and potassium markers, respectively. For the data of the plot 2070, the AUC for the organ failure group is 62%, with a sensitivity of 32% and a specificity of 88%. For the data of the plot 2075, the AUC for the organ failure group is 67%, with a sensitivity of 44% and a specificity of 82%. For the data of the plot 2080, the AUC for the organ failure group is 69%, with a sensitivity of 65% and a specificity of 63%. For the data of the plot 2085, the AUC for the organ failure group is 53%, with a sensitivity of 12% and a specificity of 95%.

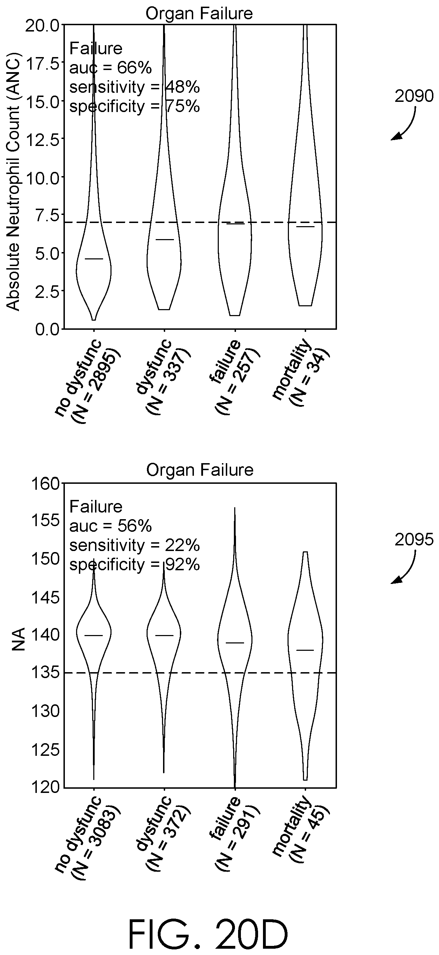

FIG. 20D includes the plots 2090 and 2095 depicting the data for the Absolute Neutrophil Count (ANC) and sodium (Na) markers, respectively. For the data of the plot 2090 the AUC for the organ failure group is 66%, with a sensitivity of 48% and a specificity of 75%. For the data of the plot 2095 the AUC for the organ failure group is 56%, with a sensitivity of 22% and a specificity of 93%.

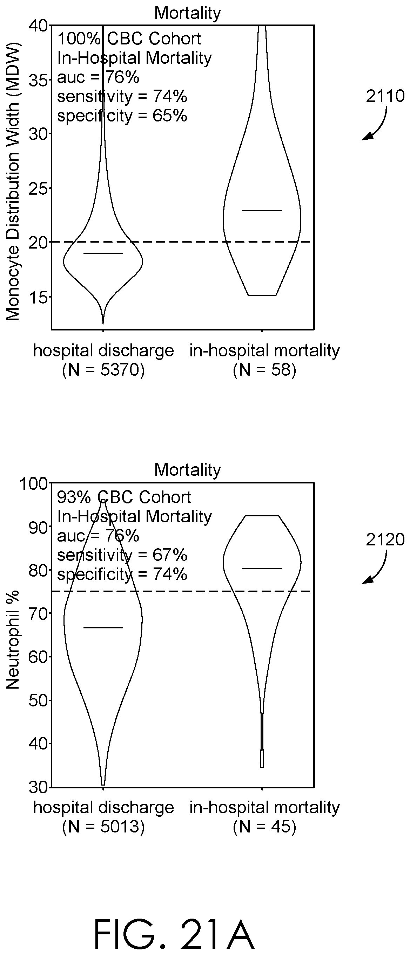

FIGS. 21A - 21D depict a series of markers (CBC and/or metabolic) and their relationship with mortality and discharge outcomes. FIG. 21A includes the plots 2110 and 2120 depicting the data for the MDW and neutrophil % markers, respectively. The plot 2110 is identical to the plot 1610 of FIG. 16A discussed above. As can be seen in the plot 2020, the neutrophil % for the in-hospital mortality group exhibits an increased value compared to the discharge group. For the data of the plot 2020, the AUC for the sepsis group is 76%, with a sensitivity of 67% and a specificity of 74%.

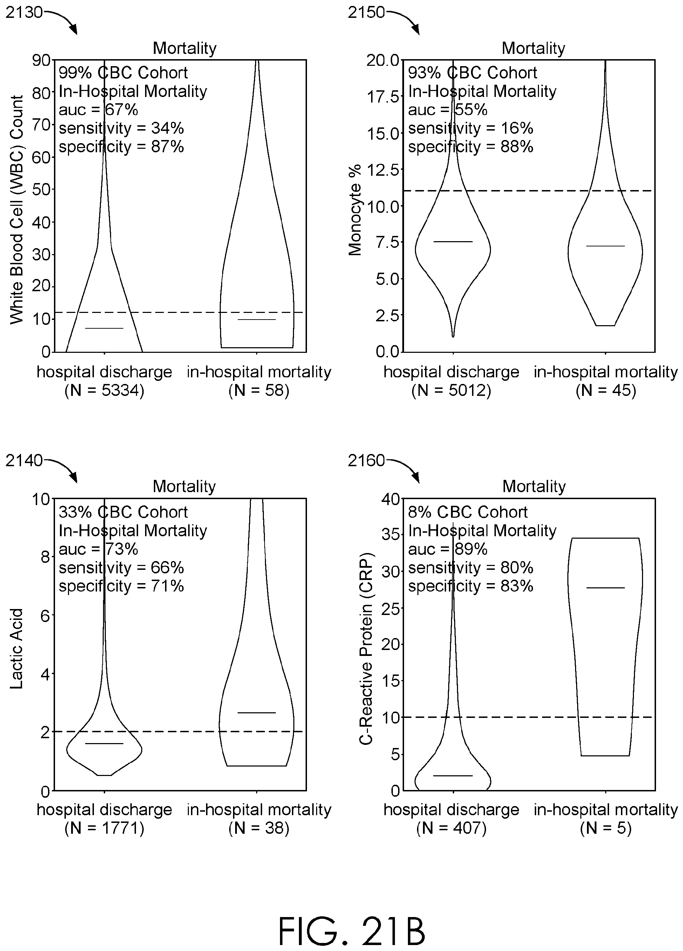

FIG. 21B depicts the plots 2130, 2140, 2150, and 2160 depicting the data for the white blood cell count (WBC), lactic acid, monocyte %, and C-Reactive Protein (CRP) markers, respectively. For the data of the plot 2130, the AUC for the in-hospital mortality group is 67%, with a sensitivity of 34% and a specificity of 87%. As can be seen in the lot 2140, the in-hospital mortality group unexpectedly exhibited increased lactic acid levels compared to the discharge group. For the data of the plot 2140, the AUC for the in-hospital mortality group is 73%, with a sensitivity of 66% and a specificity of 71%. For the data of the plot 2050, the AUC for the in-hospital mortality group is 55%, with a sensitivity of 16% and a specificity of 88%. As can be seen in the lot 2160, the in-hospital mortality group unexpectedly exhibited increased CRP levels compared to the discharge group. For the data of the plot 2060, the AUC for the in-hospital mortality group is 89%, with a sensitivity of 80% and a specificity of 83%.

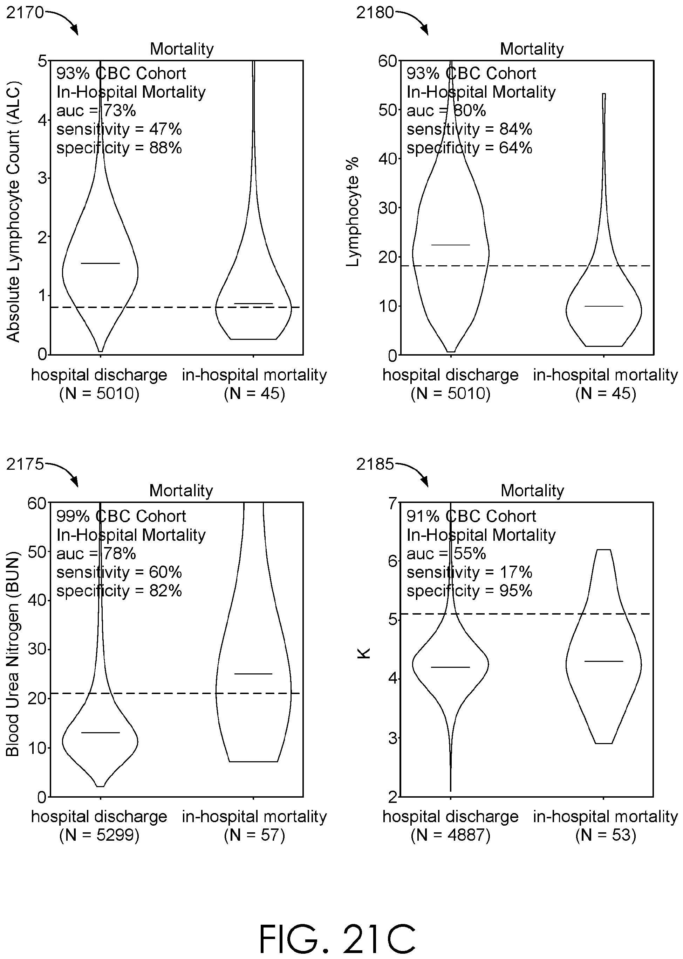

FIG. 21C depicts the plots 2170, 2175, 2180, and 2185 depicting the data for the absolute lymphocyte count (ALC), Blood Urea Nitrogen (BUN), lymphocyte % and potassium markers, respectively. As can be seen in the lot 2170, the in-hospital mortality group unexpectedly exhibited decreased ALC levels compared to the discharge group. For the data of the plot 2170, the AUC for the in-hospital mortality group is 73%, with a sensitivity of 47% and a specificity of 88%. For the data of the plot 2175, the AUC for the in-hospital mortality group is 78%, with a sensitivity of 60% and a specificity of 82%. As can be seen in the plot 2180, the lymphocyte % for the in-hospital mortality group was unexpectedly lower than that of the discharge group. As can be seen in the lot 2180, the in-hospital mortality group unexpectedly exhibited decreased lymphocyte % compared to the discharge group. For the data of the plot 2180, the AUC for the in-hospital mortality group is 80%, with a sensitivity of 84% and a specificity of 64%. For the data of the plot 2185, the AUC for the in-hospital mortality group is 55%, with a sensitivity of 17% and a specificity of 95%.

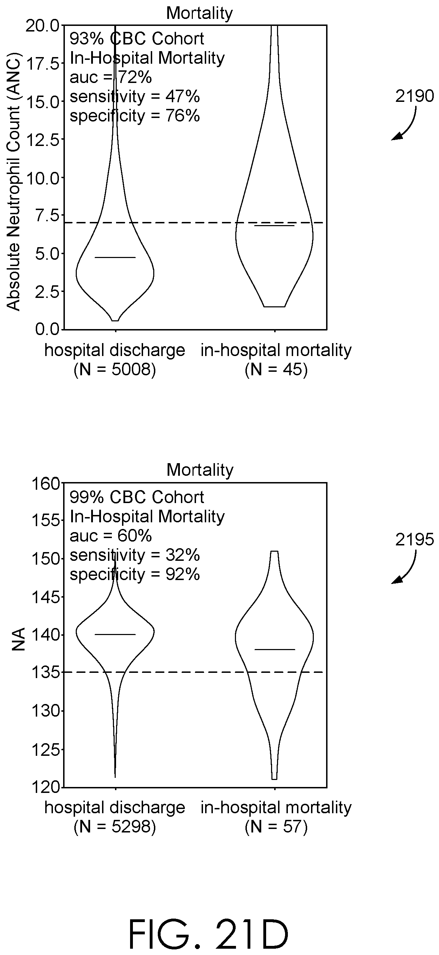

FIG. 21D includes the plots 2190 and 2195 depicting the data for the Absolute Neutrophil Count (ANC) and sodium (Na) markers, respectively. For the data of the plot 2190 the AUC for the in-hospital mortality group is 72%, with a sensitivity of 47% and a specificity of 76%. For the data of the plot 2195 the AUC for the in-hospital mortality group is 60%, with a sensitivity of 32% and a specificity of 92%.

The data in this Example and in particular as shown in FIGS. 21A-21D, MDW values, WBC measurements, ALC, lymphocyte %, ANC, Neutrophil %, lactic acid, CRP, and BUN measurements, each alone (and/or in various combinations), may aid in assessing acuity, such as in-hospital mortality.

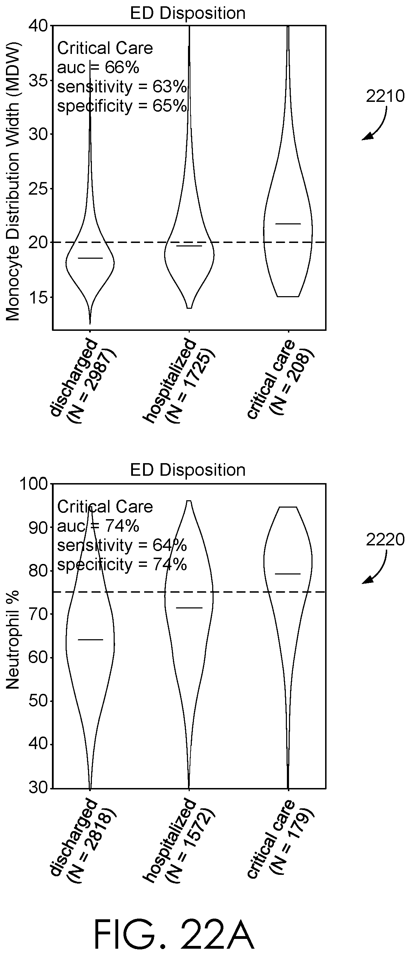

FIGS. 22A - 22D depict a series of markers (CBC and/or metabolic) and their relationship with critical care, hospitalized, or discharged outcomes. FIG. 22A includes the plots 2210 and 2220 depicting the data for the MDW and neutrophil % markers, respectively. The plot 2210 is identical to the plot 1620 of FIG. 16A discussed above. As can be seen in the plot 2220, the neutrophil % for the critical care and hospitalized groups exhibit an increased value compared to the discharge group. For the data of the plot 2220, the AUC for the critical care group is 74%, with a sensitivity of 64% and a specificity of 74%.

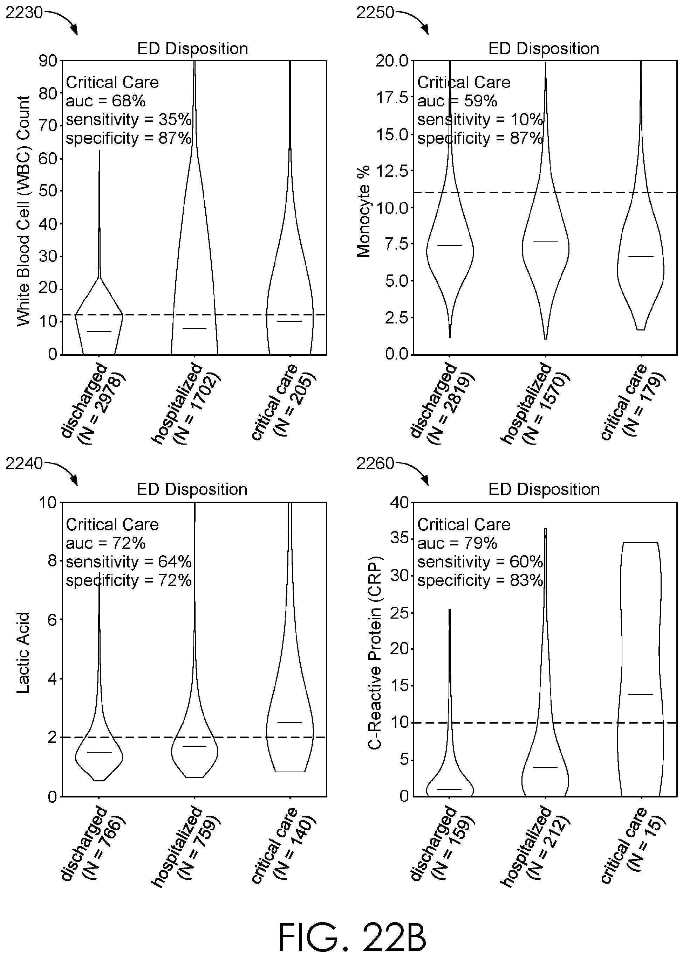

FIG. 22B depicts the plots 2230, 2240, 2250, and 2260 depicting the data for the white blood cell count (WBC), lactic acid, monocyte %, and C-Reactive Protein (CRP) markers, respectively. For the data of the plot 2230, the AUC for the critical care group is 68%, with a sensitivity of 35% and a specificity of 87%. As can be seen in the lot 2240, the critical care group unexpectedly exhibited increased lactic acid levels compared to the discharge group. For the data of the plot 2240, the AUC for the critical care group is 72%, with a sensitivity of 64% and a specificity of 72%. For the data of the plot 2250, the AUC for the critical care group is 59%, with a sensitivity of 10% and a specificity of 87%. As can be seen in the lot 2260, the critical care group unexpectedly exhibited increased CRP levels compared to the discharge group. For the data of the plot 2260, the AUC for the critical care group is 79%, with a sensitivity of 60% and a specificity of 83%.

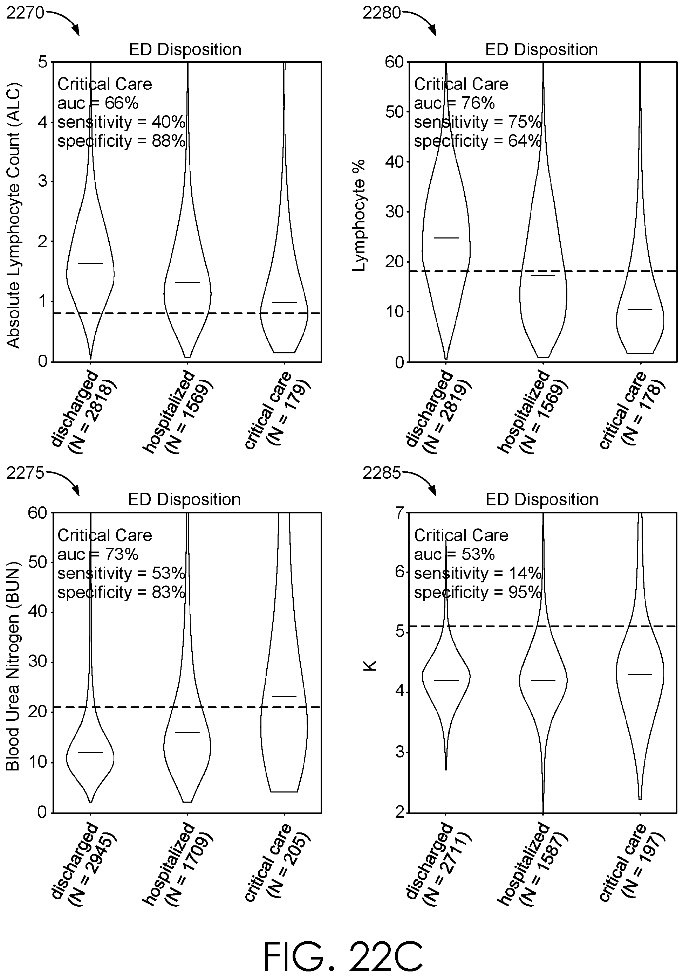

FIG. 22C depicts the plots 2270, 2275, 2280, and 2285 depicting the data for the absolute lymphocyte count (ALC), Blood Urea Nitrogen (BUN), lymphocyte % and potassium markers, respectively. As can be seen in the lot 2270, the critical care group unexpectedly exhibited decreased ALC levels compared to the discharge group. For the data of the plot 2270, the AUC for the critical care group is 66%, with a sensitivity of 40% and a specificity of 88%. For the data of the plot 2275, the AUC for the critical care group is 78%, with a sensitivity of 53% and a specificity of 83%. As can be seen in the plot 2280, the lymphocyte % for the critical care group was unexpectedly lower than that of the discharge group. For the data of the plot 2280, the AUC for the critical care mortality group is 76%, with a sensitivity of 75% and a specificity of 64%. For the data of the plot 2285, the AUC for the critical care group is 53%, with a sensitivity of 14% and a specificity of 95%.

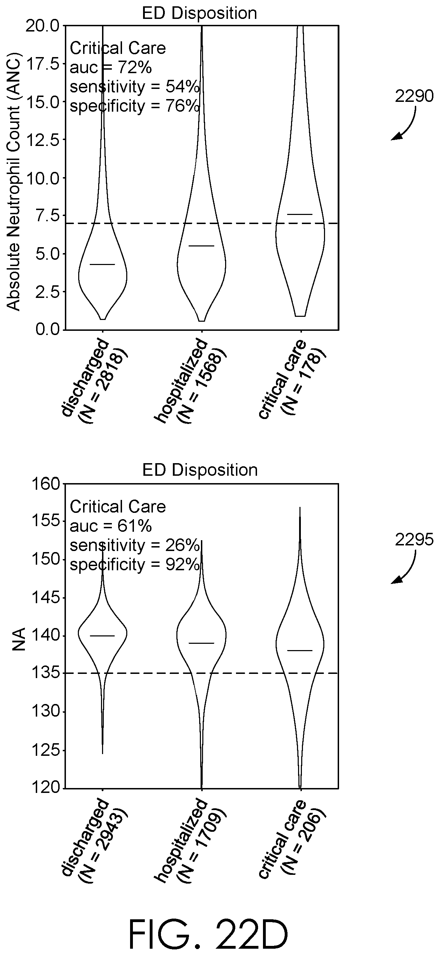

FIG. 22D includes the plots 2290 and 2295 depicting the data for the Absolute Neutrophil Count (ANC) and sodium (Na) markers, respectively. For the data of the plot 2290 the AUC for the critical care group is 72%, with a sensitivity of 54% and a specificity of 76%. For the data of the plot 2295 the AUC for the critical care group is 61%, with a sensitivity of 26% and a specificity of 92%.

The data in this Example and in particular as shown in FIGS. 22A-22D, MDW values, WBC measurements, ALC, lymphocyte %, ANC, Neutrophil %, lactic acid, CRP, and BUN measurements, each alone (and/or in various combinations), may aid in assessing acuity, such as individuals needing critical care.

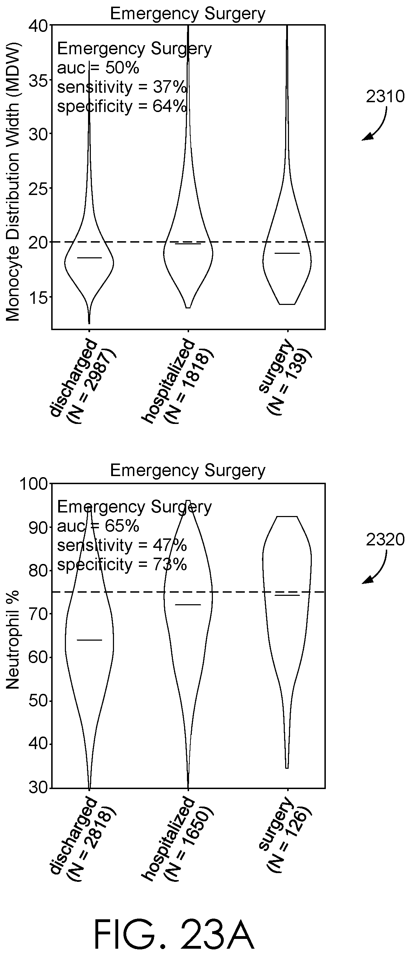

FIGS. 23A - 23D depict a series of markers (CBC and/or metabolic) and their relationship with emergency surgery, hospitalized, or discharged outcomes. FIG. 23A includes the plots 2310 and 2320 depicting the data for the MDW and neutrophil % markers, respectively. For the data of the plot 2310, the AUC for the emergency surgery group is 50%, with a sensitivity of 37% and a specificity of 64%. For the data of the plot 2320, the AUC for the emergency surgery group is 65%, with a sensitivity of 47% and a specificity of 73%.

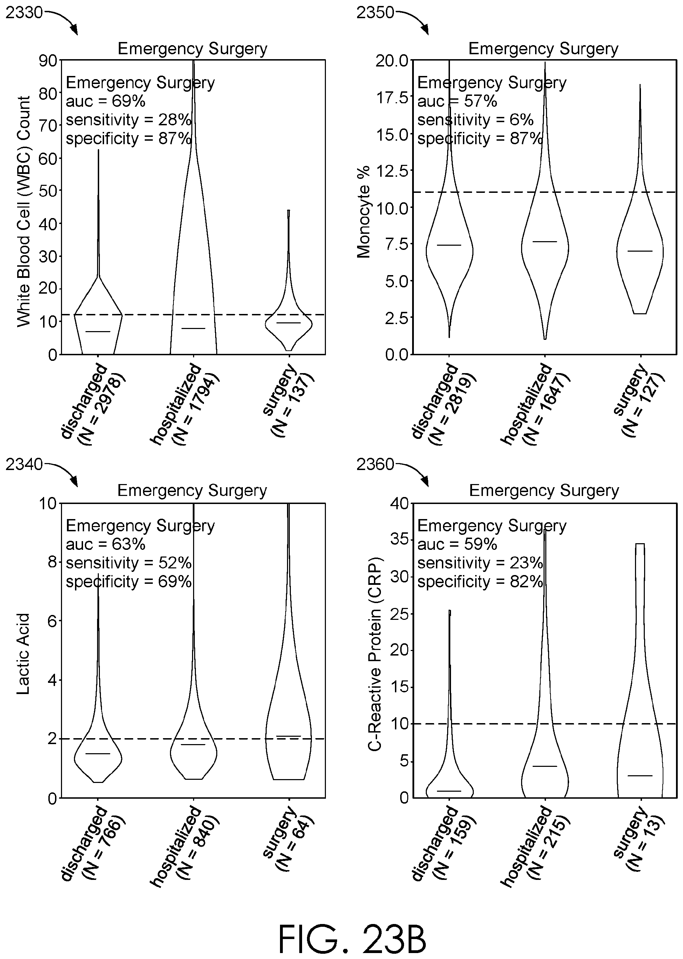

FIG. 23B depicts the plots 2330, 2340, 2350, and 2360 depicting the data for the white blood cell count (WBC), lactic acid, monocyte %, and C-Reactive Protein (CRP) markers, respectively. For the data of the plot 2330, the AUC for the emergency surgery group is 69%, with a sensitivity of 28% and a specificity of 87%. For the data of the plot 2340, the AUC for the emergency surgery group is 63%, with a sensitivity of 52% and a specificity of 69%. For the data of the plot 2350, the AUC for the emergency surgery group is 57%, with a sensitivity of 6% and a specificity of 87%. For the data of the plot 2360, the AUC for the emergency surgery group is 59%, with a sensitivity of 23% and a specificity of 82%.

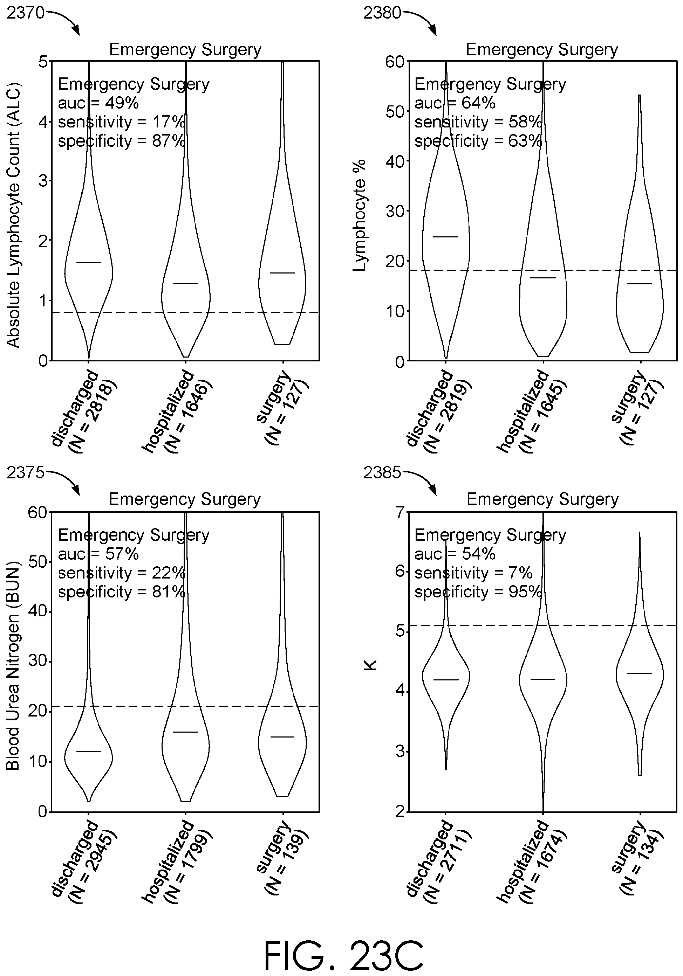

FIG. 23C depicts the plots 2370, 2375, 2380, and 2385 depicting the data for the absolute lymphocyte count (ALC), Blood Urea Nitrogen (BUN), lymphocyte % and potassium markers, respectively. For the data of the plot 2370, the AUC for the emergency surgery group is 49%, with a sensitivity of 17% and a specificity of 87%. For the data of the plot 2375, the AUC for the emergency surgery group is 57%, with a sensitivity of 22% and a specificity of 81%. For the data of the plot 2380, the AUC for the emergency surgery mortality group is 64%, with a sensitivity of 58% and a specificity of 63%. For the data of the plot 2385, the AUC for the emergency surgery group is 54%, with a sensitivity of 7% and a specificity of 95%.

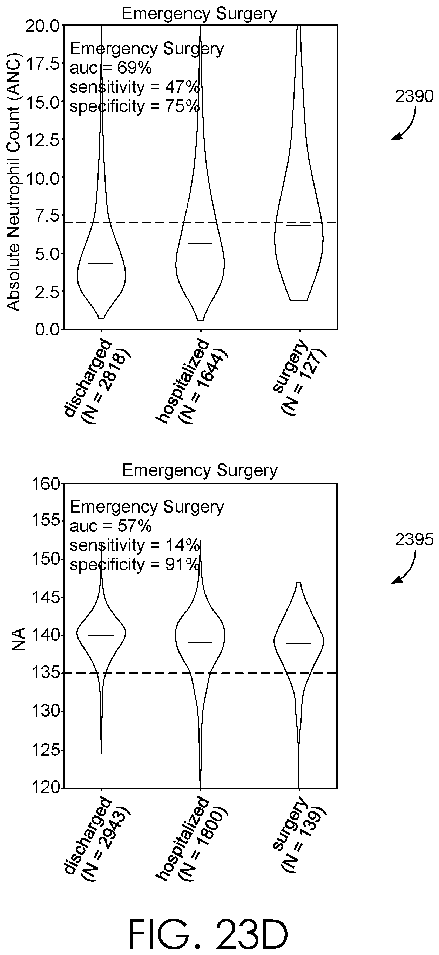

FIG. 23D includes the plots 2390 and 2395 depicting the data for the Absolute Neutrophil Count (ANC) and sodium (Na) markers, respectively. For the data of the plot 2390 the AUC for the emergency surgery group is 69%, with a sensitivity of 47% and a specificity of 75%. For the data of the plot 2395 the AUC for the emergency surgery group is 57%, with a sensitivity of 14% and a specificity of 91%.

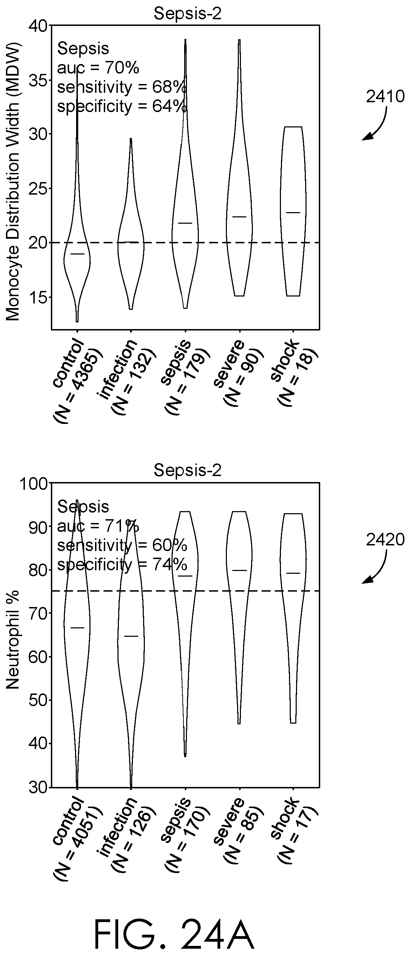

FIGS. 24A-24D depict a series of markers (CBC and/or metabolic) and their relationship with a sepsis-2 outcome for various individual groups (control, infection group, sepsis group, severe sepsis group, and shock group - all groups exclusive of one another). FIG. 24A includes the plots 2410 and 2420 depicting the data for the MDW and neutrophil % markers, respectively. As can be seen in the plot 2410, the MDW for the sepsis, severe, and shock groups exhibit an increased value compared to the infection and control groups. For the data in the plot 2410, the AUC for the sepsis group is 70%, with a sensitivity of 68% and a specificity of 64%. As can be seen in the plot 2420, the neutrophil % for the sepsis, severe, and shock groups exhibit an increased value compared to the infection and control groups. For the data of the plot 2420, the AUC for the sepsis group is 71%, with a sensitivity of 60% and a specificity of 74%.

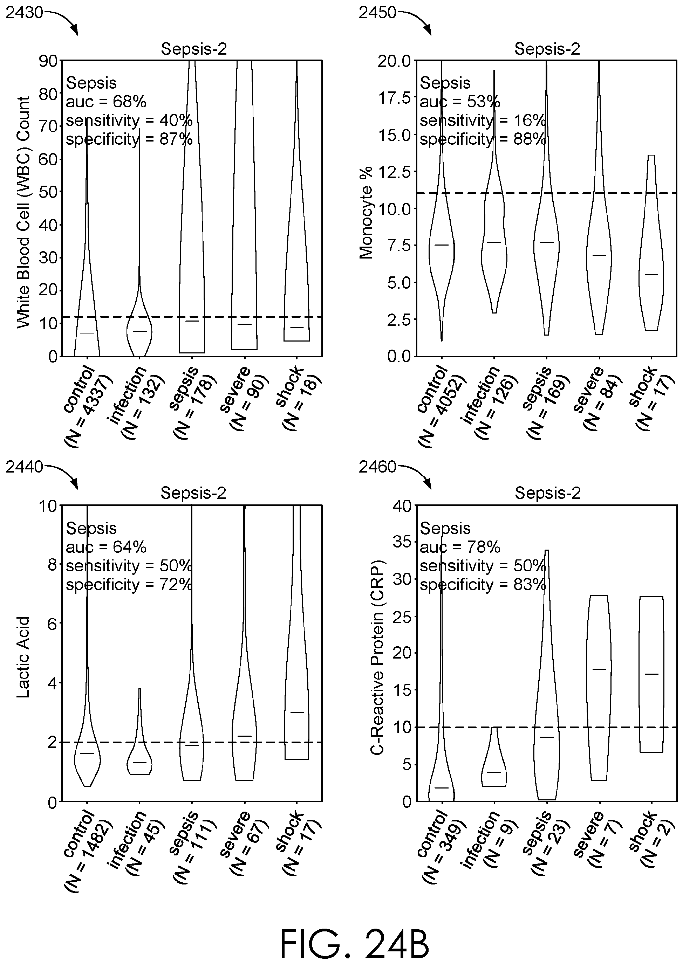

FIG. 24B depicts the plots 2430, 2440, 2450, and 2460 depicting the data for the white blood cell count (WBC), lactic acid, monocyte %, and C-Reactive Protein (CRP) markers, respectively. As can be seen in the plot 2430, the WBC for the sepsis group exhibits an increased value compared to the infection and control groups. For the data of the plot 2430, the AUC for the sepsis group is 68%, with a sensitivity of 40% and a specificity of 87%. For the data of the plot 2440, the AUC for the sepsis group is 64%, with a sensitivity of 50% and a specificity of 72%. For the data of the plot 2450, the AUC for the sepsis group is 53%, with a sensitivity of 16% and a specificity of 88%. As can be seen in the plot 2460, the CRP for the sepsis, severe, and shock groups exhibit an increased value compared to the infection and control groups. For the data of the plot 2460, the AUC for the sepsis group is 78%, with a sensitivity of 50% and a specificity of 83%.

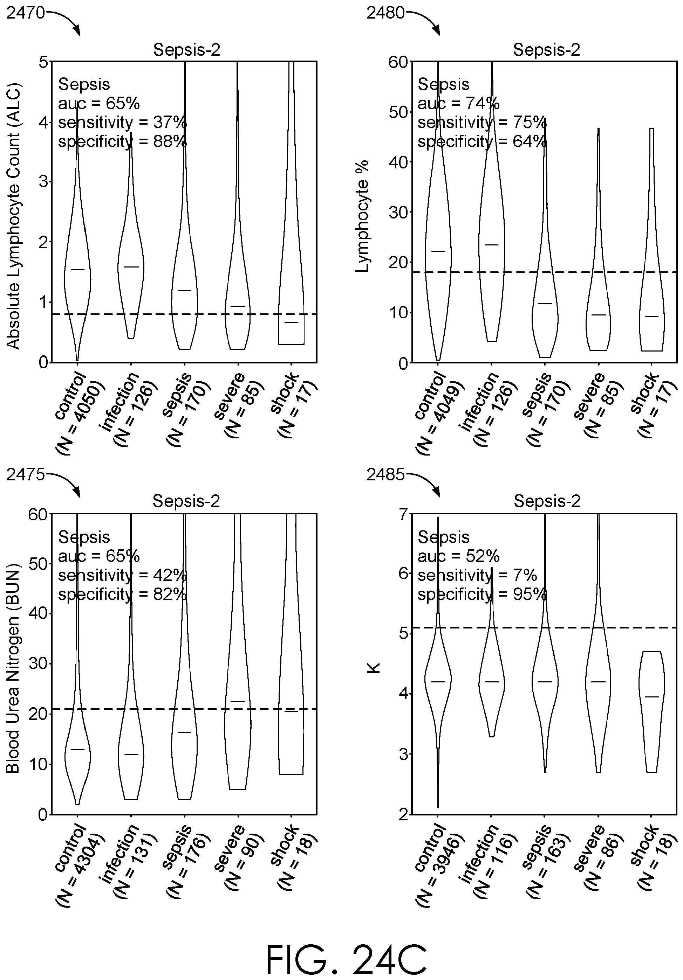

FIG. 24C depicts the plots 2470, 2475, 2480, and 2485 depicting the data for the absolute lymphocyte count (ALC), Blood Urea Nitrogen (BUN), lymphocyte % and potassium markers, respectively. For the data of the plot 2470, the AUC for the sepsis group is 65%, with a sensitivity of 37% and a specificity of 88%. For the data of the plot 2475, the AUC for the sepsis group is 65%, with a sensitivity of 42% and a specificity of 82%. As can be seen in the plot 2480, the lymphocyte % for the sepsis, severe, and shock groups was unexpectedly lower than that of the infection and control groups. For the data of the plot 2480, the AUC for the sepsis group is 74%, with a sensitivity of 75% and a specificity of 64%. For the data of the plot 2485, the AUC for the sepsis group is 52%, with a sensitivity of 7% and a specificity of 95%.

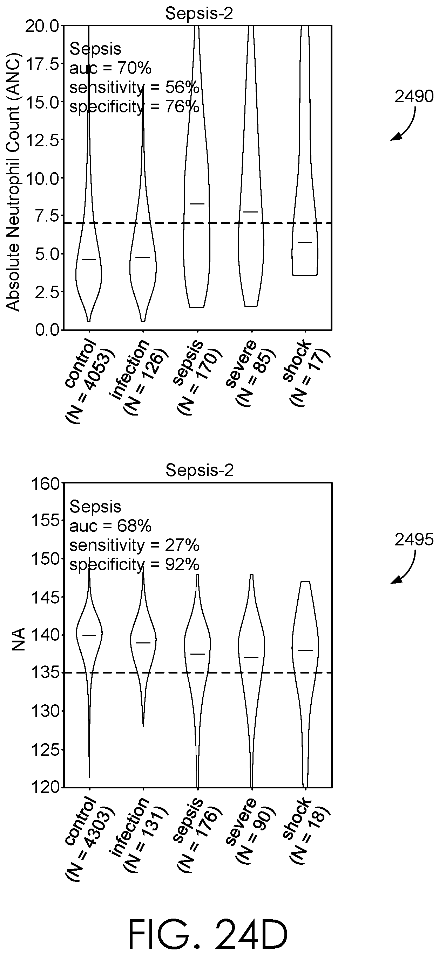

FIG. 24D includes the plots 2490 and 2495 depicting the data for the Absolute Neutrophil Count (ANC) and sodium (Na) markers, respectively. As can be seen in the plot 2490, the ANC for the sepsis, severe, and shock groups was unexpectedly higher than that of the infection and control groups. For the data of the plot 2490 the AUC for the sepsis group is 70%, with a sensitivity of 56% and a specificity of 76%. For the data of the plot 2495 the AUC for the sepsis group is 68%, with a sensitivity of 27% and a specificity of 92%.

The data in this Example and in particular as shown in FIGS. 24A-24D, MDW values, WBC measurements, lymphocyte %, ANC measurements, neutrophil %, and CRP measurements, each alone (and/or in various combinations), may aid in assessing a sepsis diagnosis and/or assessing sepsis severity.

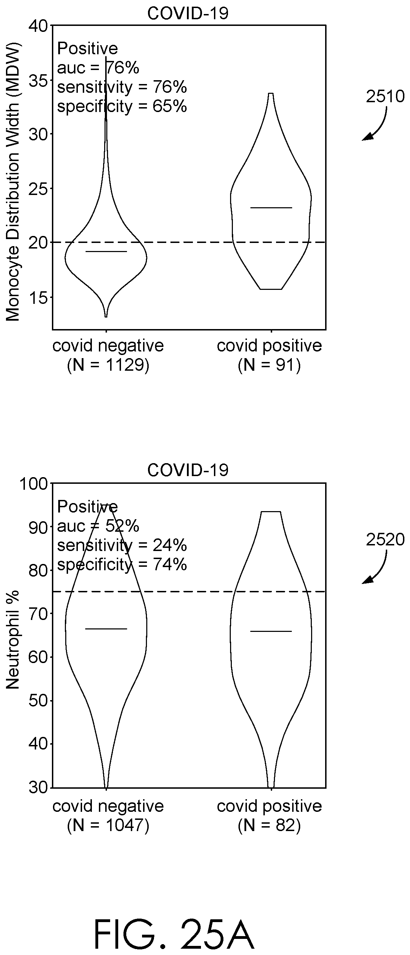

FIGS. 25A - 25D depict a series of markers (CBC and/or metabolic) and their relationship with COVID-19 positive and COVID-19 negative outcomes. FIG. 25A includes the plots 2510 and 2520 depicting the data for the MDW and neutrophil % markers, respectively. The plot 2510 is identical to the plot 1650 of FIG. 16B. For the data of the plot 2520, the AUC for the COVID-19 positive group is 52%, with a sensitivity of 24% and a specificity of 74%.

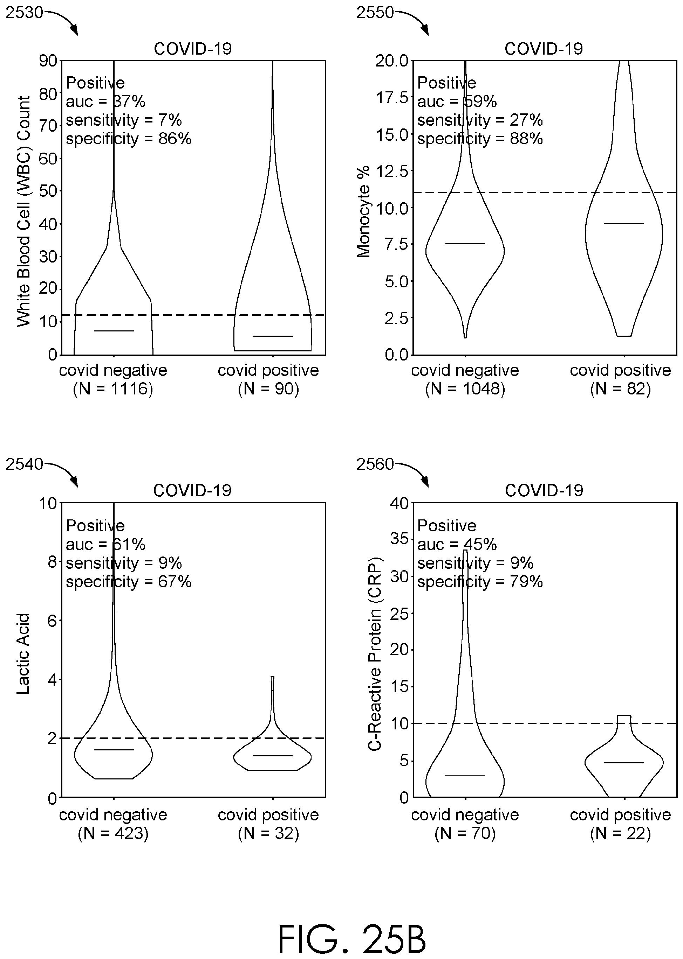

FIG. 25B depicts the plots 2530, 2540, 2550, and 2560 depicting the data for the white blood cell count (WBC), lactic acid, monocyte %, and C-Reactive Protein (CRP) markers, respectively. For the data of the plot 2530, the AUC for the COVID-19 positive group is 37%, with a sensitivity of 7% and a specificity of 86%. For the data of the plot 2540, the AUC for the COVID-19 positive group is 61%, with a sensitivity of 9% and a specificity of 67%. For the data of the plot 2550, the AUC for the COVID-19 positive group is 59%, with a sensitivity of 27% and a specificity of 88%. For the data of the plot 2560, the AUC for the COVID-19 positive group is 45%, with a sensitivity of 9% and a specificity of 79%.

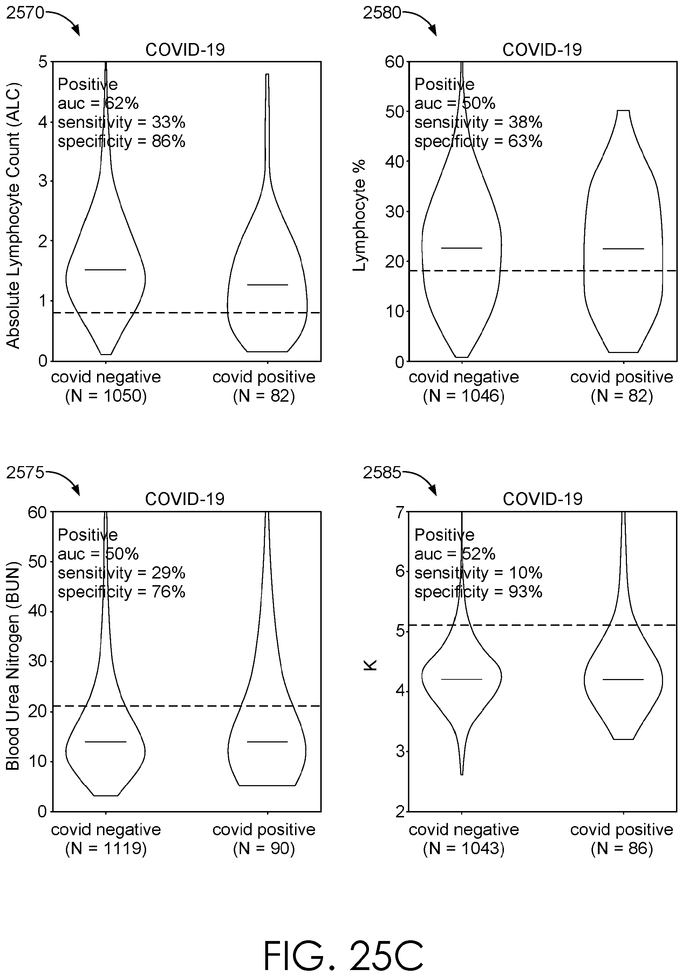

FIG. 25C depicts the plots 2570, 2575, 2580, and 2585 depicting the data for the absolute lymphocyte count (ALC), Blood Urea Nitrogen (BUN), lymphocyte % and potassium markers, respectively. For the data of the plot 2570, the AUC for the COVID-19 positive group is 62%, with a sensitivity of 33% and a specificity of 86%. For the data of the plot 2575, the AUC for the COVID-19 positive group is 50%, with a sensitivity of 29% and a specificity of 76%. For the data of the plot 2580, the AUC for the COVID-19 positive group is 50%, with a sensitivity of 38% and a specificity of 63%. For the data of the plot 2585, the AUC for the COVID-19 positive group is 52%, with a sensitivity of 10% and a specificity of 93%.

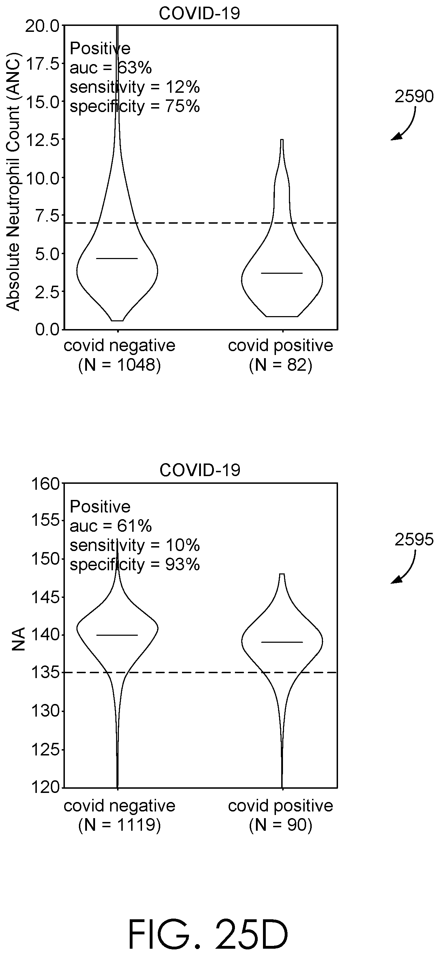

FIG. 25D includes the plots 2590 and 2595 depicting the data for the Absolute Neutrophil Count (ANC) and sodium (Na) markers, respectively. For the data of the plot 2590 the AUC for the COVID-19 positive group is 63%, with a sensitivity of 12% and a specificity of 75%. For the data of the plot 2595 the AUC for the COVID-19 positive group is 61%, with a sensitivity of 10% and a specificity of 93%.

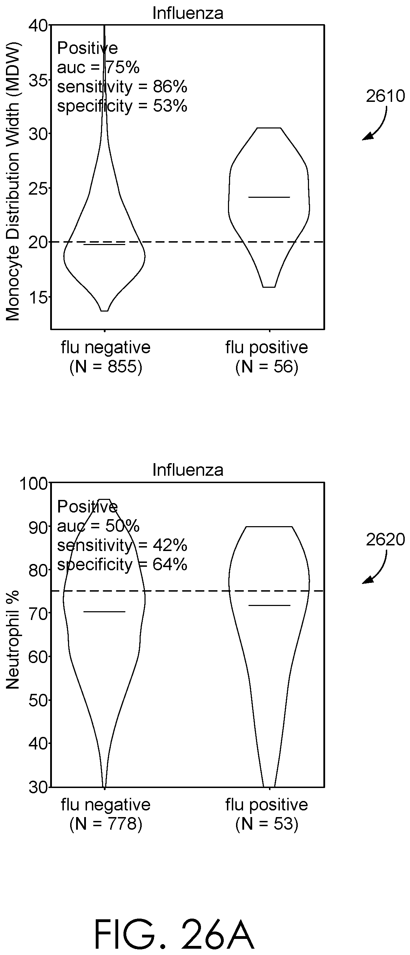

FIGS. 26A - 26D depict a series of markers (CBC and/or metabolic) and their relationship with Influenza positive and negative outcomes. FIG. 26A includes the plots 2610 and 2620 depicting the data for the MDW and neutrophil % markers, respectively. As can be seen in the plot 2610, the MDW of the Influenza positive group is unexpectedly higher than the negative group. For the data of the plot 2610, the AUC for the Influenza positive group is 75%, with a sensitivity of 86% and a specificity of 53%. For the data of the plot 2620, the AUC for Influenza positive group is 50%, with a sensitivity of 42% and a specificity of 64%.

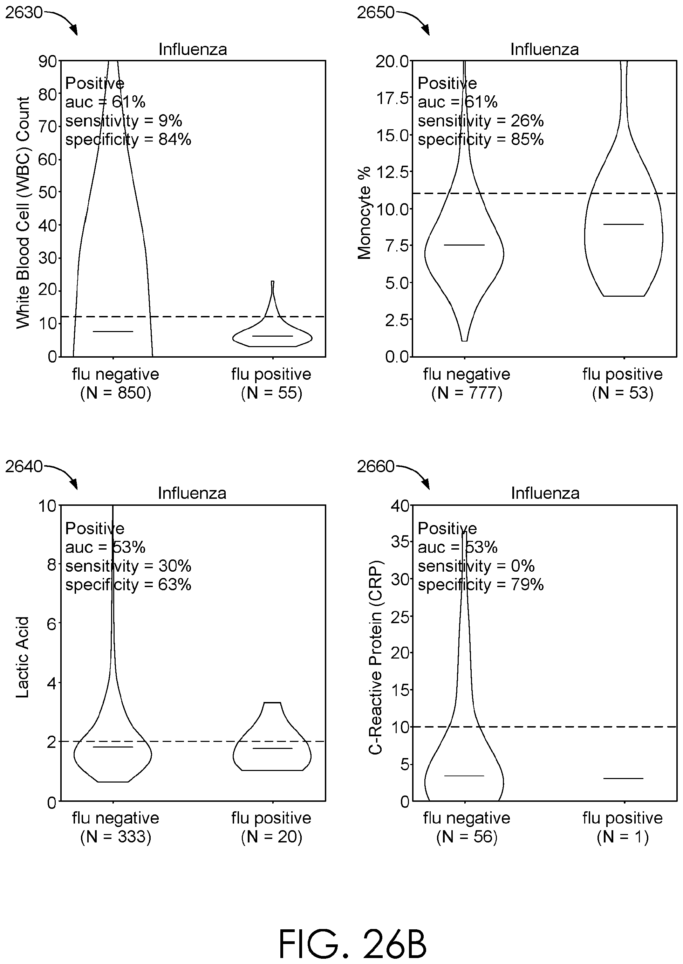

FIG. 26B depicts the plots 2630, 2640, 2650, and 2660 depicting the data for the white blood cell count (WBC), lactic acid, monocyte %, and C-Reactive Protein (CRP) markers, respectively. For the data of the plot 2630, the AUC for the Influenza positive group is 61%, with a sensitivity of 9% and a specificity of 84%. For the data of the plot 2640, the AUC for the Influenza positive group is 61%, with a sensitivity of 26% and a specificity of 85%. For the data of the plot 2650, the AUC for the Influenza positive group is 61%, with a sensitivity of 26% and a specificity of 85%. For the data of the plot 2660, the AUC for the Influenza positive group is 53%, with a sensitivity of 0% and a specificity of 79%.

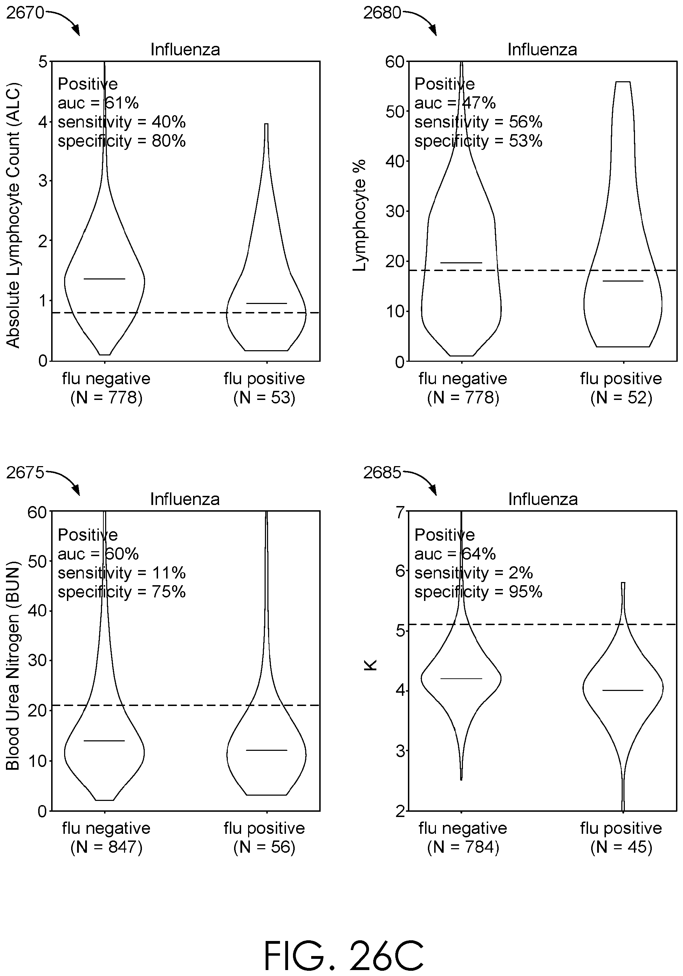

FIG. 26C depicts the plots 2670, 2675, 2680, and 2685 depicting the data for the absolute lymphocyte count (ALC), Blood Urea Nitrogen (BUN), lymphocyte % and potassium markers, respectively. For the data of the plot 2670, the AUC for the Influenza positive group is 61%, with a sensitivity of 40% and a specificity of 80%. For the data of the plot 2675, the AUC for the Influenza positive group is 60%, with a sensitivity of 11% and a specificity of 75%. For the data of the plot 2680, the AUC for the Influenza positive group is 47%, with a sensitivity of 56% and a specificity of 53%. For the data of the plot 2685, the AUC for the Influenza positive group is 64%, with a sensitivity of 2% and a specificity of 95%.

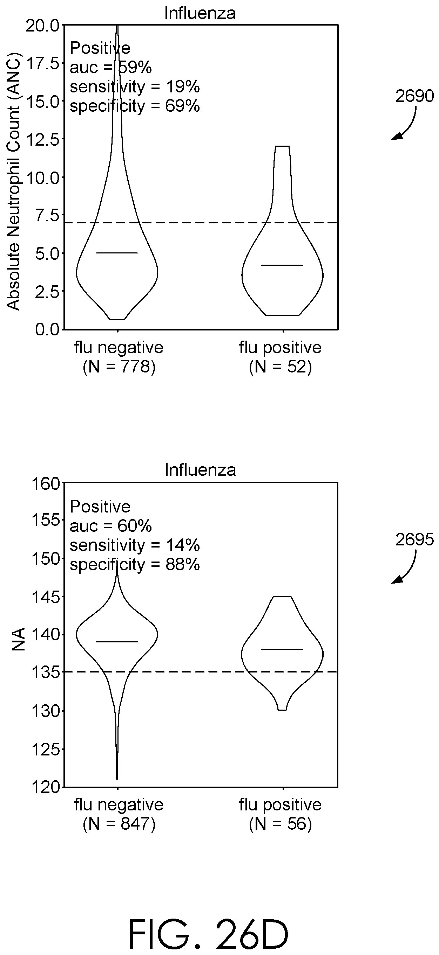

FIG. 26D includes the plots 2690 and 2695 depicting the data for the Absolute Neutrophil Count (ANC) and sodium (Na) markers, respectively. For the data of the plot 2690 the AUC for the Influenza positive group is 59%, with a sensitivity of 19% and a specificity of 69%. For the data of the plot 2695 the AUC for the Influenza positive group is 60%, with a sensitivity of 14% and a specificity of 88%.

Table 4 below illustrates the discrimination across markers for various outcomes.

**Table 4: Discrimination Across Markers**

| Lab | N # of samples | Mortality AUC (%) | Critical Care AUC (%) | Emergency Surgery AUC (%) | Organ Failure AUC (%) | Sepsis-2 AUC (%) | Sepsis-3 AUC (%) | COVID-19 AUC (%) | Influenza AUC (%) |
|---|---|---|---|---|---|---|---|---|---|
| MDW | 5371 | 76 | 66 | 50 | 62 | 69 | 81 | 76 | 86 |
| WBC | 5319 | 67 | 68 | 69 | 62 | 68 | 51 | 7 | 9 |
| Monocyte % | 4999 | 55 | 59 | 58 | 54 | 50 | 10 | 27 | 26 |
| ALC | 4993 | 73 | 66 | 49 | 62 | 63 | 50 | 33 | 40 |
| Lymphocyte % | 4998 | 80 | 76 | 64 | 67 | 73 | 86 | 38 | 57 |

(continued)

| Lab | N # of samples | Mortality AUC (%) | Critical Care AUC (%) | Emergency Surgery AUC (%) | Organ Failure AUC (%) | Sepsis-2 AUC (%) | Sepsis-3 AUC (%) | COVID-19 AUC (%) | Influenza AUC (%) |
|---|---|---|---|---|---|---|---|---|---|
| ANC | 4993 | 72 | 72 | 69 | 64 | 70 | 64 | 12 | 19 |
| Neutrophil % | 4997 | 76 | 74 | 65 | 66 | 70 | 76 | 24 | 42 |
| Lactic acid | 1766 | 73 | 72 | 64 | 63 | 59 | 65 | 9 | 30 |
| CRP | 406 | 89 | 79 | 60 | 74 | 75 | 62 | 9 | 0 |
| BUN | 5284 | 78 | 73 | 58 | 66 | 61 | 53 | 29 | 11 |
| K | 4873 | 56 | 53 | 54 | 53 | 50 | 13 | 10 | 2 |
| NA | 5283 | 60 | 61 | 57 | 55 | 68 | 34 | 10 | 14 |

As can be seen in Table 4 above, MDW is a unique CBC marker in distinguishing infection, acuity, and/or sepsis, as evidenced by the high MDW AUC values, e.g., higher than 60%, for almost all the outcomes listed in Table 2. Further, the combination of high MDW AUV values of other markers listed in Table 2 can unexpectedly provide a further differentiator of specific acuity and/or elevated risks of specific outcomes. For instance, while both an in-hospital mortality and sepsis-3 have higher AUC values for MDW measurements, 76% and 81%, respectively. in-hospital mortality also correlates with certain other markers (e.g., WBC (67% AUC), ALC (73% AUC), and BUN (78% AUC)), while sepsis-3 did not in this study.

In the preceding description, for the purposes of explanation, numerous details have been set forth in order to provide an understanding of various embodiments of the present technology. It will be apparent to one skilled in the art, however, that certain embodiments may be practiced without some of these details, or with additional details, or in varied combinations or sub-combinations of features of the embodiments.

Additionally, a number of well-known processes and elements have not been described in order to avoid unnecessarily obscuring the present invention. Additionally, details of any specific embodiment may not always be present in variations of that embodiment or may be added to other embodiments.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed. The upper and lower limits of these smaller ranges may independently be included or excluded in the range, and each range where either, neither, or both limits are included in the smaller ranges is also encompassed within the invention, subject to any specifically excluded limit in the stated range. Where the stated range includes one or both of the limits, ranges excluding either or both of those included limits are also included.

As used herein and in the appended claims, the singular forms "a", "an", and "the" include plural referents unless the context clearly dictates otherwise. Thus, for example, reference to "a method" includes a plurality of such methods and reference to "the transducer" includes reference to one or more transducers and equivalents thereof known to those skilled in the art, and so forth.

### Claims

**1.** A method for assessing viral infection, wherein the viral infection is SARS-CoV-2 or influenza, the method comprising: characterizing a white blood cell count (WBC) in a blood sample; calculating a monocyte distribution width (MDW) value for the blood sample; if the MDW value for the blood sample is less than or equal to 20, indicating that viral infection is unlikely; and if the MDW value for the blood sample is greater than or equal to 20, evaluating percent lymphocytes and providing an indication of suspected viral infection if the lymphocyte percent is less than 15%.

**2.** The method of claim 1, wherein the method is automatically performed on any blood sample tested for CBC with differential (CBC-DIFF).

**3.** The method of claim 1 or 2, further comprising assessing one or more of axial light loss (ALL) mean for monocytes;

direct current (DC) mean for monocytes; standard deviation for ALL for monocytes; percent basophils; mean corpuscular hemoglobin concentration (MCHC); Low Hemoglobin Density (LHD) value; the standard deviation, in volume, of WBC; the standard deviation, in volume, of Monocytes and Neutrophils; or percent neutrophils.

4. The method of any of claims 1-3, wherein characterizing the WBC in a blood sample comprises impedance and light scatter measurements.

5. The method of any of claims 1-4, wherein characterizing the WBC in a blood sample does not rely on cytochemical stains or affinity-based markers.

6. The method of any of claims 1-5 wherein characterizing the WBC comprises less than a full CBC-DIFF.

7. The method of any of claims 1 and 3-6, wherein the method is automated.

**Patentansprüche**

1. Verfahren zur Beurteilung einer Virusinfektion, wobei die Virusinfektion eine Infektion mit SARS-CoV-2 oder Influenza ist, wobei das Verfahren umfasst: Charakterisierung der Anzahl weißer Blutkörperchen (WBK) in einer Blutprobe; Berechnung eines Monozytenverteilungsbreitenwerts (MDW) für die Blutprobe; wenn der MDW-Wert für die Blutprobe kleiner oder gleich 20 ist, was darauf hinweist, dass eine Virusinfektion unwahrscheinlich ist; und wenn der MDW-Wert für die Blutprobe größer oder gleich 20 ist, Auswerten des prozentualen Anteils der Lymphozyten und Bereitstellen eines Hinweises auf eine vermutete Virusinfektion, wenn der prozentuale Anteil der Lymphozyten weniger als 15 % beträgt.

2. Verfahren nach Anspruch 1, wobei das Verfahren automatisch an jeder Blutprobe durchgeführt wird, die für ein vollständiges Blutbild mit einem Differentialblutbild (CBC-DIFF) getestet wird.

3. Verfahren nach Anspruch 1 oder 2, weiter umfassend die Beurteilung eines oder mehrerer der folgenden Parameter: einem Mittelwert des axialen Lichtverlusts (ALL) für Monozyten, einem Mittelwert des Gleichstroms (DC) für Monozyten, einer Standardabweichung für ALL für Monozyten, einem Basophilenanteil, einer mittleren korpuskulären Hämoglobinkonzentration (MCHC), einem Wert für niedrige Hämoglobindichte (LHD), einer Standardabweichung des Volumens der weißen Blutkörperchen (WBK), einer Standardabweichung des Volumens der Monozyten und Neutrophilen oder einem Neutrophilenanteil.

4. Verfahren nach einem der Ansprüche 1-3, wobei die Charakterisierung der WBK in einer Blutprobe eine Impedanzmessung und eine Lichtstreuungsmessung umfasst.

5. Verfahren nach einem der Ansprüche 1-4, wobei die Charakterisierung der WBK in einer Blutprobe nicht auf zytochemischen Färbungen oder affinitätsbasierten Markern beruht.

6. Verfahren nach einem der Ansprüche 1-5, wobei die Charakterisierung der WBK weniger als ein vollständiges CBC-DIFF umfasst.

7. Verfahren nach einem der Ansprüche 1 und 3-6, wobei das Verfahren automatisiert ist.

**Revendications**

1. Procédé d'évaluation d'une infection virale, dans lequel l'infection virale est le SARS-CoV-2 ou la grippe, le procédé comprenant : la caractérisation d'un nombre de globules blancs (WBC) dans un échantillon sanguin ; le calcul d'une valeur de largeur de distribution des monocytes (MDW) pour l'échantillon sanguin ; si la valeur de MDW pour l'échantillon sanguin est inférieure ou égale à 20, le fait d'indiquer qu'une infection virale est peu probable ; et si la valeur de MDW pour l'échantillon sanguin est supérieure ou égale à 20, l'évaluation du pourcentage de lymphocytes et la fourniture d'une indication d'infection virale suspectée si le pourcentage de lymphocytes est inférieur à 15 %.

2. Procédé selon la revendication 1, dans lequel le procédé est automatiquement mis en œuvre sur tout échantillon sanguin testé pour CBC avec différentiel (CBC-DIFF).

**3.** Procédé selon la revendication 1 ou revendication 2, comprenant en outre l'évaluation d'un ou plusieurs éléments parmi la moyenne de perte de lumière axiale (ALL) pour monocytes ; la moyenne de courant continu (CC) pour monocytes ; écart-type pour ALL pour monocytes ; le pourcentage de basophiles ; la concentration corpusculaire moyenne en hémoglobine (MCHC) ; valeur de faible taux d'hémoglobine (LHD) ; l'écart-type, en volume, de WBC ; l'écart-type, en volume, de Monocytes et Neutrophiles ; ou pourcentage de neutrophiles.

**4.** Procédé selon l'une quelconque des revendications 1 à 3, dans lequel la caractérisation du WBC dans un échantillon sanguin comprend des mesures d'impédance et de dispersion de lumière.

**5.** Procédé selon l'une quelconque des revendications 1 à 4, dans lequel la caractérisation du WBC dans un échantillon sanguin ne repose pas sur des colorations cytochimiques ou des marqueurs basés sur l'affinité.

**6.** Procédé selon l'une quelconque des revendications 1 à 5, dans lequel la caractérisation du WBC comprend moins d'un CBC-DIFF complet.

**7.** Procédé selon l'une quelconque des revendications 1 et 3 à 6, dans lequel le procédé est automatisé.

100

106
REMOTE
DEVICE

102
READING
DEVICE

104
NETWORK

108
DATA
STORE

# FIG. 1

FIG. 2

*300*

302 — PATIENT BLOOD SAMPLE IS DELIVERED TO ANALYZER

304 — ANALYZER CONCLUDES SAMPLE PREPARATION

306 — SAMPLE PASSES THROUGH AT LEAST ONE MEASUREMENT MODULE

308 — MEASUREMENTS EVALUATED BY DATA PROCESSING MODULE

310 — MEASUREMENTS DISPLAYED BY REPORTING MODULE

# FIG. 3

FIG. 4

FIG. 5

600

602 — CHARACTERIZE CBC AND DIFF MODULES IN A BLOOD SAMPLE, INCLUDING WBC AND RBC

604 — EXTRACT LEADING INDICATORS FOR COVID-19 POSITIVE SAMPLES USING AUC

606 — SELECT PARAMETERS

608 — CALCULATE STRENGTH OF DETECTION USING A LINEAR WEIGHTED AVERAGE

610 — GREATER THAN SECTION THRESHOLD?

NO → UNLIKELY COVID-19 — 612

YES

614 — LIKELY COVID 19

616 — CONFIRMATORY TESTING

PATIENT TREATMENT — 618

FIG. 6

700

710 — CHARACTERIZE CBC AND DIFF MODULES IN A BLOOD SAMPLE, INCLUDING WBC AND RBC

720 — EXTRACT LEADING INDICATORS FOR COVID-19 POSITIVE SAMPLES USING AUC

730 — SELECT PARAMETERS

740 — CONSTRUCT A COMPOSITE INDEX WITH MAXIMIZED DISCRIMINATING POWER

750 — GREATER THAN SECTION THRESHOLD?

NO → UNLIKELY COVID-19 — 760

YES

770 — LIKELY COVID 19

780 — CONFIRMATORY TESTING

PATIENT TREATMENT — 790

FIG. 7

FIG. 8

FIG. 9

## MDW

|  | ABNORMAL | | NORMAL | |
|---|---|---|---|---|

<table>
<tr><td rowspan="2">ABNORMAL</td><td>ABNORMAL PCT<br>71%</td><td>NORMAL PCT<br>50%</td><td>ABNORMAL PCT<br>55%</td><td>NORMAL PCT<br>23%</td></tr>
<tr><td colspan="2">60%</td><td colspan="2">27%</td></tr>
<tr><td rowspan="2">NORMAL</td><td colspan="2">20%</td><td colspan="2">2%</td></tr>
<tr><td>29%<br>ABNORMAL PCT</td><td>16%<br>NORMAL PCT</td><td>7%<br>ABNORMAL PCT</td><td>2%<br>NORMAL PCT</td></tr>
</table>

WBC

## FIG. 10

MDW

ABNORMAL                                      NORMAL

| ABNORMAL PCT | NORMAL PCT | ABNORMAL PCT | NORMAL PCT |
|---|---|---|---|
| 44% | 13% | 14% | 3% |

WBC

ABNORMAL

| | |
|---|---|
| 28% | 4% |

NORMAL

| | |
|---|---|
| 16% | 2% |

| 30% | 9% | 13% | 2% |
|---|---|---|---|
| ABNORMAL PCT | NORMAL PCT | ABNORMAL PCT | NORMAL PCT |

FIG. 11

1200

1210 — OBTAIN ONE OR MORE PARAMETERS ASSOCIATED WITH A BLOOD SAMPLE FROM AN INDIVIDUAL

1220 — COMPARE MDW VALUE WITH ONE OR MORE PREDETERMINED CRITERIA

1230 — PROVIDE A CLINICAL ACUITY RECOMMENDATION

# FIG. 12

1300

1310 — OBTAIN ONE OR MORE PARAMETERS COMPRISING AN MDW VALUE

1320 — DETERMINE THAT THE MDW VALUE EXCEEDS ONE OR MORE THRESHOLDS

1330 — PROVIDE A RISK ASSESSMENT FOR ONE OR MORE CONDITIONS

# FIG. 13

1400

```
┌─────────────────────────────────────────┐
│ OBTAIN A MONOCYTE DISTRIBUTION WIDTH     │
1410 ─┤ (MDW) VALUE AND ONE OR MORE SECONDARY    │
│ PARAMETERS FOR AN INDIVIDUAL             │
└─────────────────────────────────────────┘
```

```
┌─────────────────────────────────────────┐
│ COMPARE THE MDW WITH ONE OR MORE         │
1420 ─┤ PREDETERMINED CRITERIA                   │
└─────────────────────────────────────────┘
```

```
┌─────────────────────────────────────────┐
│ COMPARE THE ONE OR MORE SECONDARY        │
1430 ─┤ PARAMETERS WITH CORRESPONDING            │
│ PREDETERMINED CRITERIA                   │
└─────────────────────────────────────────┘
```

```
┌─────────────────────────────────────────┐
1440 ─┤ PROVIDE A CLINICAL ACUITY RECOMMENDATION │
└─────────────────────────────────────────┘
```

FIG. 14

1500

```
┌─────────────────────────────────────────┐
1510 ─┤ MEASURING A FIRST MDW VALUE AT A FIRST TIME │
└─────────────────────────────────────────┘
```

```
┌─────────────────────────────────────────┐
│ MEASURING A SECOND MDW VALUE AT A SECOND │
1520 ─┤ TIME THAT IS AT LEAST 24 HOURS AFTER THE FIRST │
│ TIME                                     │
└─────────────────────────────────────────┘
```

```
┌─────────────────────────────────────────┐
│ ASSESSING THE CLINICAL ACUITY OF THE IMMUNE- │
│ COMPROMISED INDIVIDUAL BASED ON THE      │
1530 ─┤ DIFFERENCE BETWEEN THE FIRST AND SECOND  │
│ MDW VALUES                               │
└─────────────────────────────────────────┘
```

FIG. 15

FIG. 16A

FIG. 16B

FIG. 17

FIG. 18A

FIG. 18B

FIG. 18C

FIG. 18D

FIG. 19

FIG. 20A

FIG. 20B

FIG. 20C

FIG. 20D

EP 4 139 655 B1

FIG. 21A

60

FIG. 21B

FIG. 21C

FIG. 21D

FIG. 22A

FIG. 22B

FIG. 22C

FIG. 22D

FIG. 23A

FIG. 23B

FIG. 23C

FIG. 23D

FIG. 24A

FIG. 24B

FIG. 24C

FIG. 24D

COVID-19

Positive
auc = 76%
sensitivity = 76%
specificity = 65%

Monocyte Distribution Width (MDW)

covid negative
(N = 1129)

covid positive
(N = 91)

2510

COVID-19

Positive
auc = 52%
sensitivity = 24%
specificity = 74%

Neutrophil %

covid negative
(N = 1047)

covid positive
(N = 82)

2520

FIG. 25A

FIG. 25B

FIG. 25C

**COVID-19**

2590

**COVID-19**

2595

FIG. 25D

Influenza

Positive
auc = 75%
sensitivity = 86%
specificity = 53%

2610

flu negative
(N = 855)

flu positive
(N = 56)

Influenza

Positive
auc = 50%
sensitivity = 42%
specificity = 64%

2620

flu negative
(N = 778)

flu positive
(N = 53)

FIG. 26A

FIG. 26B

FIG. 26C

FIG. 26D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5125737 A
- US 62288091
- US 62927835 B
- US 62660795 B
- US 62873806 B
- US 62873575 B
- US 62685753 B
- US 6228652 B
- US 7390662 B
- US 8094299 B

- US 8189187 B
- US 9939453 B
- US 073757
- US 16925933 B
- US 1928486 W
- US 16925943 B
- US 16925937 B
- US 16390597 B
- US 2017014708 W

**Non-patent literature cited in the description**

- **CROUSER et al.** 2017 relates to the early detection of sepsis with a monocyte distribution width biomarker. *CHEST*, 2017, vol. 152 (3), 518-526
- **CROUSER et al.** 2019 relates to monocyte distribution width as an indicator of sepsis-2 and sepsis-3 in high-risk emergency department patients. *Critical Care Medicine*, 2019, vol. 47 (8), 1018-1025

- **FRATER et al.** 2020 discusses the clinical hematology laboratory's role in the diagnosis of COVID-19 infection, as well as biomarker analysis in COVID-19 infection. *International Journal of Laboratory Hematology*, 2020, vol. 42 (S1), 11-18
- **PARK, D.-H**. Screening of sepsis using leukocyte cell population data from the Coulter automatic blood cell analyzer DxH800. *Int. Jnl. 15 Lab. Hem*, 2011, vol. 33, 391-399